(19) Europäisches Patentamt
European Patent Office
Office européen des brevets

(11) EP 4 186 893 A1

(12) EUROPEAN PATENT APPLICATION
published in accordance with Art. 153(4) EPC

(43) Date of publication:
**31.05.2023 Bulletin 2023/22**

(21) Application number: **21847375.9**

(22) Date of filing: **22.07.2021**

(51) International Patent Classification (IPC):
***C07D 401/12*** (2006.01) ***A61K 31/4545*** (2006.01)
***A61P 25/16*** (2006.01) ***A61P 25/00*** (2006.01)
***A61P 25/22*** (2006.01) ***A61P 25/28*** (2006.01)
***A61P 25/20*** (2006.01) ***A61P 25/18*** (2006.01)

(52) Cooperative Patent Classification (CPC):
**A61K 31/4545; A61P 25/00; A61P 25/16; A61P 25/18; A61P 25/20; A61P 25/22; A61P 25/28; C07D 401/12**

(86) International application number:
**PCT/CN2021/107774**

(87) International publication number:
**WO 2022/017440 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.07.2020 CN 202010708352**
**03.04.2021 CN 202110364023**

(71) Applicant: **Geneora Pharma (Shijiazhuang) Co., Ltd.**
**Shijiazhuang city, Hebei province 050000 (CN)**

(72) Inventors:
- **ZHANG, Rui**
  **Yantai, Shandong 264670 (CN)**
- **YE, Liang**
  **Yantai, Shandong 264670 (CN)**
- **MA, Mingxu**
  **Yantai, Shandong 264670 (CN)**
- **WANG, Wenyan**
  **Yantai, Shandong 264670 (CN)**
- **DAI, Yusen**
  **Yantai, Shandong 264670 (CN)**
- **TIAN, Jingwei**
  **Yantai, Shandong 264670 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(54) **5-HT2A RECEPTOR INHIBITOR OR INVERSE AGONIST, PREPARATION METHOD THEREFOR, AND APPLICATION THEREOF**

(57) The present invention relates to a novel compound as a 5-$HT_{2A}$ receptor inhibitor or inverse agonist, a preparation method therefor, and a pharmaceutical composition thereof. The present invention also relates to an application of the compound or the pharmaceutical composition in the preparation of a drug for treating 5-$HT_{2A}$ receptor-related diseases, the diseases comprising: non-motor symptoms caused by Parkinson's disease: delusion, illusion, depression, anxiety, cognitive disorder, and sleep disorder; dementia-related mental diseases; major depressive disorder; or negative symptoms of schizophrenia, etc.

EP 4 186 893 A1

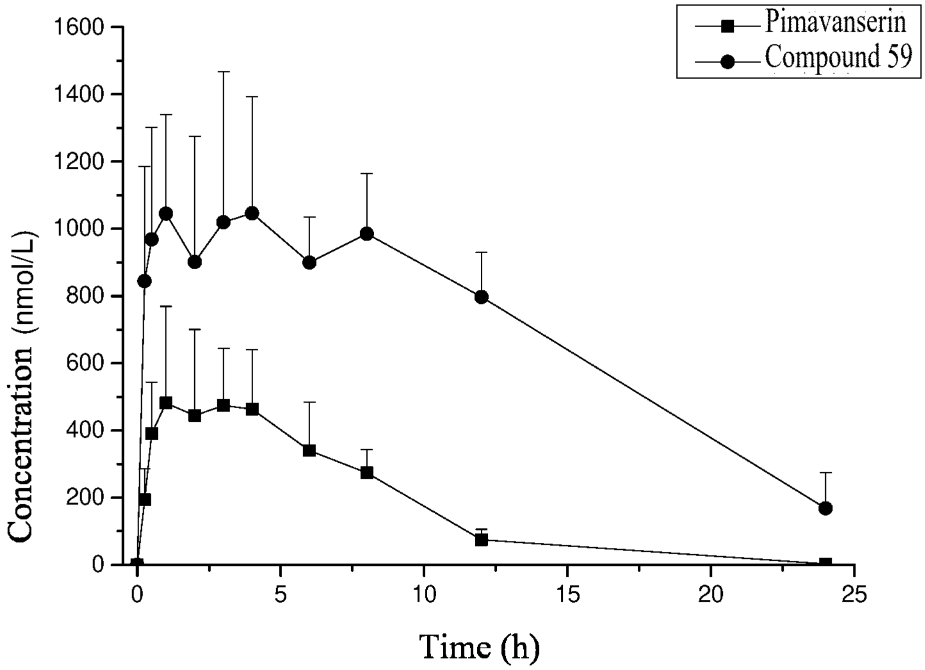
Pimavanserin
Compound 59
Concentration (nmol/L)
1600
1400
1200
1000
800
600
400
200
0
0
5
10
15
20
25
Time (h)

*Fig. 1*

## Description

### Technical Field

The present invention relates to a class of compounds as selective 5-hydroxytryptamine 2A (5-$HT_{2A}$) receptor inhibitors or inverse agonists, a preparation method therefor, and the use thereof in the field of 5-$HT_{2A}$ receptor-related diseases.

### Background Art

Parkinson's disease (PD) is a common neurodegenerative disease with an average age of onset of around 60 years (Degirmenci, Yildiz. Cumhuriyet Medical Journal (2017), 39 (3), 509-517). According to data from the National Institutes of Health (NIH) in 2018, there are about 4 million to 6 million patients with Parkinson's disease worldwide. Among them, up to 50% of patients with Parkinson's disease develop severe symptoms of illusion or delusion in the course of their illness, which seriously affects the quality of life of patients and leads to high morbidity and mortality.

In 2016, pimavanserin comes into the market for treating illusion and delusion symptoms accompanying Parkinson's disease as approved by the U.S. FDA, and becomes the first approved drug for treating such indications.

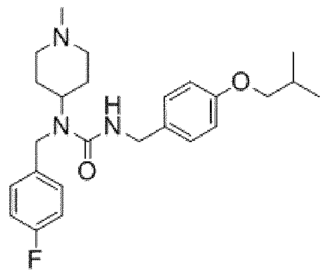

Pimavanserin

Pimavanserin acts on a 5-$HT_{2A}$ receptor which is a main excitatory receptor subtype in the 5-HT receptor family and is a ligand-gated channel receptor and a G protein-coupled receptor. The 5-$HT_{2A}$ receptor has a function closely related to neuronal excitation, behavioral effects, learning and memory, anxiety, etc., and thus is an important target for antipsychotic drugs and schizophrenia treatment (Price, D. L., et al. Behavioral Pharmacology (2012), 23(4), 426-433). The first-generation antipsychotic drug is mainly used to inhibit a dopamine $D_2$ receptor and thus has serious extrapyramidal side effects. The second-generation antipsychotic drug, in addition to inhibiting the $D_2$ receptor, has more inhibitory effects on a specific 5-HT receptor, particularly the 5-$HT_{2A}$ receptor, and has higher safety, i.e., fewer extrapyramidal side effects in comparison with the first-generation antipsychotic drug. However, the second-generation antipsychotic drug still has inhibitory activities against the $D_2$ receptor and thus still has extrapyramidal side effects. Moreover, such drug has the side effect of weight gain in different degrees.

Therefore, developing a selective 5-$HT_{2A}$ receptor inhibitor or inverse agonist can eliminate the extrapyramidal side effects related to dopamine receptor inhibition and the side effect of weight gain of the first-generation and second-generation antipsychotic drugs. The receptor inhibitor or inverse agonist has higher safety, and can also be applied to the treatment of other diseases related to 5-$HT_{2A}$ receptors to meet the needs of clinical patients.

### Summary of the Invention

In one aspect, the present invention provides a compound of formula (I) or a pharmaceutically acceptable salt thereof,

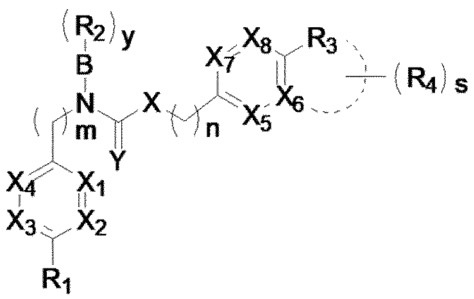

(I)

wherein

at least one of $X_1$ and $X_4$ is N, and the other one is optionally $CR_1$ or N;
$X_2$ and $X_3$ are each independently selected from $CR_1$ and N;
$X_5$ is independently selected from $CR_{3a}$ or N;
$X_6$ is independently selected from $CR_{3b}$ or N;
$X_7$ is independently selected from $CR_{3c}$ or N;
$X_8$ is independently selected from $CR_{3d}$ or N;
group B is linear or branched $C_{1-6}$ alkyl or a 5-6-membered nitrogen heterocyclic group, and the linear or branched $C_{1-6}$ alkyl or the 5-6-membered nitrogen heterocyclic group is optionally substituted with one or more deuterium atoms;
each $R_1$ is the same or different and is independently selected from a hydrogen atom, linear or branched $C_{1-10}$ alkyl or halogen;
each $R_2$ is the same or different and is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;
$R_3$, $R_{3a}$, $R_{3b}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;
or when $X_6$ is $CR_{3b}$, $X_6$ and $R_3$ together with the atoms to which they are attached form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy;
X is selected from -NH- or $-(CH_2)_{1-4}NH-$;
Y is selected from O or S;
m and n are independently selected from 0, 1, 2 and 3;
s is independently selected from 1, 2, 3, 4, 5 and 6; and
y is independently selected from 0, 1, 2, 3, 4 and 5.

In one embodiment of the compound of formula (I), at least one of $X_1$ and $X_4$ is N, and the other one is optionally $CR_1$; and $X_2$ and $X_3$ are each independently selected from $CR_1$, preferably CH.

In one embodiment of the compound of formula (I), $X_1$ and $X_4$ are both N, and $X_2$ and $X_3$ are each independently selected from $CR_1$.

In one embodiment of the compound of formula (I), at least one of $X_1$ and $X_4$ is N, and the other one is optionally $CR_1$; and at least one of $X_2$ and $X_3$ is N, and the other one is optionally $CR_1$.

In one embodiment of the compound of formula (I), $X_1$ and $X_4$ are both N; and at least one of $X_2$ and $X_3$ is N, and the other one is optionally $CR_1$.

In one embodiment of the compound of formula (I), at least one of $X_1$ and $X_4$ is N, and the other one is optionally $CR_1$; and $X_2$ and $X_3$ are both N.

In one embodiment of the compound of formula (I), $X_1$ and $X_4$ are both N, and $X_2$ and $X_3$ are both N.

In one embodiment of the compound of formula (I), $X_1$ is preferably N, and $X_2$, $X_3$ and $X_4$ are all $CR_1$.

In one embodiment of the compound of formula (I), group B is $-CH_2-$, $-(CH_2)_2-$, $-(CH_2)_3-$, $-(CH_2)_4-$, $-CD_2-$,

$-(CD_2)_2-$, $-(CD_2)_3-$ or $-(CD_2)_4-$, and $R_2$ is independently selected from dimethylamine or diethylamine, wherein the dimethylamine or the diethylamine is optionally substituted with one or more deuterium atoms;
or group B is selected from piperidinyl, wherein the piperidinyl is optionally substituted with one or more deuterium atoms, and $R_2$ is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms.

In one embodiment of the compound of formula (I), each $R_1$ is the same or different and is independently selected from a hydrogen atom, F, Cl, Br, I, methyl, ethyl, propyl, isopropyl, butyl, isobutyl, sec-butyl or tert-butyl.

In one embodiment of the compound of formula (I), each $R_2$ is the same or different and is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms.

In one embodiment of the compound of formula (I), $R_3$, $R_{3a}$, $R_{3b}$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and i-Pr-O-$CH_2$-.

In one embodiment of the compound of formula (I), when $X_6$ is $CR_{3b}$, $X_6$ and $R_3$ together with the atoms to which they are attached form a ring system and are optionally substituted with one or more of the same or different $R_4$,

wherein the ring system is preferably selected from dihydrofuran, dihydropyrrole or dihydrothiophene, and
wherein each $R_4$ is the same or different and is independently selected from a hydrogen atom, methyl, ethyl, propyl, isopropyl, butyl, isobutyl, sec-butyl or tert-butyl.

In one specific embodiment of the present invention, any one of the above-mentioned compounds of formula (I) may be a deuterated analog thereof. The deuterated analog refers to an analog formed by substituting one or more hydrogen atoms of a compound with deuterium atoms.

Compared with pimavanserin, the compound of formula (I) provided by the present invention has higher 5-$HT_{2A}$ antagonistic activity and 5-$HT_{2A}$ inverse agonistic activity, and/or lower cardiotoxicity. Particularly, when $X_1$ and/or $X_4$ are/is N, and $X_2$ and $X_3$ are CH, the 5-$HT_{2A}$ antagonistic activity and/or the 5-$HT_{2A}$ inverse agonistic activity can be further improved.

In another aspect, the present invention provides a compound of formula (II) or a pharmaceutically acceptable salt thereof,

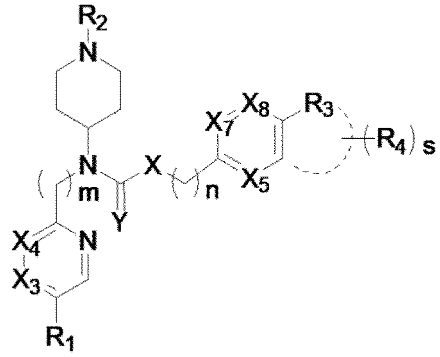

(II)

wherein

$X_3$ and $X_4$ are each independently selected from $CR_1$ and N;
$X_5$ is independently selected from $CR_{3a}$ or N;
$X_7$ is independently selected from $CR_{3c}$ or N;

$X_8$ is independently selected from $CR_{3d}$ or N;
each $R_1$ is the same or different and is independently selected from a hydrogen atom, linear or branched $C_{1-10}$ alkyl or halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;
$R_3$, $R_{3a}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;
or $R_3$ and the carbon atom to which it is attached together with adjacent carbon atoms form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy;
X is selected from -NH- or $-(CH_2)_{1-4}NH-$;
Y is selected from O or S;
m and n are independently selected from 0, 1, 2 and 3; and
s is independently selected from 1, 2, 3, 4, 5 and 6.

In one embodiment of the compound of formula (II), $X_3$ and $X_4$ are both $CR_1$.
In one embodiment of the compound of formula (II), $X_3$ is N and $X_4$ is $CR_1$.
In one embodiment of the compound of formula (II), $X_3$ is $CR_1$ and $X_4$ is N.
In one embodiment of the compound of formula (II), each $R_1$ is the same or different and is independently selected from a hydrogen atom, F, Cl, Br, I, methyl, ethyl, propyl, isopropyl, butyl, isobutyl, sec-butyl or tert-butyl.
In one embodiment of the compound of formula (II), each $R_2$ is the same or different and is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms, preferably a hydrogen atom, a deuterium atom, methyl, ethyl, deuterated methyl or deuterated ethyl, and more preferably methyl or deuterated methyl.
In one embodiment of the compound of formula (II), $R_3$, $R_{3a}$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and $i\text{-}Pr\text{-}O\text{-}CH_2\text{-}$.
In one embodiment of the compound of formula (II), $R_3$ and the carbon atom to which it is attached together with adjacent carbon atoms form a ring system and are optionally substituted with one or more of the same or different $R_4$,

wherein the ring system is preferably selected from dihydrofuran, dihydropyrrole or dihydrothiophene, and
wherein each $R_4$ is the same or different and is independently selected from a hydrogen atom, methyl, ethyl, propyl, isopropyl, butyl, isobutyl, sec-butyl or tert-butyl.

In one specific embodiment of the present invention, any one of the above-mentioned compounds of formula (II) may be a deuterated analog thereof. The deuterated analog refers to an analog formed by substituting one or more hydrogen atoms of a compound with deuterium atoms.
Compared with pimavanserin, the compound of formula (II) provided by the present invention has higher $5\text{-}HT_{2A}$ antagonistic activity and $5\text{-}HT_{2A}$ inverse agonistic activity, and/or lower cardiotoxicity. In another aspect, the present invention provides a compound of formula (III) or a pharmaceutically acceptable salt thereof,

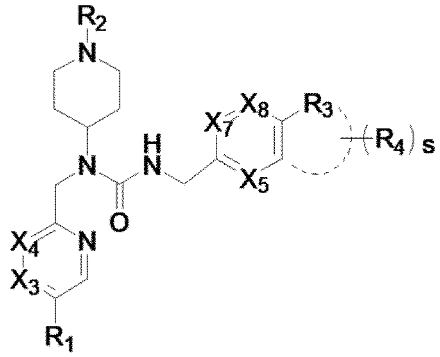

(III)

wherein

$X_3$ and $X_4$ are each independently selected from $CR_1$ and N;
$X_5$ is independently selected from $CR_{3a}$ or N;
$X_7$ is independently selected from $CR_{3c}$ or N;
$X_8$ is independently selected from $CR_{3d}$ or N;
each $R_1$ is the same or different and is independently selected from a hydrogen atom, linear or branched $C_{1-10}$ alkyl or halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;
$R_3$, $R_{3a}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;
or $R_3$ and the carbon atom to which it is attached together with adjacent carbon atoms form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy; and
s is independently selected from 1, 2, 3, 4, 5 and 6.

In one embodiment of the compound of formula (III), $X_3$ and $X_4$ are both $CR_1$.
In one embodiment of the compound of formula (III), $X_3$ is N and $X_4$ is $CR_1$.
In one embodiment of the compound of formula (III), $X_3$ is $CR_1$ and $X_4$ is N.
In one embodiment of the compound of formula (III), each $R_1$ is the same or different and is independently selected from a hydrogen atom, F, Cl, Br, I, methyl, ethyl, propyl, isopropyl, butyl, isobutyl, sec-butyl or tert-butyl.
In one embodiment of the compound of formula (III), each $R_2$ is the same or different and is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms, preferably a hydrogen atom, a deuterium atom, methyl, ethyl, deuterated methyl or deuterated ethyl, and more preferably methyl or deuterated methyl.
In one embodiment of the compound of formula (III), $R_3$, $R_{3a}$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and i-Pr-O-$CH_2$-.
In one embodiment of the compound of formula (III), $R_3$ and the carbon atom to which it is attached together

with adjacent carbon atoms form a ring system and are optionally substituted with one or more of the same or different $R_4$,

wherein the ring system is preferably selected from dihydrofuran, dihydropyrrole or dihydrothiophene, and wherein each $R_4$ is the same or different and is independently selected from a hydrogen atom, methyl, ethyl, propyl, isopropyl, butyl, isobutyl, sec-butyl or tert-butyl.

In one specific embodiment of the present invention, any one of the above-mentioned compounds of formula (III) may be a deuterated analog thereof. The deuterated analog refers to an analog formed by substituting one or more hydrogen atoms of a compound with deuterium atoms.

Compared with pimavanserin, the compound of formula (III) provided by the present invention has higher 5-$HT_{2A}$ antagonistic activity and 5-$HT_{2A}$ inverse agonistic activity, and/or lower cardiotoxicity. In another aspect, the present invention provides a compound of formula (IV) or a pharmaceutically acceptable salt thereof,

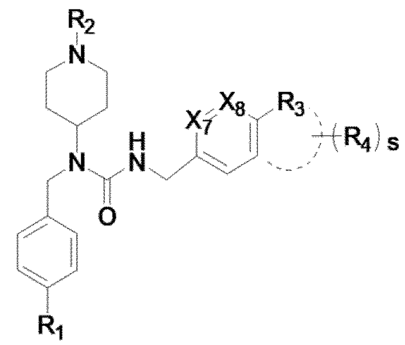

(IV)

wherein

$X_7$ is independently selected from $CR_{3c}$ or N;

$X_8$ is independently selected from $CR_{3d}$ or N;

$R_1$ is independently selected from a hydrogen atom, linear or branched $C_{1-10}$ alkyl or halogen;

$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;

$R_3$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;

or $R_3$ and the carbon atom to which it is attached together with adjacent carbon atoms form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy; and

s is independently selected from 1, 2, 3, 4, 5 and 6.

In one embodiment of the compound of formula (IV), each $R_1$ is the same or different and is independently selected from a hydrogen atom, F, Cl, Br, I, methyl, ethyl, propyl, isopropyl, butyl, isobutyl, sec-butyl or tert-butyl.

In one embodiment of the compound of formula (IV), each $R_2$ is the same or different and is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms.

In one embodiment of the compound of formula (IV), $R_3$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and i-Pr-O-$CH_2$-.

In one embodiment of the compound of formula (IV), $R_3$ and the carbon atom to which it is attached together with adjacent carbon atoms form a ring system and are optionally substituted with one or more of the same or different $R_4$,

wherein the ring system is preferably selected from dihydrofuran, dihydropyrrole or dihydrothiophene, and
wherein each $R_4$ is the same or different and is independently selected from a hydrogen atom, methyl, ethyl, propyl, isopropyl, butyl, isobutyl, sec-butyl or tert-butyl.

In one specific embodiment of the present invention, any one of the above-mentioned compounds of formula (IV) may be a deuterated analog thereof. The deuterated analog refers to an analog formed by substituting one or more hydrogen atoms of a compound with deuterium atoms.

Compared with pimavanserin, the compound of formula (IV) provided by the present invention has higher 5-$HT_{2A}$ antagonistic activity and 5-$HT_{2A}$ inverse agonistic activity, and/or lower cardiotoxicity.

In another aspect, the present invention provides a compound of formula (V) or a pharmaceutically acceptable salt thereof,

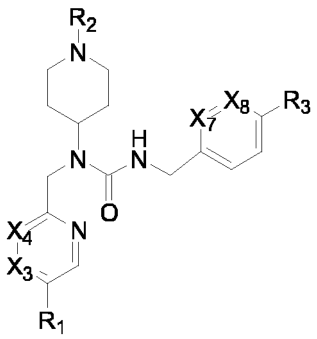

(V)

wherein

$X_3$ is independently selected from $CR_5$ or N;
$X_4$ is independently selected from $CR_6$ or N;
$X_7$ is independently selected from $CR_{3c}$ or N;
$X_8$ is independently selected from $CR_{3d}$ or N;
$R_1$, $R_5$ and $R_6$ are the same or different and are each independently selected from a hydrogen atom, linear or branched $C_{1-10}$ alkyl and halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms; and
$R_3$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy.

In one embodiment of the compound of formula (V), $X_3$ and $X_4$ are $CR_5$ and $CR_6$, respectively.

In one embodiment of the compound of formula (V), $X_3$ is N and $X_4$ is $CR_6$.

In one embodiment of the compound of formula (V), $X_3$ is $CR_5$ and $X_4$ is N.

In one embodiment of the compound of formula (V), $R_1$, $R_5$ and $R_6$ are the same or different and are each independently selected from a hydrogen atom, F, Cl, Br, I, methyl, ethyl, propyl, isopropyl, butyl, isobutyl, sec-butyl and tert-butyl, preferably F, Cl, Br or I, and more preferably F.

In one embodiment of the compound of formula (V), $R_2$ is independently selected from a hydrogen atom, a deuterium atom, methyl, ethyl, propyl, isopropyl, butyl, isobutyl, sec-butyl, tert-butyl, cyclopropyl, cyclobutyl, cyclopentyl

or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms, preferably a hydrogen atom, a deuterium atom, methyl, ethyl, deuterated methyl or deuterated ethyl, and more preferably methyl or deuterated methyl.

In one embodiment of the compound of formula (V), $R_3$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and i-Pr-O-$CH_2$-.

In one embodiment of the compound of formula (V),

$X_3$ and $X_7$ are both CH;
$X_4$ is $CR_6$, wherein $R_6$ is a hydrogen atom or halogen, and $X_4$ is preferably CH or CF;
$X_8$ is $CR_{3d}$, and $R_{3d}$ is a hydrogen atom or halogen, preferably a hydrogen atom, F, Cl or Br;
$R_1$ is halogen, preferably F;
$R_2$ is independently selected from a hydrogen atom, a deuterium atom or linear or branched $C_{1-5}$ alkyl, wherein the linear or branched $C_{1-5}$ alkyl is optionally substituted with one or more deuterium atoms, preferably a hydrogen atom, a deuterium atom, methyl, ethyl, propyl or isopropyl, wherein the methyl, the ethyl, the propyl or the isopropyl is optionally substituted with one or more deuterium atoms, preferably a hydrogen atom, methyl or ethyl, and more preferably methyl;
$R_3$ is selected from hydroxyl, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy; $R_3$ is preferably substituted or unsubstituted linear or branched $C_{2-5}$ alkyl, substituted or unsubstituted linear or branched $C_{2-5}$ alkoxy, or substituted or unsubstituted linear or branched $C_{2-5}$ haloalkoxy; and $R_3$ is more preferably ethoxy, tert-butyl, isobutyloxy, 2-fluoroethoxy, 2,2-difluoroethoxy, 2,2,2-trifluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy or hydroxyl.

In one embodiment of the compound of formula (V),
$X_3$, $X_4$, $X_7$ and $X_8$ are all CH; $R_1$ is halogen, preferably F; $R_2$ is methyl; and $R_3$ is selected from linear or branched $C_{1-10}$ alkoxy substituted with halogen, preferably linear or branched $C_{2-5}$ haloalkoxy, and more preferably 2-fluoroethoxy, 2,2-difluoroethoxy, 2,2,2-trifluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy or 4,4,4-trifluorobutoxy.

In one specific embodiment of the present invention, any one of the above-mentioned compounds of formula (V) may be a deuterated analog thereof. The deuterated analog refers to an analog formed by substituting one or more hydrogen atoms of a compound with deuterium atoms.

Compared with pimavanserin, the compound of formula (V) provided by the present invention has higher 5-$HT_{2A}$ antagonistic activity and 5-$HT_{2A}$ inverse agonistic activity, and/or lower cardiotoxicity. Particularly, when $R_3$ is linear or branched $C_{1-10}$ alkoxy substituted with halogen, and $X_3$ and $X_4$ are CH, the 5-$HT_{2A}$ antagonistic activity and/or the 5-$HT_{2A}$ inverse agonistic activity can be further improved.

In another aspect, the present invention provides a compound of formula (VI) or a pharmaceutically acceptable salt thereof,

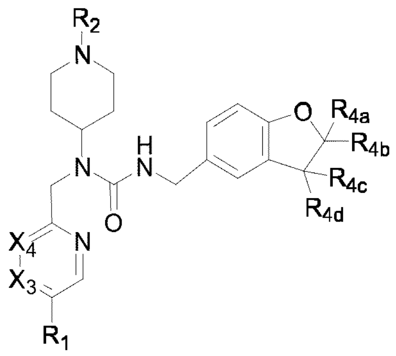

(VI)

wherein

$X_3$ is independently selected from $CR_5$ or N;
$X_4$ is independently selected from $CR_6$ or N;
$R_1$, $R_5$ and $R_6$ are the same or different and are each independently selected from a hydrogen atom, linear or branched $C_{1-10}$ alkyl and halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms; and
$R_{4a}$, $R_{4b}$ and $R_{4c}$ and $R_{4d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy.

In one embodiment of the compound of formula (VI), $X_3$ and $X_4$ are $CR_5$ and $CR_6$, respectively.
In one embodiment of the compound of formula (VI), $X_3$ is N and $X_4$ is $CR_6$.
In one embodiment of the compound of formula (VI), $X_3$ is $CR_5$ and $X_4$ is N.
In one embodiment of the compound of formula (VI), $R_1$ is independently selected from a hydrogen atom, F, Cl, Br, I, methyl, ethyl, propyl, isopropyl, butyl, isobutyl, sec-butyl or tert-butyl, preferably F, Cl, Br or I, and more preferably F.
In one embodiment of the compound of formula (VI), $R_2$ is the same or different and is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms, preferably a hydrogen atom, a deuterium atom, methyl, ethyl, deuterated methyl or deuterated ethyl, and more preferably methyl or deuterated methyl.
In one embodiment of the compound of formula (VI), $R_{4a}$, $R_{4b}$, $R_{4c}$ and $R_{4d}$ are the same or different and are each independently selected from a hydrogen atom, methyl, ethyl, propyl, isopropyl, butyl, isobutyl, sec-butyl and tert-butyl.
In one embodiment of the compound of formula (VI),

$X_3$ is CH;
$X_4$ is $CR_6$, wherein $R_6$ is a hydrogen atom or halogen, and $X_4$ is preferably CH or CF;
$R_1$ is halogen, preferably F;
$R_2$ is independently selected from a hydrogen atom, a deuterium atom or linear or branched $C_{1-5}$ alkyl, wherein the linear or branched $C_{1-5}$ alkyl is optionally substituted with one or more deuterium atoms, preferably a hydrogen atom, a deuterium atom, methyl, ethyl, propyl or isopropyl, wherein the methyl, the ethyl, the propyl or the isopropyl is optionally substituted with one or more deuterium atoms, preferably a hydrogen atom, a deuterium atom, methyl, ethyl, deuterated methyl or deuterated ethyl, and more preferably methyl or deuterated methyl;
$R_{4a}$ and $R_{4b}$ are independently selected from a hydrogen atom, methyl and trifluoromethyl; and $R_{4c}$ and $R_{4d}$ are hydrogen atoms.

In one specific embodiment of the present invention, any one of the above-mentioned compounds of formula

(VI) may be a deuterated analog thereof. The deuterated analog refers to an analog formed by substituting one or more hydrogen atoms of a compound with deuterium atoms.

Compared with pimavanserin, the compound of formula (VI) provided by the present invention has higher 5-$HT_{2A}$ antagonistic activity and/or 5-$HT_{2A}$ inverse agonistic activity, and lower cardiotoxicity.

In another aspect, the present invention provides compounds or pharmaceutically acceptable salts thereof or deuterated analogs thereof as shown below:

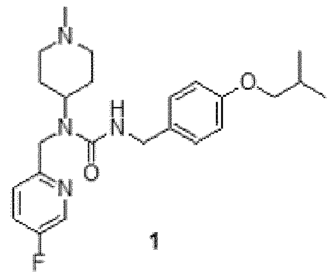
1

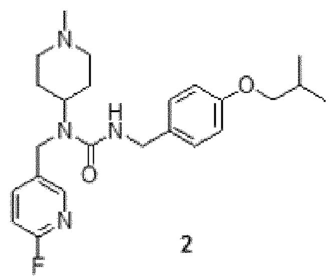
2

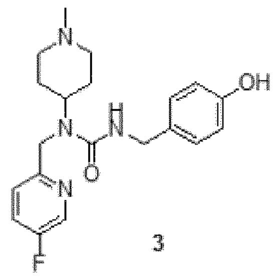
3

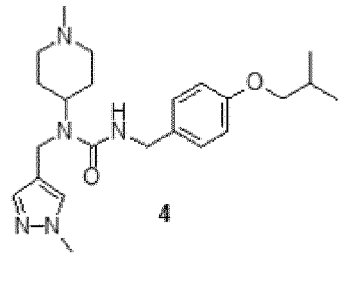
4

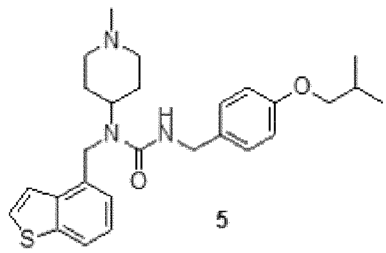
5

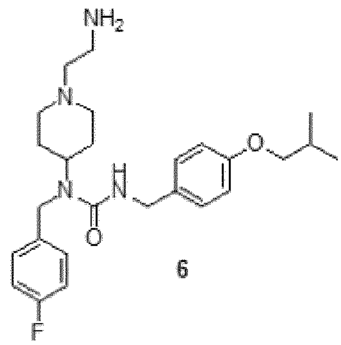
6

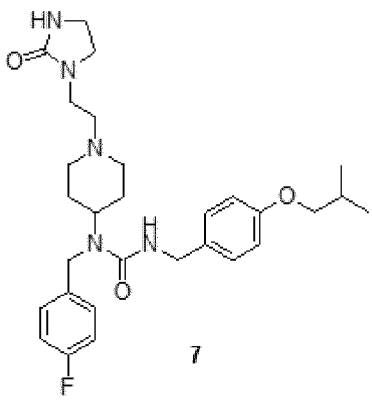
7

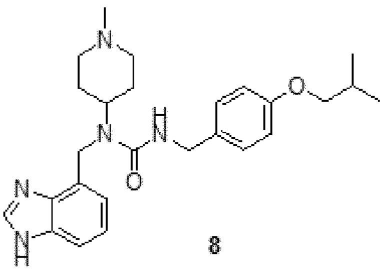
8

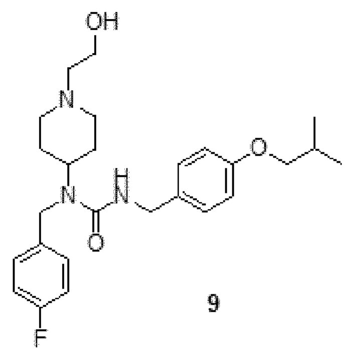
9

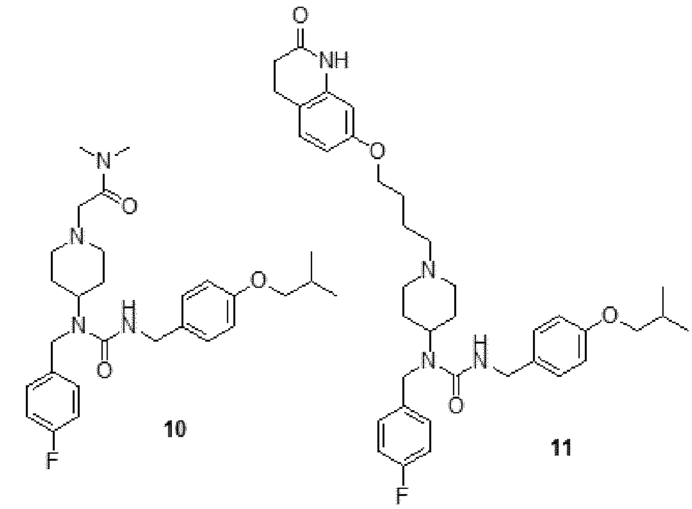

10

11

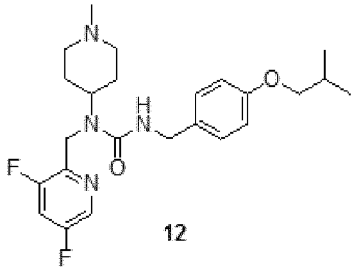

12

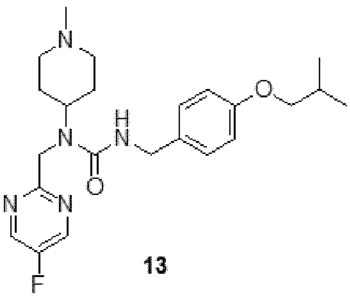

13

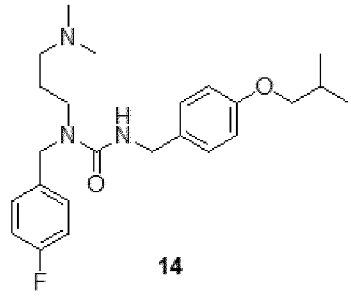

14

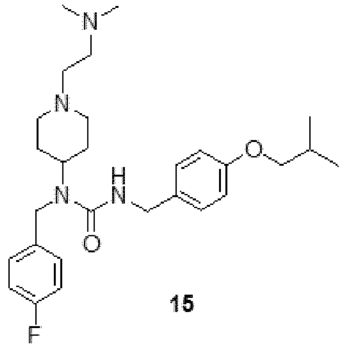

15

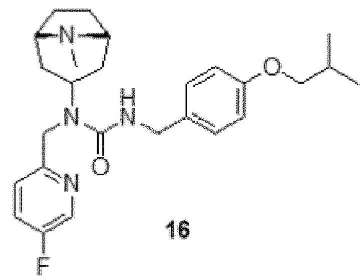

16

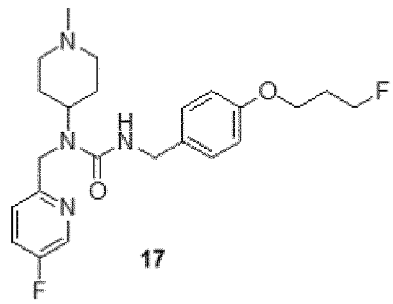

17

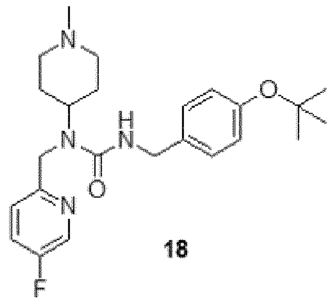

18

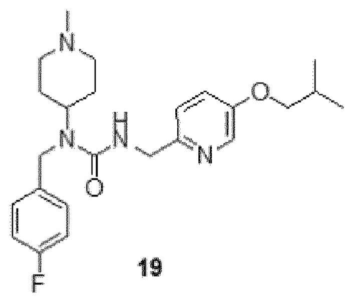

19

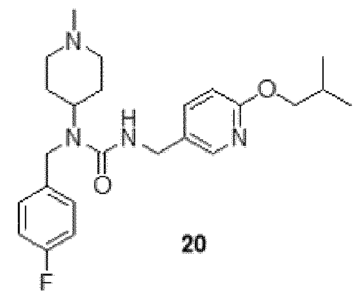

20

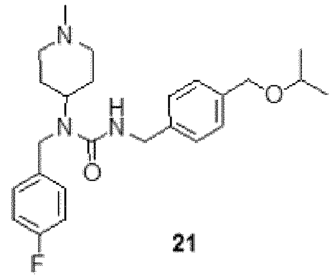

21

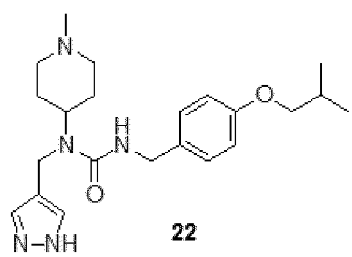

22

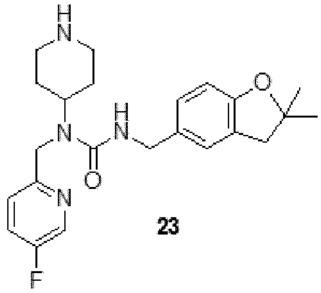

23

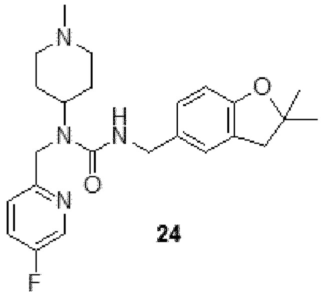

24

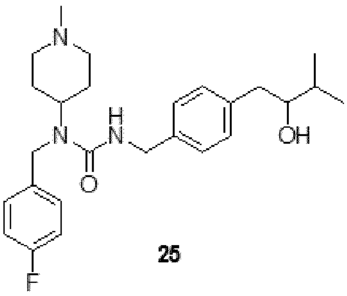

25

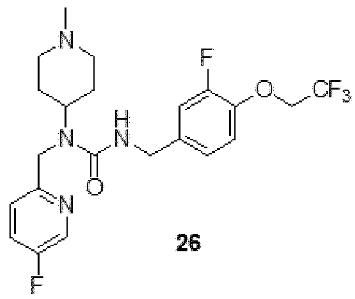

26

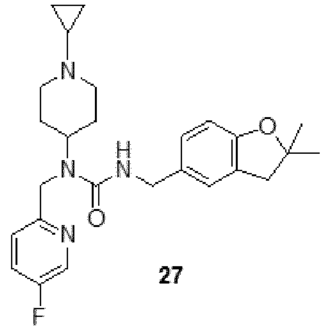

27

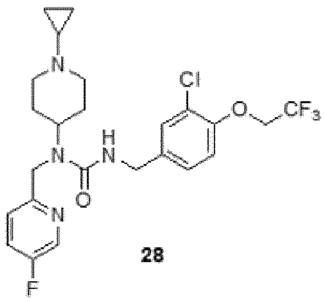

28

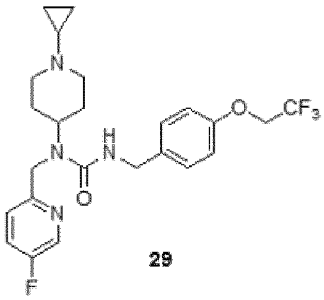

29

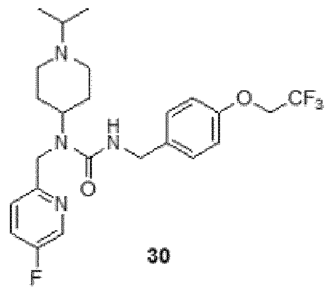

30

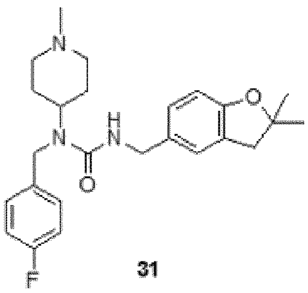

31

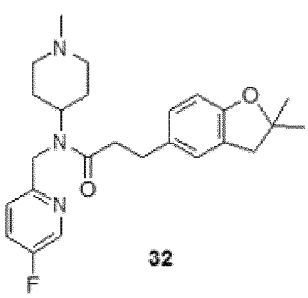

32

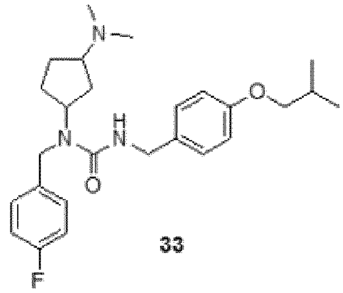

33

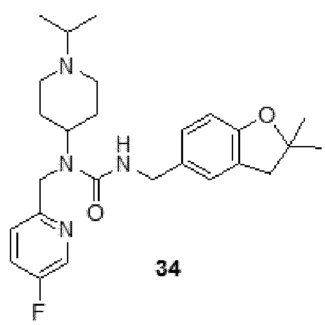

34

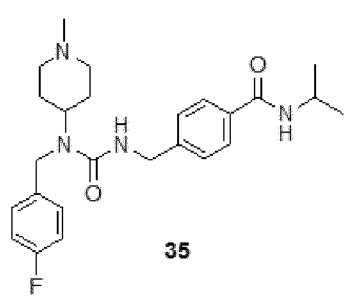

35

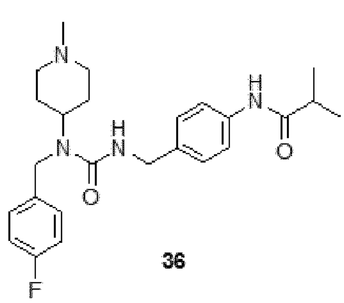

36

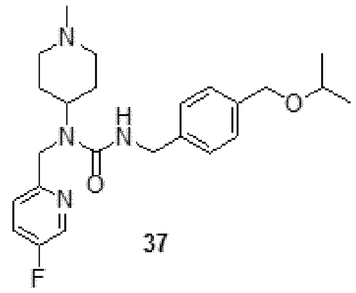

37

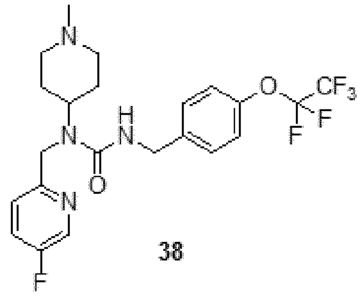

38

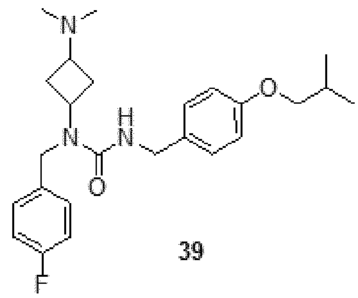

39

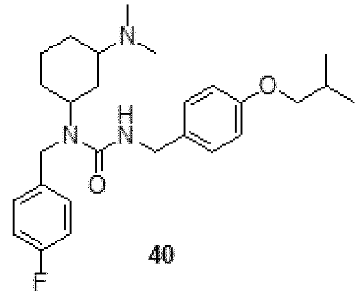

40

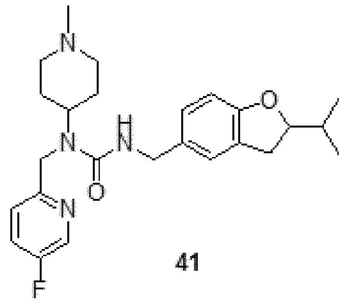

41

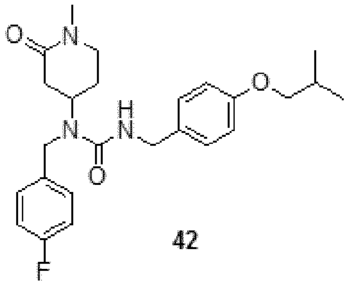

42

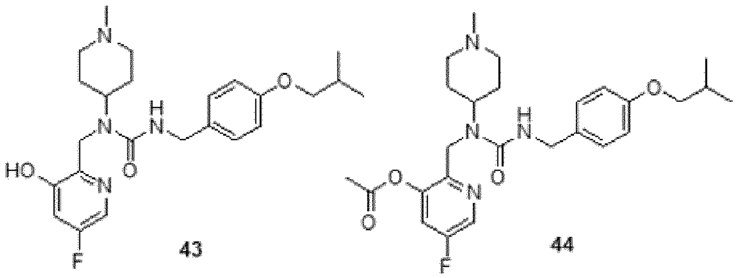

43

44

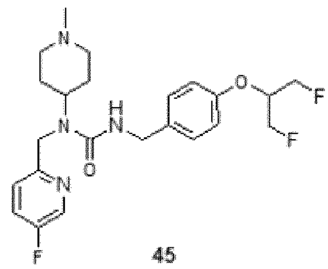

45

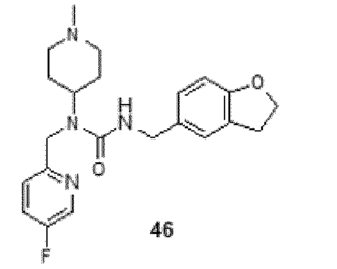

46

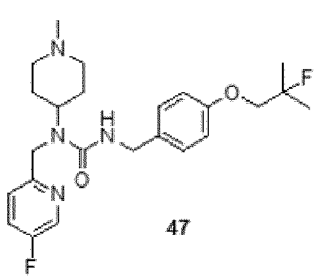

47

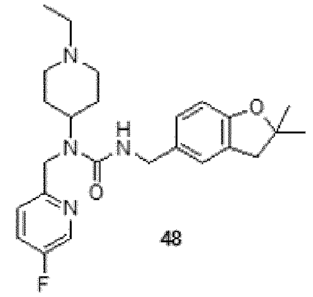

48

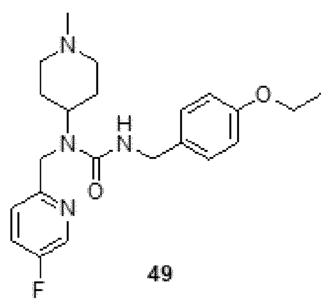

49

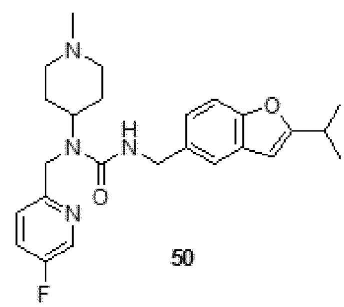

50

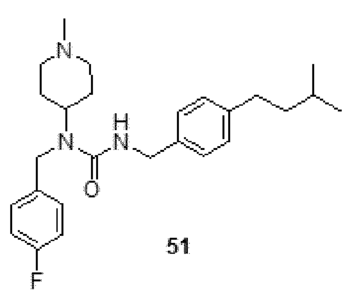

51

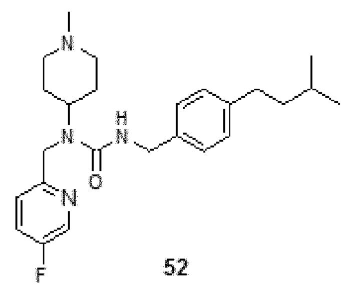
52

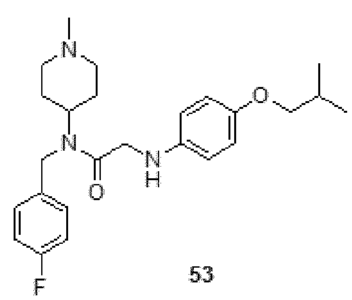
53

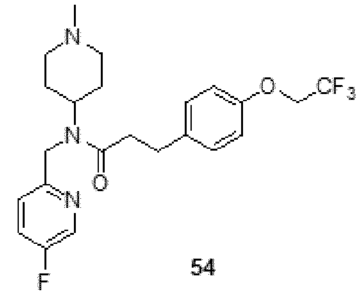
54

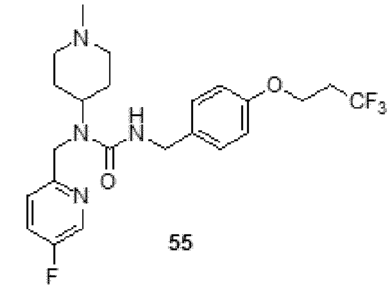
55

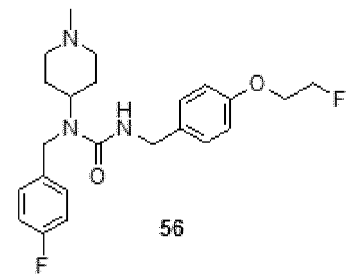
56

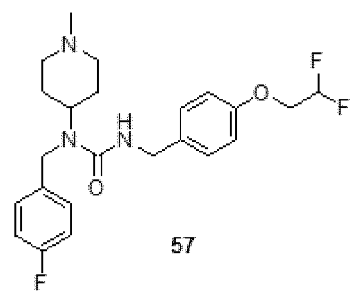
57

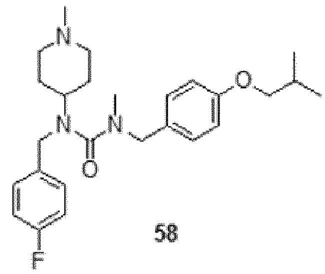
58

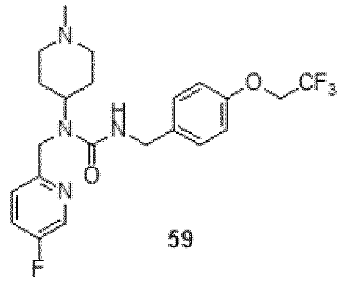
59

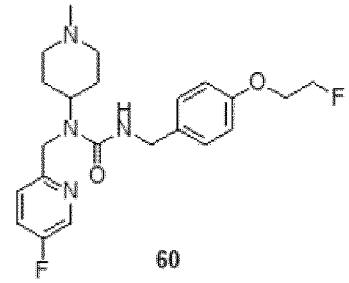
60

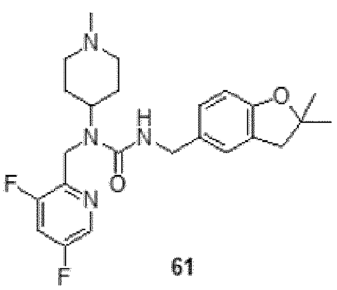
61

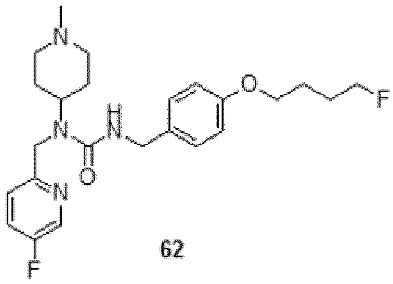
62

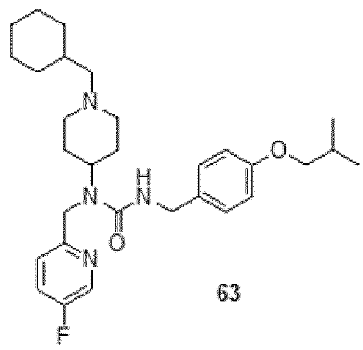
63

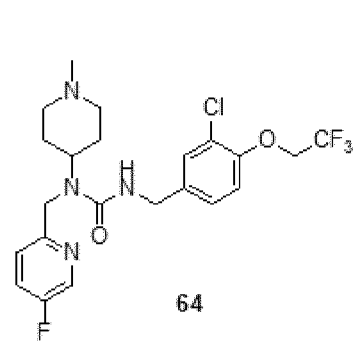
64

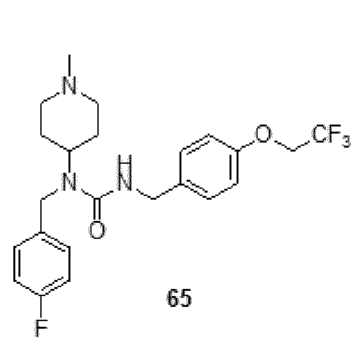
65

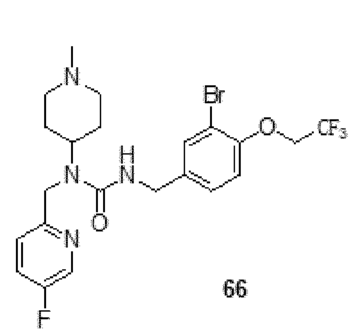
66

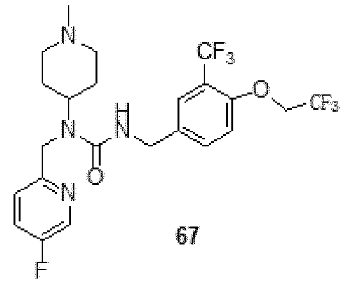
67

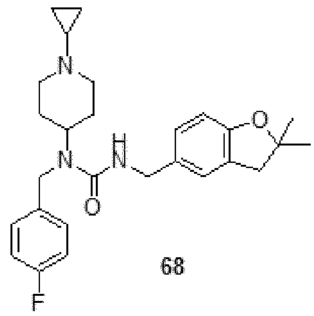
68

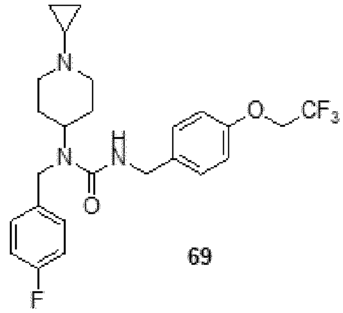
69

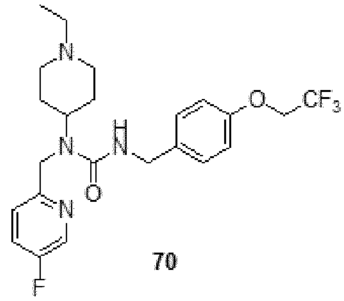
70

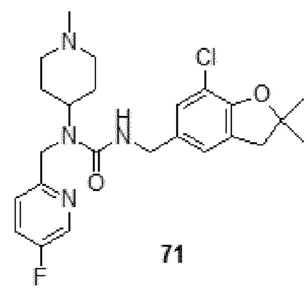
71

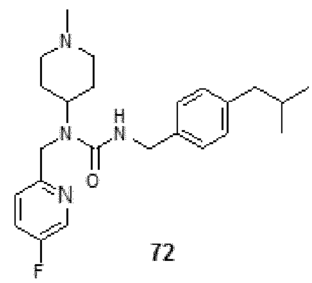
72

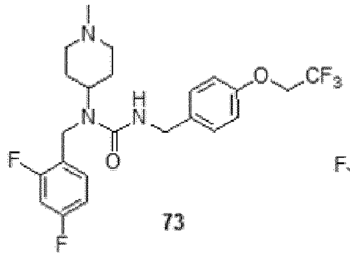
73

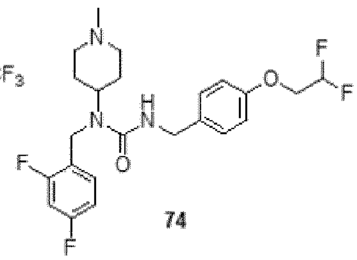
74

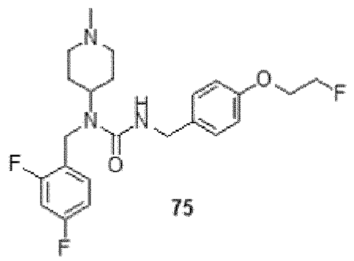
75

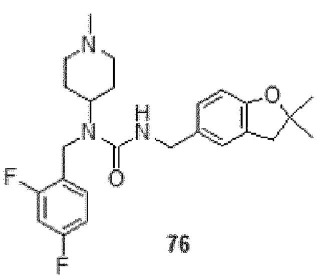
76

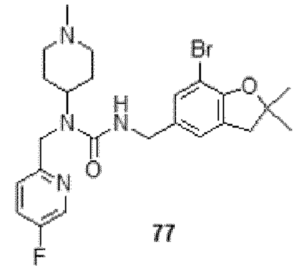
77

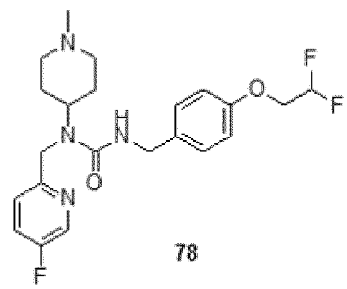
78

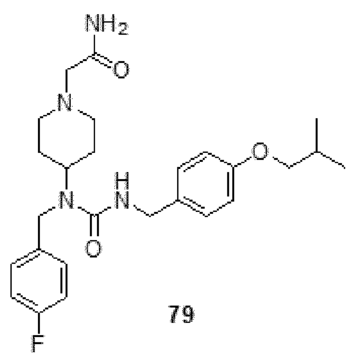
79

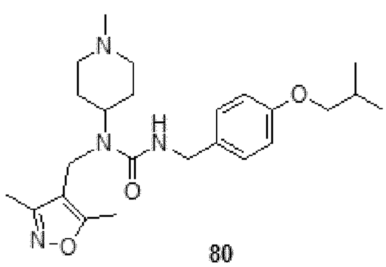
80

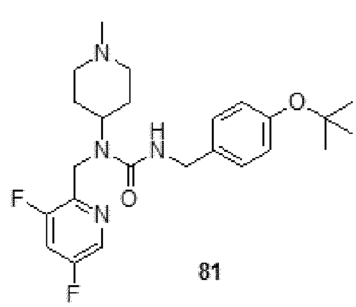
81

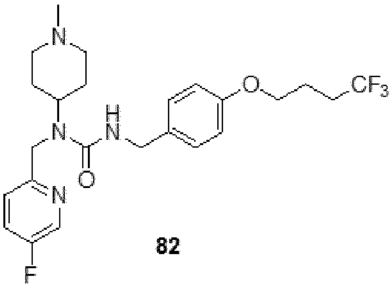

82

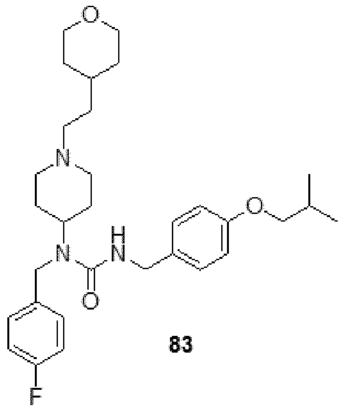

83

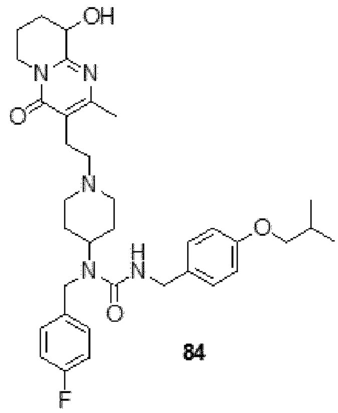

84

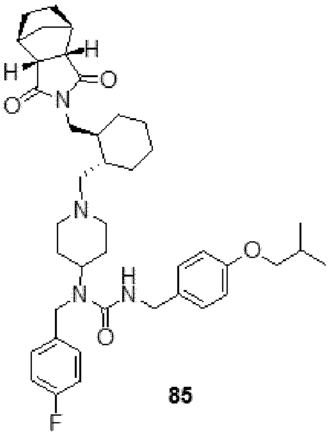

85

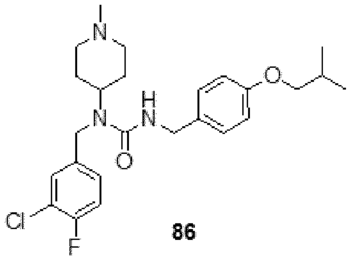

86

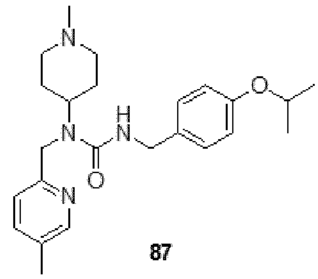

87

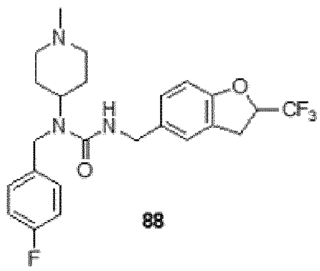

88

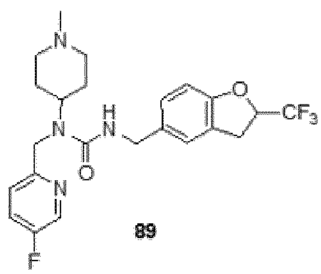

89

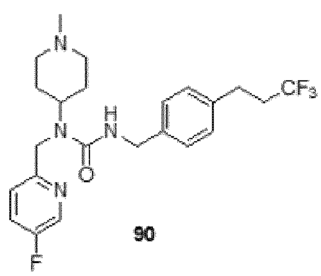

90

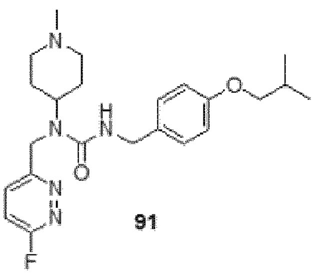

91

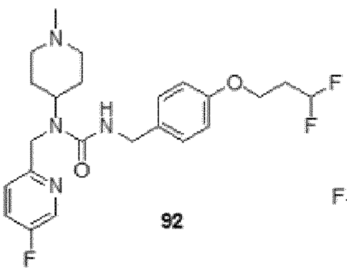

92

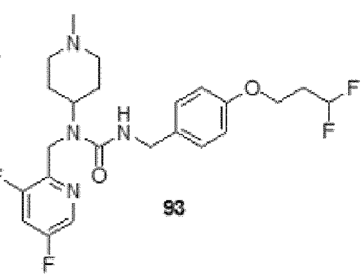

93

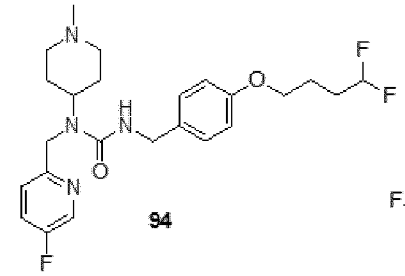

94

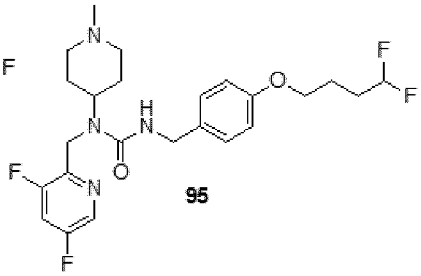

95

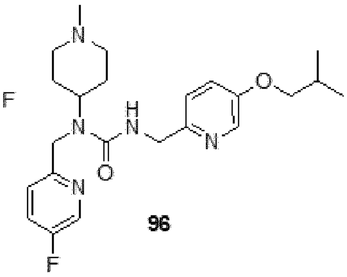

96

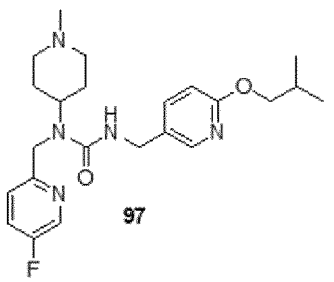

97

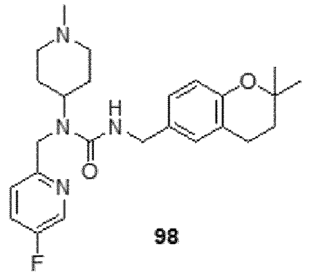

98

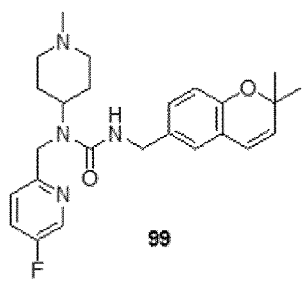

99

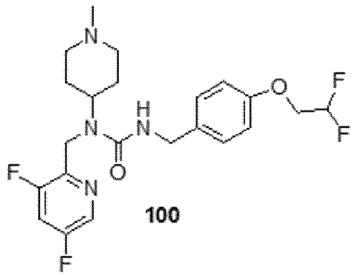

100

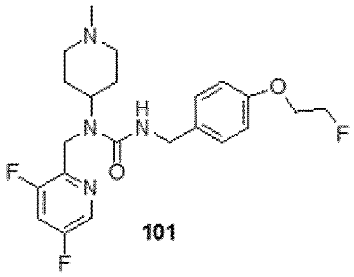

101

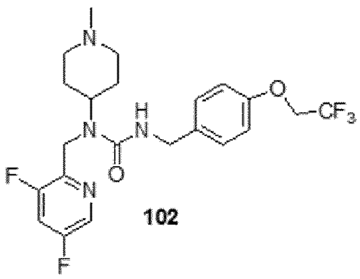

102

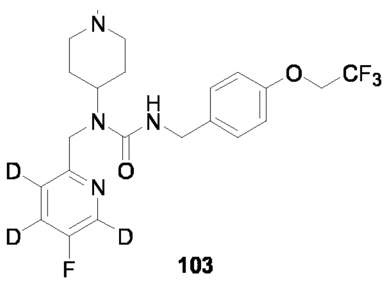

103

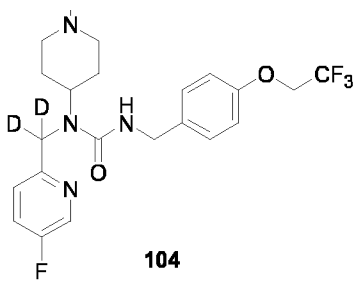

104

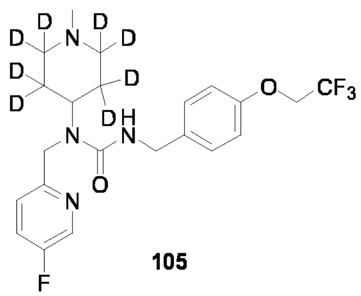

105

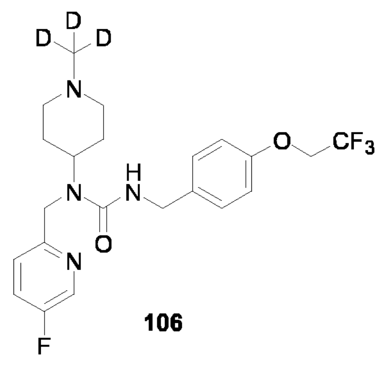

106

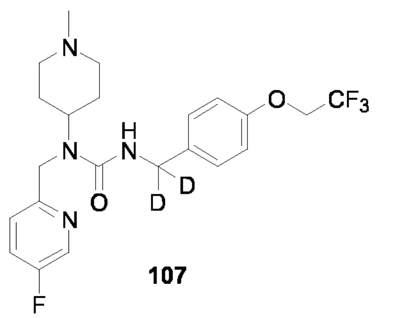

107

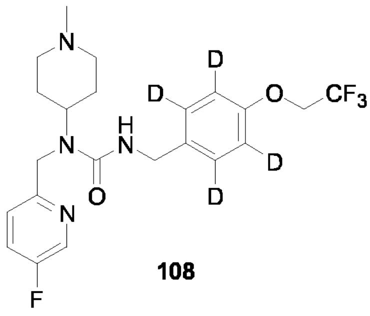

108

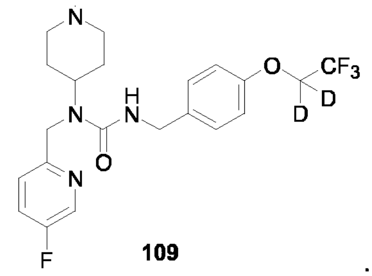

109

.

In one aspect, the present invention provides a pharmaceutical composition comprising a therapeutically effective amount of any one of the above-mentioned compounds or the stereoisomers thereof or the pharmaceutically acceptable salts thereof, or a crystalline form of any one of the above-mentioned compounds and a pharmaceutically acceptable carrier. The carrier comprises conventional auxiliary ingredients in the art, such as a filler, a binder, a diluent, a disintegrant, a lubricant, a colorant, a flavoring agent, an antioxidant and a wetting agent.

The pharmaceutical composition can be prepared into various pharmaceutically acceptable dosage forms, such as a tablet, a capsule, an oral liquid, a suspension, a granule, a powder, a microparticle, a pill, a micro-tablet, an instantly soluble film, a nasal spray, a transdermal patch, an injection or various sustained and controlled release preparations. The pharmaceutical composition can be administered orally, transmucosally, rectally or parenterally (including intravascular, intravenous, intraperitoneal, subcutaneous, intramuscular and intrasternal administration). The administration dosage can be appropriately adjusted according to the age, gender and disease type of a patient.

For oral administration, the pharmaceutical composition may be in the form of, for example, a tablet, a capsule, a liquid capsule, a suspension or a liquid. The pharmaceutical composition is preferably prepared in a dosage unit form containing a specified amount of active ingredients. For example, the pharmaceutical composition can be provided as a tablet or a capsule containing active ingredients in an amount ranging from about 0.1 mg to 1000 mg, preferably from about 0.25 mg to 250 mg, and more preferably from about 0.5 mg to 100 mg. The suitable daily dosage for humans or other mammals can vary widely depending on the patient's condition and other factors, but can be determined by means of conventional methods.

In one aspect, the present invention provides the use of any one of the above-mentioned compounds or pharmaceutically acceptable salts thereof or stereoisomers thereof in the preparation of a drug for treating 5-$HT_{2A}$ receptor-related diseases. The diseases or symptoms comprise: schizophrenia, psychosis, schizoaffective disorder, mania, psychotic depression, affective disorder, dementia, anxiety disorder, sleep disorder, dysorexia, bipolar disorder, psychosis secondary to hypertension, migraine, hypertension, thrombosis, vasospasm, ischemia, motor tics, depression, major depressive disorder, anxiety, sleep disturbance, eating disorder, non-motor symptoms caused by Parkinson's disease, delusion, illusion, cognitive disorder, dementia-related mental diseases, negative symptoms of schizophrenia, Parkinson's disease, Huntington's disease, Alzheimer's disease, spinocerebellar ataxia, Tourette's syndrome, Friedreich's ataxia, Machado-Joseph disease, Lewy body dementia, dyskinesia, dysmyotonia, myoclonus, tremor, progressive supranuclear paralysis and frontotemporal dementia, or other disease states and conditions that would have been obvious to a person skilled in the art.

## Definition and Description

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The term "pharmaceutically acceptable" as used herein refers to those compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to a salt of the compound of the present invention, which is prepared from the compound having specific substituents found in the present invention with relatively non-toxic acids or bases. When compounds of the present invention contain relatively acidic functional groups, base addition salts can be obtained by contacting the neutral form of such compounds with a sufficient amount of base, either in a pure solution or a suitable inert solvent. Examples of pharmaceutically acceptable acid addition salts include inorganic acid salts and organic acid salts, and further include salts of amino acids (e.g., arginine) and salts of organic acids (e.g., glucuronic acid) (see Berge et al., "Pharmaceutical Salts", Journal of Pharmaceutical Science 66: 1-19 (1977)). Certain specific compounds of the present invention contain basic and acidic functional groups and thus can be converted to any base

or acid addition salt.

The pharmaceutically acceptable salts of the present invention can be synthesized from a parent compound containing acid radicals or base radicals by conventional chemical methods. In general, a method for preparing such salts comprises: in water or an organic solvent or a mixture of both, reacting these compounds in free acid or base forms with a stoichiometric amount of a suitable base or acid to prepare the salts.

Certain compounds of the present invention may have asymmetric carbon atoms (optical centers) or double bonds. Racemates, diastereomers, geometric isomers and individual isomers are encompassed within the scope of the present invention.

The compounds of the present invention may exist in specific geometric or stereoisomeric forms. The present invention contemplates all such compounds, including cis and trans isomers, (-)- and (+)-enantiomers, (*R*)- and (*S*)-enantiomers, diastereomers, (*D*)-isomers, (*L*)-isomers, and racemic mixtures and other mixtures thereof, such as enantiomerically or diastereomerically enriched mixtures, all of which fall within the scope of the present invention. Additional asymmetric carbon atoms may be present in a substituent such as an alkyl group. All these isomers and mixtures thereof are encompassed within the scope of the present invention.

Optically active (*R*)- and (*S*)-isomers and *D* and *L* isomers can be prepared using chiral synthesis or chiral reagents or other conventional techniques. If a particular enantiomer of a compound of the present invention is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. In addition, separation of enantiomers and diastereomers is frequently accomplished by using chromatography, which uses chiral stationary phases, optionally in combination with chemical derivatization methods (e.g., formation of carbamates from amines).

The term "pharmaceutically acceptable carrier" refers to any preparation or carrier medium that can deliver an effective amount of active substances in the present invention, does not interfere with the biological activity of the active substances, and has no toxic or side effects on a host or patient. Representative carriers include, but are not limited to: a binder, a filler, a lubricant, a disintegrant, a wetting agent, a dispersing agent, a solubilizer, a suspending agent, etc.

For a drug or a pharmacologically active agent, the term "effective amount" or "therapeutically effective amount" refers to a sufficient amount of a drug or an agent that is nontoxic but can achieve desired effects. For the oral dosage form in the present invention, the "effective amount" of an active substance in a composition refers to an amount required to achieve desired effects when such active substance is used in combination with another active substance in the composition. Determination of the effective amount, varying from person to person, depends on the age and general condition of a recipient and also depends on a specific active substance. The appropriate effective amount in individual cases can be determined by a person skilled in the art according to conventional tests.

The present invention is intended to include all isotopes of atoms present in the compounds of the present invention. The isotopes comprise those atoms with the same atomic number but different mass numbers. By way of general example, and not as a limitation, isotopes of hydrogen comprise deuterium and tritium. Isotopes of carbon comprise $^{13}C$ and $^{14}C$. Isotope-labeled compounds of the present invention can generally be prepared by using an appropriate isotope-labeled reagent in place of a non-labeled reagent otherwise used by means of conventional techniques known to a person skilled in the art or by means of methods analogous to those described herein.

The term "deuterated analog" refers to an analog formed by substituting one or more hydrogen atoms of a compound with deuterium atoms. The term "optional" or "optionally" means that the subsequently described event or circumstance may, but not necessarily occur, and that the description includes instances where said event or circumstance occurs and instances where said event or circumstance does not occur. For example, "optionally substituted with one or more deuterium atoms" means that the group may be unsubstituted with deuterium atoms or substituted with one or more deuterium atoms, i.e., including the situation that the group is not deuterated, partially deuterated and/or fully deuterated.

The term "substituted" means that any one or more hydrogen atoms on the designated atom are substituted with a substituent, which may include heavy hydrogen and hydrogen variants, provided that the valence state of the designated atom is normal, and the substituted compound is stable. When the substituent is ketone (i.e., = O), it means that two hydrogen atoms are substituted. Ketone substitution does not occur on aromatic groups.

Where any variable (e.g., R) appears more than once in the composition or structure of a compound, its definition in each case is independent. Thus, for example, if a group is substituted with 0-2 R, the group can optionally be substituted with up to two R, and R in each case has independent options. In addition, combinations of substituents and/or variants thereof are permissible only if such combinations result in stable compounds.

Unless otherwise specified, the term "alkyl" is used to represent a linear or branched saturated hydrocarbon group, which may be monosubstituted (e.g., $-CH_2F$) or polysubstituted (e.g., $-CF_3$) and may be monovalent (e.g., methyl),

divalent (e.g., methylene) or polyvalent (e.g., methine). For example, $C_1$-$C_{10}$ represents 1 to 10 carbons, and $C_{1-10}$ is selected from $C_1$, $C_2$, $C_3$, $C_4$, $C_5$, $C_6$, $C_7$, $C_8$, $C_9$ and $C_{10}$. Examples of alkyl include methyl (Me), ethyl (Et), propyl (e.g., n-propyl and isopropyl), butyl (e.g., n-butyl, isobutyl, s-butyl and t-butyl), pentyl (e.g., n-pentyl, isopentyl, neopentyl and 1-ethylpropyl), hexyl (e.g., n-hexyl, isohexyl, 1,1-dimethylbutyl, 2,2-dimethylbutyl, 3,3-dimethylbutyl and 2-ethylbutyl), heptyl, octyl, nonyl, decyl, etc.

Unless otherwise specified, the term "halo" or "halogen" by itself or as part of another substituent means a fluorine, chlorine, bromine or iodine atom. The term "haloalkoxy" is intended to include monohaloalkoxy and linear or branched polyhaloalkoxy. For example, the term "$C_{1-10}$ haloalkoxy" is intended to include, but is not limited to, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy, 3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy and 6,6,6-trifluorohexyloxy.

The term "haloalkyl" is intended to include monohaloalkyl and linear or branched polyhaloalkyl. For example, the term "($C_1$-$C_4$) haloalkyl" is intended to include, but is not limited to, trifluoromethyl, 2,2,2-trifluoroethyl, 4-chlorobutyl, 3-bromopropyl, etc. Unless otherwise specified, examples of haloalkyl include, but are not limited to: trifluoromethyl, trichloromethyl, pentafluoroethyl and pentachloroethyl.

Unless otherwise specified, the "alkoxy" represents the above alkyl having a specific number of carbon atoms connected via an oxygen bridge. Typical alkoxy includes $C_{1-10}$ alkoxy, such as $C_1$ alkoxy, $C_2$ alkoxy, $C_3$ alkoxy, $C_4$ alkoxy, $C_5$ alkoxy, $C_6$ alkoxy, $C_7$ alkoxy, $C_8$ alkoxy, $C_9$ alkoxy and $C_{10}$ alkoxy. Examples of alkoxy include, but are not limited to: methoxy, ethoxy, n-propoxy, isopropoxy, n-butoxy, sec-butoxy, tert-butoxy, n-pentoxy, S-pentoxy, hexyloxy, 2-ethylbutoxy, heptyloxy, octyloxy, nonyloxy, decyloxy, etc.

Unless otherwise specified, cycloalkyl includes any stable cyclic or polycyclic hydrocarbon group, and any carbon atom is saturated, which may be monosubstituted or polysubstituted and may be monovalent, divalent or polyvalent. Examples of the cycloalkyl include, but are not limited to, cyclopropyl, cyclobutyl, cyclopentyl, cyclohexyl, cycloheptyl, cyclooctyl, norbornyl, [2.2.2] bicyclooctane, [4.4.0] bicyclodecane, etc.

Compounds are named by hand or ChemDraw® software, and commercially available compounds are named by the supplier catalog names.

**Brief Description of the Drawings**

Figure 1: Comparison diagram of drug-time curves of pimavanserin and compound 59 after intragastric administration

**Detailed Description of Embodiments**

The present invention is further described below in conjunction with specific examples and test examples, but the scope of the present invention is not limited in any way.

**Example 1:**

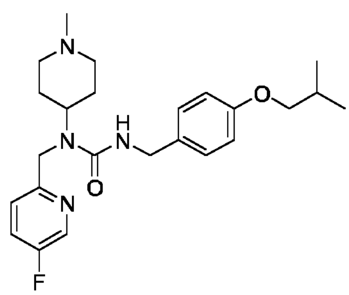

Synthesis route:

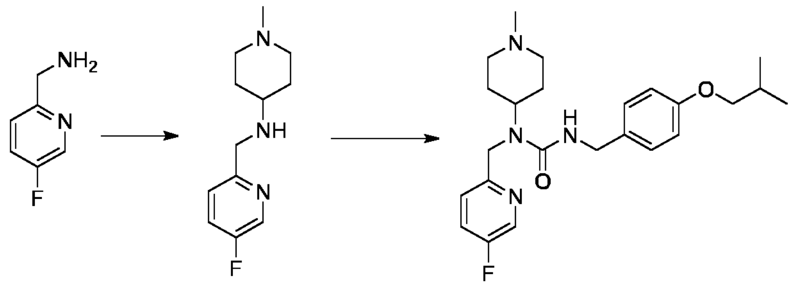

**1a** **1**

1. Under nitrogen protection, in an ice-water bath, 2-(aminomethyl)-5-fluoropyridine (504 mg, 4.0 mmol) was dissolved in 10 ml of methanol. N-methyl-4-piperidone (452 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **1a** (538 mg), which was directly used in the next reaction without purification.

2. Under nitrogen protection, compound **1a** (446 mg, 2.0 mmol) was dissolved in 10 ml of acetonitrile. N-(4-isobutyloxybenzyl)-1*H*-imidazol-formamide (546 mg, 2.0 mmol) and potassium carbonate (414 mg, 3.0 mmol) were added, and the resulting mixture was heated to 60°C and reacted under stirring for 12 h. The reaction solution was cooled to room temperature and filtered. 20 ml of water was added to the filtrate, and then the resulting solution was extracted with dichloromethane (10 mL * 3). The organic phases were combined, dried over anhydrous sodium sulfate, filtered, concentrated under reduced pressure and separated by column chromatography (dichloromethane:methanol = 10:1) to obtain **compound 1** (414 mg, a light yellow solid, yield: 48%). MS m/z (ESI): 429.3 [M+1]; $^1H$ NMR(400 MHz, $CDCl_3$) δ 8.26(d, 1H), 7.38-7.32(m, 1H), 7.31-7.28(m, 1H), 7.18-7.14(m, 2H), 6.85-6.80(m, 2H), 6.64-6.58(m, 1H), 4.37(s, 2H), 4.34(d, 2H), 3.70(d, 2H), 2.93-2.87(m, 2H), 2.28(s, 3H), 2.13-1.98(m, 4H), 1.81-1.64(m, 4H), 1.02(d, 6H).

Compounds 2-3, 12-13, 16, 19-20, 23-31, 33-52, 58, 61, 64, 66-68, 70-72, 75-77, 80-81 and 83-101 were prepared in a similar manner to example 1.

Table 1: Structures and characterization data of compounds 2-3, 12-13, 16, 19-20, 23-31, 33-52, 58, 61, 64, 66-68, 70-72, 75-77, 80-81 and 83-101

| Compound number | Structural formula | Characterization data |
|---|---|---|
| 2 | 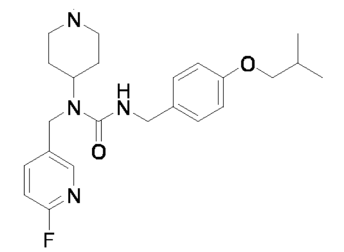 | MS m/z (ESI): 429.3 [M+1]; $^1H$ NMR(400 MHz, $CDCl_3$) δ 8.1(s, 1H), 7.69-7.65(m, 1H), 7.06-7.02(m, 2H), 6.90-6.87(m, 1H), 6.81-6.77(m, 2H), 4.69-4.60(m, 2H), 4.46(s, 2H), 4.29(d, 2H), 3.68(d, 2H), 3,45-3.38(m, 2H), 2,84-2.74(m, 2H), 2.70(s, 3H), 2.52-2.41(m, 2H), 2.09-2.02(m, 1H), 1.88-1.81(m, 2H), 1.01(d, 6H). |
| 3 | 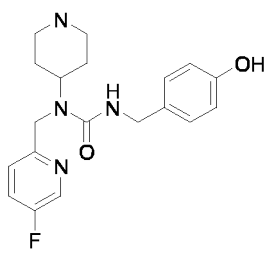 | MS m/z (ESI): 373.2[M+1]$^1H$ NMR (600 MHz, DMSO-$d_6$) δ 9.23 (s, 1H), 8.48 (d, 1H), 7.81 -7.48 (m, 1H), 7.33 (m, 1H), 7.03 (m, 3H), 6.67 (d, 2H), 4.46 (s, 2H), 4.25 -4.17 (m, 1H), 4.14 (d,2H), 2.76 (d, 2H), 2.62 -2.46 (m, 3H), 1.88 (m, ,2H), 1.68 -1.58 (m, 2H), 1.25 (d, 2H). |

(continued)

| Compound number | Structural formula | Characterization data |
|---|---|---|
| 12 | 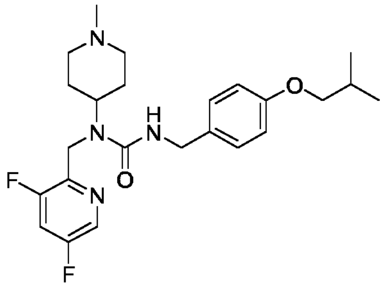 | MS m/z (ESI): 447.3 [M+1]; $^1$H NMR(400 MHz,$CDCl_3$) δ 8.15-8.13(m, 1H), 7.23-7.17(m, 3H), 6.96-6.92(m, 1H), 6.85-6.80(m, 2H), 4.46(s, 2H), 4.35-4.32(m, 2H), 4.30-4.22(m, 1H), 3.68(d, 2H), 3.04-2.98(m, 2H), 2.37(s, 3H), 2.26-2.20(m, 2H), 2.09-2.02(m, 1H), 1.96-1.88(m, 2H), 1.70-1.65(m, 2H), 1.01(d, 6H) |
| 13 | 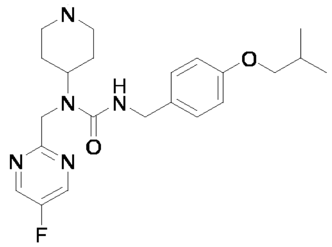 | MS m/z (ESI): 430.3 [M+1]; $^1$H NMR(400 MHz,$CDCl_3$) δ 8.48(s, 2H), 7.16-7.13(m, 2H), 6.85-6.80(m, 2H), 6.42-6.37(m, 1H), 4.56(s, 2H), 4.52-4.47(m, 1H), 4.33(d, 2H), 3.70(d, 2H), 3.40-3.36(m, 2H), 2.74-2.71(m, 1H), 2.65(s, 3H), 2.50-2.41(m, 3H), 2.09-2.02(m, 1H), 1.88-1.81(m, 2H), 1.01(d, 6H). |
| 16 | 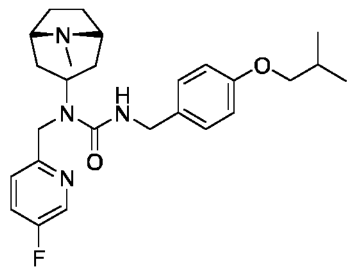 | MS m/z (ESI): 455.3 [M+1]; $^1$H NMR(400 MHz, $CDCl_3$) δ 8.23(s, 1H), 7.72-7.68(m, 1H), 7.45-7.38(m, 1H), 7.25-7.20(m, 2H), 6.87-6.84(m, 2H), 4.67-4.62(m, 1H), 4.44(s, 2H),4.35-4.30(d, 2H) 3.71(d, 2H), 3.63-3.58(m, 1H), 3.47-3.44(m, 1H), 2.74-2.71(m, 1H), 2.64-2.60(m, 2H), 2.50(s, 3H), 2.25-2.17(m, 3H), 2.13-2.05(m, 2H), 1.65-1.61(m, 2H), 1.03(d, 6H). |
| 19 | 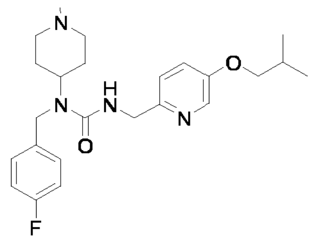 | MS m/z (ESI): 429.3 [M+1]; $^1$H NMR(400 MHz, $CDCl_3$) δ 8.10(s, 1H), 7.25-7.20(m, 2H), 7.10-7.05(m, 2H), 6.99-6.89(m, 2H), 5.50-5.42(m, 1H), 4.45-4.35(m, 4H), 4.30-4.20(m, 1H), 3.70(d, 2H), 2.88-2.85(m, 2H), 2.26(s, 3H), 2.09-2.00(m, 3H), 1.75-1.60(m, 4H), 1.02(d, 6H). |
| 20 | 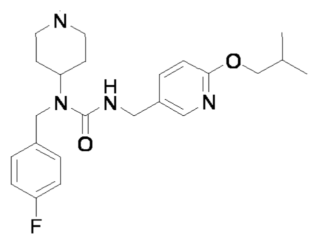 | MS m/z (ESI): 429.3 [M+1]; $^1$H NMR(400 MHz, $CDCl_3$) δ 7.91(s, 1H), 7.38-7.33(m, 1H), 7.24-7.20(m, 2H), 7.05-6.95(m, 2H), 6.69-6.65(m, 1H), 4.55-4.45(m, 1H), 4.40-4.20(m, 5H), 3.99(d, 2H), 2.98(d, 2H), 2.30(s, 3H), 2.19-2.00(m, 3H), 1.75-1.65(m, 4H), 1.01(d, 6H). |
| 21 | 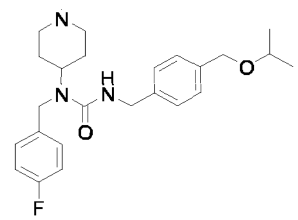 | MS m/z (ESI): 428.3 [M+1]; $^1$H NMR(400 MHz, $CDCl_3$) δ 7.25-7.15(m, 4H), 7.10-6.97(m, 4H), 4.55-4.45(m, 1H), 4.42(s, 2H), 4.40-4.25(m, 5H), 3.70-3.63(m, 1H), 2.88(d, 2H), 2.28(s, 3H), 2.19-2.09(m, 2H), 1.75-1.65(m, 4H), 1.01(d, 6H). |

(continued)

| Compound number | Structural formula | Characterization data |
|---|---|---|
| 23 | 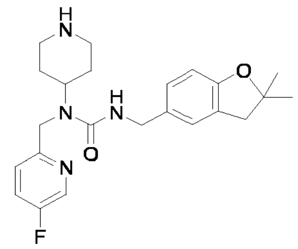 | MS m/z (ESI): 413.2 [M+1]; $^1$H NMR(400 MHz, $CDCl_3$) δ 9.49-9.34(m, 2H), 8.26(s, 1H), 7.41-7.38(m, 2H), 7.07-7.02(m, 2H), 6.68-6.66(m, 1H), 4.58-4.44(m, 1H), 4.41-4.32(m, 4H), 3.45(d, 2H), 3.00-2.88(m, 4H), 2.30-2.24(m, 2H), 1.75-1.68(m, 2H), 1.47(s, 6H). |
| 24 | 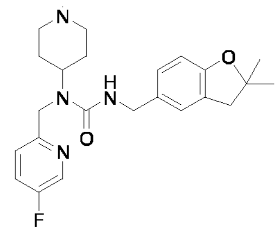 | MS m/z (ESI): 427.2 [M+1]; $^1$H NMR(400 MHz, $CDCl_3$) δ 8.51(s, 1H), 7.62-7.54(m, 2H), 7.24-7.20(m, 1H), 6.89-6.77(m, 2H), 4.63(s, 2H), 4.58-4.44(m, 3H), 3.25-3.10(m, 4H), 2.80-2.71(m, 1H), 2.52(s, 3H), 2.30-2.24(m, 2H), 2.08-1.88(m, 4H), 1.70(s, 6H). |
| 25 | 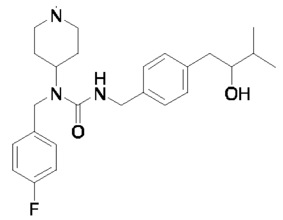 | MS m/z (ESI): 442.3 [M+1]; $^1$H NMR(400 MHz, DMSO-$d_6$) δ 7.25-7.21(m, 2H), 7.15-7.03(m, 6H), 6.94-6.82(m, 1H), 4.42(s, 2H), 4.38-4.29(m, 1H), 4.24-4.18(m, 2H), 3.94-3.81(m, 1H), 3.48-3.32(m, 1H), 2.74(d, 2H), 2.70-2.58(m, 2H), 2.10(s, 3H), 1.90-1.75(m, 2H), 1.59-1.41(m, 5H), 0.87(d, 6H). |
| 26 | 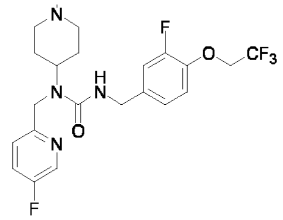 | MS m/z (ESI): 473.2 [M+1]; $^1$H NMR(400 MHz, $CDCl_3$) δ 8.28(d, 1H), 7.88(s, 1H), 7.70-7.65(m, 1H), 7.46-7.42 (m, 1H), 7.13-7.08(m, 1H), 7.05-6.97(m, 2H), 4.60-4.53 (m, 1H), 4.50-4.45(s, 2H), 4.43-4.37(m, 4H), 3.46-3.40 (m, 2H), 2.80-2.69(m, 5H), 2.67-2.58(m, 2H), 1.79-1.73 (m, 2H). |
| 27 | 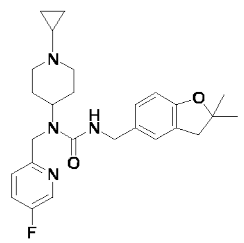 | MS m/z (ESI): 453.3 [M+1]; $^1$H NMR(400 MHz, $CDCl_3$) δ 8.27(s, 1H), 7.42-7.34(m, 2H), 7.05-6.98(m, 2H), 6.67-6.64(m, 1H), 4.43-4.34(m, 5H), 3.25-3.10(m, 2H), 3.00-2.95(m, 2H), 2.50-2.31(m, 2H), 1.82-1.60(m, 5H), 1.48(s, 6H), 0.63-0.35(m, 4H). |
| 28 | 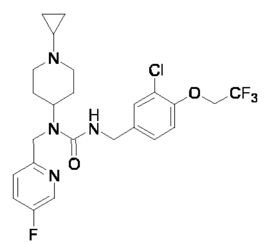 | MS m/z (ESI): 515.2 [M+1]; $^1$H NMR(400 MHz, $CDCl_3$) δ 8.33(d, 1H), 7.44-7.29(m, 3H), 7.17-7.13(m, 1H), 6.94-6.90(m, 1H), 4.50-4.44(m, 3H), 4.42-4.34(m, 4H), 3.26-3.10(m, 2H), 2.40-2.30(m, 2H), 1.89-1.63(m, 5H), 0.58-0.38(m, 4H). |

(continued)

| Compound number | Structural formula | Characterization data |
|---|---|---|
| 29 | 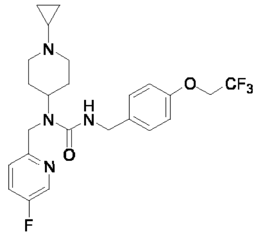 | MS m/z (ESI): 480.2 [M+1]; $^1$H NMR(400 MHz, $CDCl_3$) δ 8.38(d, 1H), 7.45-7.28(m, 4H), 7.22-7.16(m, 1H), 6.99-6.90(m, 1H), 4.43-4.24(m, 4H), 3.96-3.90(m, 2H), 3.17-3.08(m, 2H), 2.40-2.30(m, 2H), 1.95-1.85(m, 1H), 1.79-1.60(m, 5H), 0.52-0.40(m, 4H). |
| 30 | 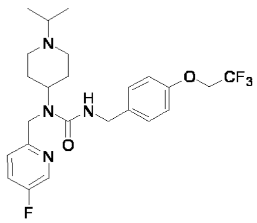 | MS m/z (ESI): 473.2 [M+1]; $^1$H NMR(400 MHz, $CDCl_3$) δ 8.23(d, 1H), 7.98(s, 1H), 7.80-7.75(m, 1H), 7.43-7.40 (m, 1H), 7.32-7.27(m, 2H), 6.95-6.90(m, 2H), 4.61-4.55 (m, 1H), 4.53-4.49(s, 2H), 4.41-4.32(m, 4H), 3.44-3.38 (m, 3H), 2.90-2.78(m, 4H), 1.79-1.73(m, 2H), 1.42(d, 6H). |
| 31 | 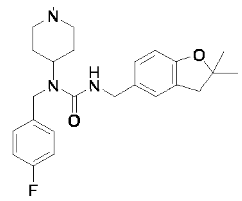 | MS m/z (ESI): 426.2 [M+1]; $^1$H NMR(400 MHz, $CDCl_3$) δ 7.22-7.17(m, 2H), 7.02-6.98(m, 2H), 6.84-6.81(m, 2H), 6.62-6.58(m, 1H), 4.55-4.47(m, 1H), 4.37(s, 2H), 4.26-4.24(m, 2H), 3.15-3.08(m, 2H), 2.96-2.91(m, 2H), 2.45(s, 3H), 2.40-2.34(m, 2H), 2.04-1.94(m, 2H), 1.82-1.76(m, 2H), 1.46(s, 6H). |
| 33 | 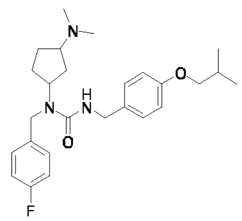 | MS m/z (ESI): 442.3 [M+1]; $^1$H NMR(400 MHz, DMSO-$d_6$) δ 7.28-7.22(m, 2H), 7.19-7.08(m, 4H), 6.88-6.76(m, 2H), 4.58-4.41(m, 4H), 4.24-4.20(m, 2H), 3.71(d, 2H), 2.35-2.20(m, 2H), 2.17(s, 6H), 2.06-1.92(m, 3H), 1.70-1.52(m, 2H), 1.44-1.38(m, 1H), 0.97(d,6H). |
| 34 | 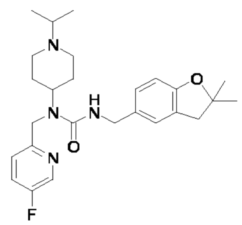 | MS m/z (ESI): 455.3 [M+1]; $^1$H NMR(400 MHz, DMSO-$d_6$) δ 8.47(s, 1H), 7.72-7.64(m, 1H), 7.35-7.28(m, 1H), 7.07-6.94(m, 3H), 6.68-6.65(m, 1H), 4.50(s, 1H), 4.23-4.04(m, 3H), 2.95(s, 2H), 2.80-2.60(m, 3H), 2.30-2.11(m, 2H), 1.62-1.45(m, 4H), 1.38(s, 6H), 0.97(d, 6H). |
| 35 | 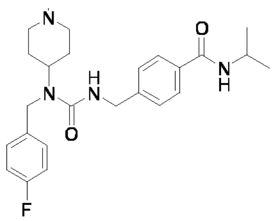 | MS m/z (ESI): 441.3 [M+1]; $^1$H NMR(400 MHz, DMSO-$d_6$) δ 8.21-8.12(m, 1H), 7.82-7.76(m, 2H), 7.26-7.20(m, 4H), 7.12-7.01(m, 3H), 4.45-4.29(m, 4H), 4.18-3.94(m, 2H), 2.78-2.68(m, 2H), 2.11(s, 3H), 1.87-1.65(m, 2H), 1.60-1.42(m, 4H), 0.99(d, 6H). |

(continued)

| Compound number | Structural formula | Characterization data |
|---|---|---|
| 36 | 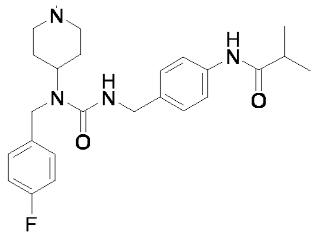 | MS m/z (ESI): 441.3 [M+1]; $^1$H NMR(400 MHz, DMSO-*d*$_6$) δ 9.76(s, 1H), 7.52-7.46(m, 2H), 7.26-7.22(m, 2H), 7.12-7.01(m, 4H), 6.93-6.88(m, 1H), 4.45-4.39(m, 2H), 4.18-4.14(m, 2H), 3.99-3.90(m, 1H), 2.78-2.73(m, 2H), 2.60-2.52(m, 1H), 2.15(s, 3H), 2.01-1.88(m, 2H), 1.61-1.42(m, 4H), 1.01 (d, 6H). |
| 37 | 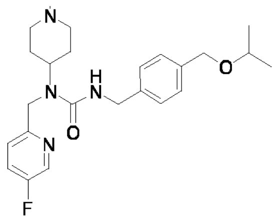 | MS m/z (ESI): 429.3 [M+1]; $^1$H NMR(400 MHz, DMSO-*d*$_6$) δ 8.46(d, 1H), 7.68-7.62(m, 1H), 7.31-7.28(m, 1H), 7.22-7.10(m, 6H), 4.50-4.40(m, 4H), 4.24(d, 2H), 4.00-3.87(m, 1H), 3.70-3.60(m, 1H), 2.83-2.77(m, 2H), 2.21(s, 3H), 2.03-1.88(m, 2H), 1.61-1.44(m, 4H), 1.12(d, 6H). |
| 38 | 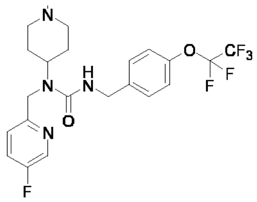 | MS m/z (ESI): 491.2 [M+1]; $^1$H NMR(400 MHz, DMSO-*d*$_6$) δ 8.49(d, 1H), 7.66-7.61(m, 1H), 7.36-7.18(m, 6H), 4.50(s, 2H), 4.36-4.30(m, 2H), 4.00-3.87(m, 1H), 2.81-2.72(m, 2H), 2.11(s, 3H), 2.03-1.88(m, 2H), 1.63-1.45(m, 4H). |
| 39 | 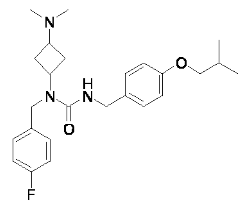 | MS m/z (ESI): 428.3 [M+1]; $^1$H NMR(400 MHz, $CDCl_3$) δ 7.25-7.02(m, 7H), 6.88-6.76(m, 2H), 4.58-4.41(m, 4H), 4.26-4.21(m, 2H), 3.65(d, 2H), 2.45-2.30(m, 3H), 2.13(s, 6H), 2.06-1.99(m, 1H), 1.44-1.38(m, 1H), 0.97(d,6H). |
| 40 | 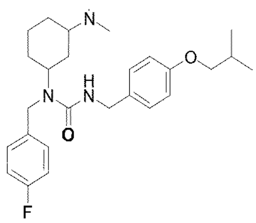 | MS m/z (ESI): 456.3 [M+1]; $^1$H NMR(400 MHz, DMSO-*d*$_6$) δ 7.27-7.23(m, 2H), 7.20-7.06(m, 4H), 6.85-6.76(m, 2H), 4.44-4.25(m, 3H), 4.20-4.07(m, 2H), 3.73(d, 2H), 3.30-3.21(m, 1H), 2.17(s, 6H), 2.06-1.97(m, 1H), 1.80-1.52(m, 4H), 1.47-1.31(m, 3H), 0.98(d,6H). |
| 41 | 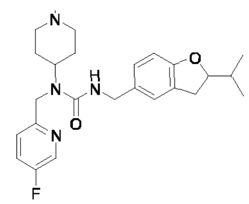 | MS m/z (ESI): 441.3 [M+1]; $^1$H NMR(400 MHz, $CDCl_3$) δ 8.23(d, 1H), 7.90-7.60(s, 1H), 7.50-7.32(m, 3H), 7.08-6.86(m, 2H), 6.65-6.60(m, 1H), 4.50-4.31(m, 4H), 4.26-4.21(m, 2H), 3.25(d, 2H), 3.12-2.92(m, 2H), 2.69-2.55(m, 4H), 2.46-2.29(m, 2H), 1.84-1.68(m, 3H), 0.95(d,6H). |
| 42 | 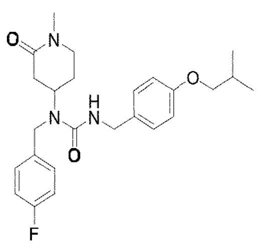 | MS m/z (ESI): 442.2 [M+1]; $^1$H NMR(400 MHz, DMSO-*d*$_6$) δ 7.28-7.12(m, 6H), 7.01-6.95(m, 1H), 6.88-6.79(m, 2H), 4.48-4.31(m, 3H), 4.26-4.21(m, 2H), 3.72(d, 2H), 3.25-3.15(m, 2H), 2.75(s, 3H), 2.35-2.20(m, 2H), 2.01-1.79(m, 2H), 1.64-1.58(m, 1H), 0.95(d,6H). |

(continued)

| Compound number | Structural formula | Characterization data |
|---|---|---|
| 43 | 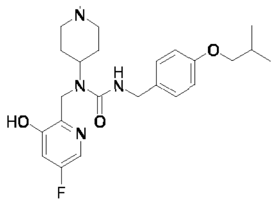 | MS m/z (ESI): 445.2 [M+1]; $^1$H NMR(400 MHz, $CDCl_3$) δ 7.98(d, 1H), 7.48-7.36(m, 3H), 7.01-6.95(m, 3H), 4.68-4.54(m, 4H), 4.01-3.85(m, 3H), 3.22(d, 2H), 2.55(s, 3H), 2.39-2.20(m, 5H), 1.79-1.73(m, 2H), 1.15(d,6H). |
| 44 | 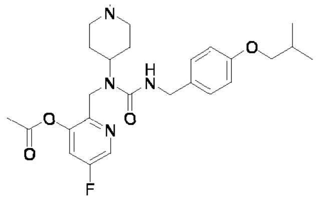 | MS m/z (ESI): 487.3 [M+1]; $^1$H NMR(400 MHz, $CDCl_3$) δ 8.22(d, 1H), 7.38-7.32(m, 1H), 7.21-7.05(m, 3H), 6.80-6.73(m, 2H), 4.48-4.34(m, 5H), 3.71(d, 2H), 3.52(d, 2H), 2.95-2.75(m, 5H), 2.45(s, 3H), 2.39-2.33(m, 2H), 2.12-2.00(m, 1H), 1.79-1.73(m, 2H), 1.00(d,6H). |
| 45 | 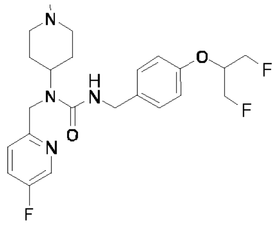 | MS m/z (ESI): 451.2 [M+1]; $^1$H NMR(400 MHz, DMSO-*d*$_6$) δ 8.48(d, 1H), 7.70-7.65(m, 1H), 7.32-7.27(m, 1H), 7.18-7.07(m, 3H), 6.95-6.88(m, 2H), 4.94-4.60(m, 5H), 4.44(s, 2H), 4.23(d, 2H), 3.96-3.86(m, 1H), 2.72(d, 2H), 2.12(s, 3H), 1.89-1.75(m, 2H), 1.55-1.43(m, 4H). |
| 46 | 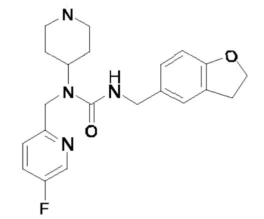 | MS m/z (ESI): 399.2 [M+1]; $^1$H NMR(400 MHz, $CDCl_3$) δ 8.21(d, 1H), 7.35-7.30(m, 2H), 7.10-6.92(m, 3H), 6.65-6.60(m, 1H), 4.52-4.41(m, 2H), 4.36-4.14(m, 5H), 3.15-2.99(m, 4H), 2.42(s, 3H), 2.35-2.20(m, 2H), 2.06-1.86(m, 2H), 1.64-1.58(m, 2H). |
| 47 | 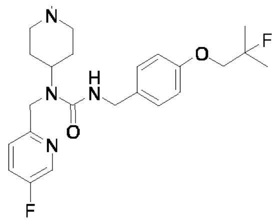 | MS m/z (ESI): 447.3 [M+1]; $^1$H NMR(400 MHz, MeOD-*d*$_4$) δ 8.38(d, 1H), 7.55-7.48(m, 1H), 7.32-7.28(m, 1H), 7.25-7.17(m, 2H), 6.85-6.78(m, 2H), 4.51(s, 2H), 4.27(s, 2H), 4.20-4.11(m, 1H), 3.92(s, 2H), 2.98(d, 2H), 2.34(s, 3H), 2.28-2.22(m, 2H), 1.79-1.65(m, 4H), 1.45(d, 6H). |
| 48 | 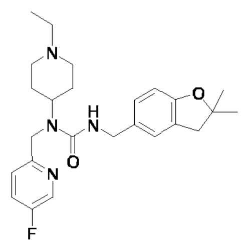 | MS m/z (ESI): 441.3 [M+1]; $^1$H NMR(400 MHz, $CDCl_3$) δ 8.26(s, 1H), 7.42-7.34(m, 2H), 7.04-6.90(m, 2H), 6.75-6.65(m, 2H), 4.43(s, 2H), 4.35-4.27(m, 3H), 3.10(d, 2H), 2.95(s, 2H), 2.60-2.51(m, 2H), 2.20-2. 11(m, 2H), 1.98-1.78(m, 2H), 1.74-1.67(m, 2H), 1.48(s, 6H), 1.14(t, 3H). |
| 49 | 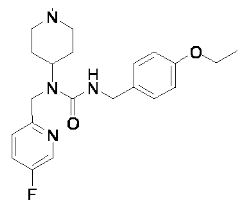 | MS m/z (ESI): 401.2 [M+1]; $^1$H NMR(400 MHz, $CDCl_3$) δ 8.25(d, 1H), 7.39-7.29(m, 2H), 7.21-7.13(m, 2H), 6.85-6.80(m, 2H), 6.74-6.64(m, 1H), 4.37-4.24(m, 5H), 3.99(q, 2H), 2.93-2.85(d, 2H), 2.27(s, 3H), 2.13-1.98(m, 2H), 1.81-1.64(m, 4H), 1.42(t, 3H). |

(continued)

| Compound number | Structural formula | Characterization data |
|---|---|---|
| 50 | 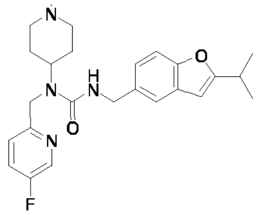 | MS m/z (ESI): 439.3 [M+1]; $^1$H NMR(400 MHz, $CDCl_3$) δ 8.25(d, 1H), 7.40-7.32(m, 4H), 7.09-7.06(m, 1H), 6.79-6.70(m, 1H), 6.29(s, 1H), 4.50-4.44(d, 2H), 4.41-4.25(m, 3H), 3.12-3.04(m, 1H), 2.93(d, 2H), 2.30(s, 3H), 2.16-2.09(m, 2H), 1.84-1.65(m, 4H), 1.29(d,6H). |
| 51 | 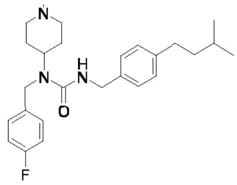 | MS m/z (ESI): 426.2 [M+1]; $^1$H NMR(400 MHz, $CDCl_3$) δ 7.22-7.16(m, 2H), 7.09-6.88(m, 7H), 4.54-4.47(m, 1H), 4.36-4.24(m, 5H), 2.91(d, 2H), 2.65-2.52(m, 2H), 2.27(s, 3H), 2.20-2.05(m, 2H), 1.80-1.66(m, 4H), 1.60-1.42(m, 3H), 0.91(d, 6H). |
| 52 | 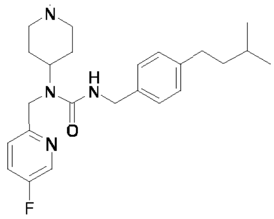 | MS m/z (ESI): 427.2 [M+1]; $^1$H NMR(400 MHz, $CDCl_3$) δ 8.24(d, 1H), 7.55-7.50(m, 1H), 7.45-7.35(m, 2H), 7.20-7.10(m, 4H), 4.55-4.47(m, 1H), 4.42-4.34(m, 4H), 3.28(d, 2H), 2.65-2.52(m, 7H), 2.40-2.31(m, 2H), 1.76-1.70(m, 2H), 1.60-1.45(m, 3H), 0.93(d, 6H). |
| 58 | 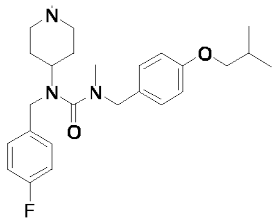 | MS m/z (ESI): 442.3 [M+1]; $^1$H NMR(400 MHz, DMSO-$d_6$) δ 7.28-7.24(m, 2H), 7.15-6.97(m, 4H), 6.85-6.75(m, 2H), 4.25-4.15(m, 4H), 3.70(d, 2H), 2.80-2.70(m, 2H), 2.63(s, 3H), 2.08(s, 3H), 2.05-1.90(m, 2H), 1.88-1.68(m, 4H), 1.61-1.49(m, 2H), 0.99(d, 6H). |
| 61 | 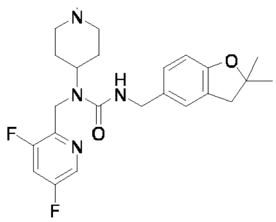 | MS m/z (ESI): 445.2[M+1],467.23[M+23]$^1$H NMR (600 MHz, $CDCl_3$) δ 8.13 (d,1H), 7.23 (m, 1H), 7.04 (d, 1H), 7.01 -6.98 (m, 1H), 6.93 (s, 1H), 6.65 (d, 1H), 4.48 (d, 2H), 4.30 (d, 2H), 3.48 (s, 1H), 3.34 (d, 2H), 2.97 (s, 2H), 2.62 (s, 5H), 2.37 - 2.30 (m, 2H), 1.82 -1.76 (m, 2H), 1.46 (s, 6H). |
| 64 | 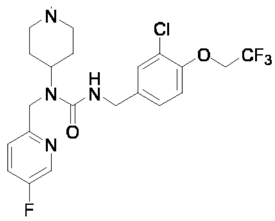 | MS m/z (ESI): 489.2[M+1]$^1$H NMR (600 MHz, DMSO-$d_6$) δ 8.49 (d, 1H), 7.74 -7.56 (m, 1H), 7.33 (m, 1H), 7.22 - 7.16 (m, 3H), 7.02 (s, 1H), 4.83 (d, 2H), 4.48 (s, 2H), 4.19 (d, 2H), 4.13 (d, 1H), 3.43 -3.28 (m, 2H), 3.09 -2.96 (m, 2H), 2.41 (s, 3H), 1.83 -1.75 (m, 2H), 1.69 - 1.48 (m, 2H). |
| 66 | 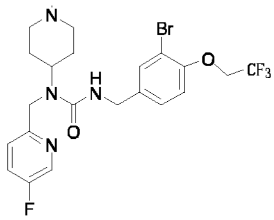 | MS m/z (ESI): 534.2[M+1]$^1$H NMR (600 MHz, DMSO-$d_6$) δ 8.48 (m, 1H), 7.71 -7.60 (m, 1H), 7.32 (m, 1H), 7.26 - 7.11 (m, 3H), 7.02 -6.94 (m, 1H), 4.82 (d, 1H), 4.71 (d , 1H), 4.47 (d, 2H), 4.19 (t, 2H), 4.06 (s, 1H), 3.32 (s, 1H), 2.94 (s, 2H), 2.31 (s, 4H), 1.70 (d, 2H), 1.52 (d, 2H). |

(continued)

| Compound number | Structural formula | Characterization data |
|---|---|---|
| 67 | 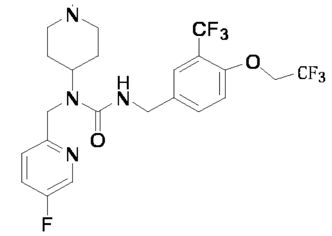 | MS m/z (ESI): 523.4[M+1], 545.2[M+23] $^1$H NMR (600 MHz, DMSO-$d_6$) $\delta$ 8.48 (s, 1H), 7.65 (m, 1H), 7.54 -7.47 (m, 1H), 7.35 -7.29 (m, 1H), 6.97 (m, 3H), 4.91 (d, 1H), 4.70 (d, 1H), 4.48 (d, 2H), 4.24 (d, 1H), 4.17 (d, 1H), 4.07 (s, 1H), 3.37 -3.30 (m, 1H), 3.07 (s, 2H), 2.43 (s, 3H), 2.13 (s, 1H), 1.79 (d, 2H), 1.58 (d, 2H). |
| 68 | 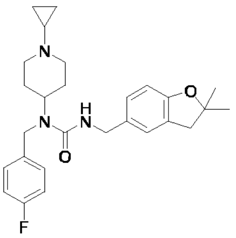 | MS m/z (ESI): 452.5 [M+1]$^1$ H NMR (600 MHz, DMSO-$d_6$) $\delta$ 7.22 (m, 2H), 7.15 -7.01 (m, 2H), 6.98 -6.87 (m, 2H), 6.81 (t, 1H), 6.58 (d, 1H), 4.39 (s, 2H), 4.15 (d, 2H), 4.05 (d, 1H), 2.95 (d, 3H), 2.88 (s, 2H), 2.78 (s, 1H), 2.16 (s, 1H), 1.44 (s, 4H), 1.38 (d, 10H). |
| 70 | 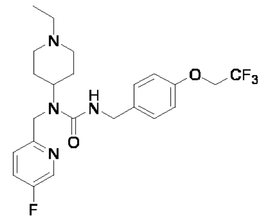 | MS m/z (ESI): 469.1[M+1]$^1$ H NMR (600 MHz, DMSO-$d_6$) $\delta$ 8.49 (d, 1H), 7.69 (d, 1H), 7.34 (d, 1H), 7.23 -7.15 (m, 3H), 7.03 -6.94 (m, 2H), 4.71 (t, 2H), 4.48 (s, 2H), 4.20 (d, 3H), 3.32 (s, 2H), 2.81(m,2H),2.50 (m, 2H), 2.00 - 1.81 (m, 2H), 1.62 (s, 2H), 1.18 -1.07 (m, 3H). |
| 71 | 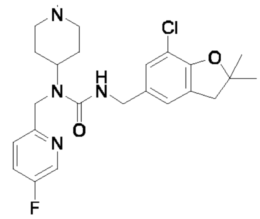 | MS m/z (ESI): 461.1[M+1], 483.0[M+23] $^1$H NMR (600 MHz, Methanol-$d_4$) $\delta$ 8.39 (d, 1H), 7.57 (d, 1H), 7.42 (m, 1H), 6.97 (d, 2H), 4.56 (s, 2H), 4.34 -4.26 (m, 1H), 4.24 (s, 2H), 3.40 -3.34 (m, 2H), 3.06 (s, 2H), 2.90 (s, 2H), 2.71 (s, 3H), 2.05 -1.96 (m, 2H), 1.89 -1.83 (m, 2H), 1.47 (s, 6H). |
| 72 | 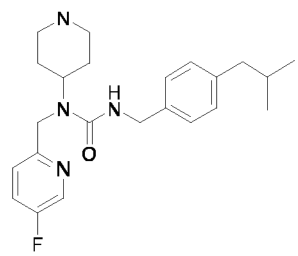 | MS m/z (ESI): 413.4[M+1], 435.4[M+23] $^1$H NMR (600 MHz, DMSO-$d_6$) $\delta$ 8.48 (d, 1H), 7.68 (m, 1H), 7.35 (m, 1H), 7.16 (t, 1H), 7.11 (d, 2H), 7.06 (d, 2H), 4.48 (s, 2H), 4.26 (d, 1H), 4.23 (d, 2H), 3.28 -3.16 (m, 2H),2.82 (s,2H), 2.57 (s, 3H), 2.40 (d, 2H), 2.06 - 1.88 (m, 2H), 1.79 (m, 1H), 1.70 -1.59 (m, 2H), 0.84 (d, 6H). |
| 75 | 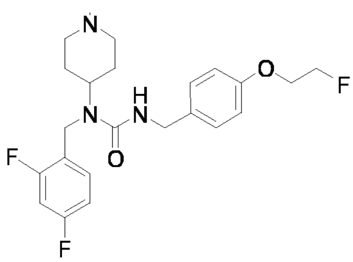 | MS m/z (ESI): 436.4[M+1]$^1$H NMR (600 MHz, DMSO-$d_6$) $\delta$ 7.24 -7.17 (m, 2H), 7.17 -7.13 (m, 2H), 7.11 (t, 1H), 7.03 (m, 1H), 6.91 -6.85 (m, 2H), 4.79 - 4.74 (m, 1H), 4.70 -4.67 (m, 1H), 4.41 (s, 2H), 4.24 -4.21 (m, 1H), 4.19 (s, 2H), 4.17 (d, 2H), 3.23 -3.08 (m, 2H), 2.51 (m, 3H), 2.49(s,2H), 1.85 (s, 2H), 1.66 -1.59 (m, 2H). |

(continued)

| Compound number | Structural formula | Characterization data |
|---|---|---|
| 76 | 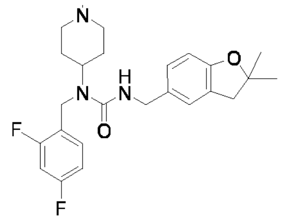 | MS m/z (ESI): 444.3[M+1]$^1$H NMR (600 MHz, DMSO-$d_6$) $\delta$ 7.25 -7.15 (m, 2H), 7.03 (m, 2H), 6.99 (d, 1H), 6.94 - 6.86 (m, 1H), 6.58 (d, 1H), 4.41 (s, 2H), 4.27 -4.18 (m, 1H), 4.15 (d, 2H), 3.17 (d, 2H), 2.94 (s, 2H), 2.51 (m,3H), 2.47 (m, 2H), 1.85 (d, 2H), 1.61 (d,2H), 1.38 (s, 6H). |
| 77 | 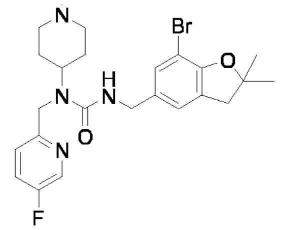 | MS m/z (ESI): 505.3[M+1]$^1$H NMR (600 MHz, DMSO-$d_6$) $\delta$ 8.48 (d,1H), 7.68 (m, 1H), 7.33 (m, 1H), 6.97 (d, 1H), 6.91 (m, 1H), 6.58 (d, 1H), 4.46 (s, 2H), 4.24 -4.17 (m, 1H), 4.15 (d, 2H), 3.16 (d, 2H), 2.94 (s, 2H), 2.55 -2.51 (m, 2H), 2.50 (m, 3H), 1.89 (s, 2H), 1.62 (d, 2H), 1.38 (s, 6H). |
| 80 | 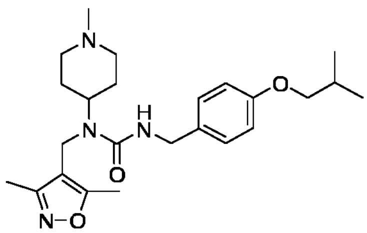 | MS m/z (ESI): 429.2[M+1]$^1$H NMR (600 MHz, $CDCl_3$) $\delta$ 7.16 (m, 2H), 6.89 -6.68 (m, 2H), 4.63 (t, 1H), 4.34 (d, 2H), 4.21 -4.18 (m, 2H),3.91(m,1H) ,3.69 (m, 2H), 2.88 (m,2H),2.26 (s, 2H), 2.22 (s, 3H), 2.17 (s, 3H), 2.04 (m, 2H), 1.68 (d, 4H), 1.01 (d, 7H). |
| 81 | 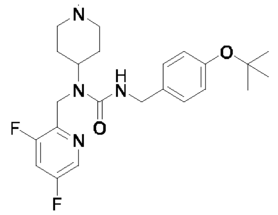 | MS m/z (ESI): 447.0[M+1]$^1$H NMR (600 MHz, $CDCl_3$) $\delta$ 8.11 (d, 1H), 7.22 (m, 1H), 7.19 -7.14 (m, 2H), 7.04 (t, 1H), 6.95 -6.89 (m, 2H), 4.49 (d, 2H), 4.38 (d, 3H), 3.24 (s,2H),2.55 (s, 5H), 2.23 (s, 2H), 1.84 -1.70 (m, 2H), 1.33 (s, 9H). |
| 83 | 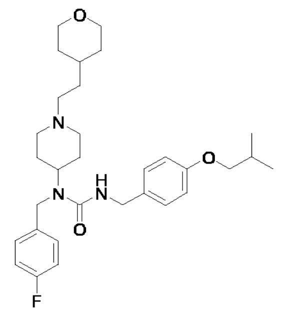 | MS m/z (ESI): 526.4[M+1]$^1$H NMR (600 MHz, $CDCl_3$) $\delta$ 7.17 (m, 2H), 7.02 -6.91 (m, 4H), 6.77 (d, 2H), 4.51 (s, 2H), 4.37 (s, 2H), 4.26 (d, 2H), 3.98 (d, 2H), 3.96 -3.90 (m, 1H), 3.67 (d, 2H), 3.35 (d, 3H), 2.73 (t, 1H), 2.57 -2.42 (m, 1H), 2.11 -1.95 (m, 1H), 1.86 -1.72 (m, 3H), 1.62 -1.53 (m, 5H), 1.48 (tq, 1H), 1.38 -1.25 (m, 3H), 1.00 (d, 6H). |

(continued)

| Compound number | Structural formula | Characterization data |
|---|---|---|
| 84 | 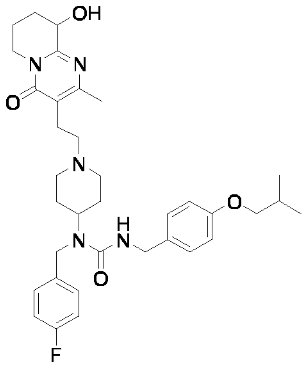 | MS m/z (ESI): 642.5[M+1]$^1$H NMR (600 MHz, $CDCl_3$) $\delta$ 7.18 (dd, 2H), 7.02 -6.95 (m, 4H), 6.77 (d,2H), 4.48 (dd, 2H), 4.38 (s, 2H), 4.27 (d, 2H), 4.07 (s, 1H), 3.98 -3.83 (m, 2H), 3.68 (d, 2H), 3.39 -2.49 (m, 6H), 2.35 (s, 5H), 2.12 (m, 1H), 2.06 (m, 1H), 2.00 -1.91 (m, 1H), 1.88 -1.79 (m, 2H), 1.75 (m, 2H), 1.65 -1.47 (m, 1H), 1.26 (d, 1H), 1.01 (d, 6H). |
| 85 | 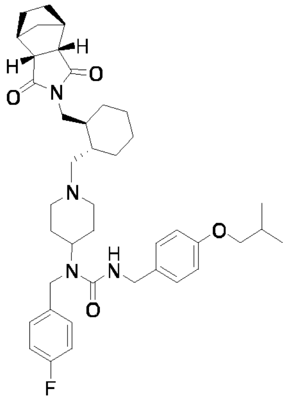 | MS m/z (ESI): 687.1[M+1]$^1$H NMR (600 MHz, $CDCl_3$) $\delta$ 7.23 -7.10 (m, 2H), 6.99 (td, 4H), 6.84 -6.50 (m, 2H), 4.39 (d, 3H), 4.27 (d, 2H), 3.86 -3.73 (m, 1H), 3.68 (d, 2H), 3.26 (s, 1H), 2.90 (d, 2H), 2.72 -2.65 (m, 2H), 2.58 (s, 2H), 2.46 (s,1H) ,2.06 (m, 3H), 1.88 - 1.51 (m, 9H), 1.48 (d , 2H), 1.39 -1.30 (m, 2H), 1.29 -1.07 (m, 6H), 1.01 (d, 6H), 0.98 -0.83 (m, 2H). |
| 86 | 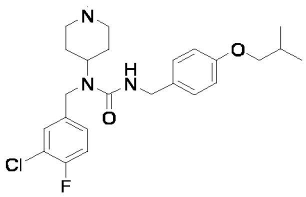 | MS m/z (ESI): 463.1[M+1]$^1$H NMR (600 MHz, $CDCl_3$) $\delta$ 7.13 -7.06 (m, 2H), 7.05 -6.98 (m, 2H), 6.80 (d, 2H), 4.54 -4.42 (m, 2H), 4.36 (s, 2H), 4.29 (d, 2H), 3.68 (d, 2H), 3.16 (d, 2H), 2.49 (s, 3H), 2.43 (s, 2H), 2.06 (s, 3H), 1.92 -1.71 (m, 2H), 1.01 (d, 6H). |
| 87 | 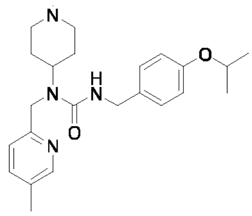 | MS m/z (ESI): 411.3[M+1]$^1$H NMR (600 MHz, $CDCl_3$) $\delta$ 8.22 (s, 1H), 7.45 (d, 1H), 7.18 (t, 3H), 6.81 (d, 2H), 4.51 (s, 1H), 4.34 (t, 5H), 2.99 -2.90 (m, 2H), 2.31 (d, 6H), 2.19 -2.10 (m, 2H), 1.91 - 1.79 (m, 2H), 1.69 (d, 2H), 1.32 (d, 6H). |
| 88 | 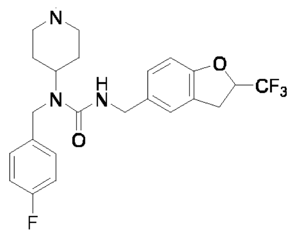 | MS m/z (ESI): 466.4[M+1]$^1$H NMR (600 MHz, DMSO-$d_6$) $\delta$ 7.25 (m, 2H), 7.15-7.10 (m, 2H), 7.06 (s, 1H), 7.03-6.97 (m, 1H), 6.90 (t, 1H), 6.82 (d, 1H), 5.46 (m, 1H), 4.41 (s, 2H), 4.19 (d, 2H), 4.00 (d, 1H), 3.53 (m, 1H), 3.23 (m, 1H), 2.93-2.85 (m, 2H), 2.26 (s, 5H), 1.70-1.60 (m, 2H), 1.50 (d, 2H). |

(continued)

| Compound number | Structural formula | Characterization data |
|---|---|---|
| 89 | 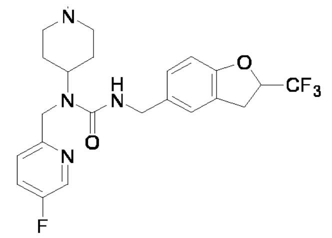 | MS m/z (ESI): 465.2 [M+1]; $^{1}H$ NMR (400 MHz, $CDCl_3$) δ 8.26 (d, 1H), 7.39-7.36 (m, 2H), 7.21-7.08 (m, 3H), 6.84-6.81(m, 2H), 6.07-5.75 (m, 1H), 4.47-4.32 (m, 5H), 4.03 (t, 2H), 3.03 (d, 2H), 2.41-2.15 (m, 5H), 2.11-1.96 (m, 4H), 1.75-1.56(m, 4H). |
| 90 | 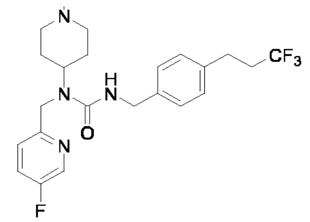 | MS m/z (ESI): 453.5[M+1]$^{1}H$ NMR (600 MHz, DMSO-$d_6$) δ 8.48 (d, 1H), 7.68 (m, 1H), 7.33 (m, 1H), 7.20 (d, 2H), 7.14 (m, 3H), 4.48 (s, 2H), 4.23 (d, 2H), 4.11-4.02 (m, 1H), 2.98 (d, 2H), 2.81-2.73 (m, 2H), 2.55 (m, 2H), 2.35 (s, 5H), 1.71 (m, 2H), 1.55 (m, 2H). |
| 91 | 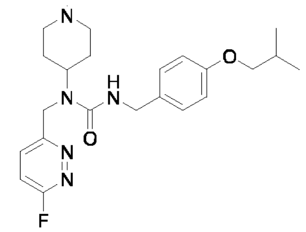 | MS m/z (ESI): 430.3 [M+1]; $^{1}H$ NMR (400 MHz, $CDCl_3$) δ7.69-7.65 (m, 1H), 7.17-7.13 (m, 3H), 6.83-6.81 (m, 2H), 5.67-5.62(m, 1H), 4.65 (s, 2H), 4.31 (d, 2H), 4.10-4.01 (m, 1H), 3.69(d, 2H), 2.88 (d, 2H), 2.26 (s, 3H), 2.14-1.97 (m, 3H), 1.76-1.66 (m, 4H), 1.01 (d, 6H). |
| 92 | 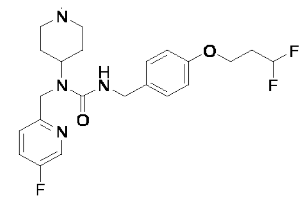 | MS m/z (ESI): 451.2 [M+1]; $^{1}H$ NMR (400 MHz, $CDCl_3$) δ 8.27 (d, 1H), 7.39-7.36 (m, 2H), 7.21 (d, 2H), 6.86-6.82 (m, 3H), 6.24-5.96 (m, 1H), 4.42-4.33 (m, 5H), 4.11 (t, 2H), 3.02 (d, 2H), 2.38-2.21 (m, 7H), 1.95-1.72(m, 4H). |
| 93 | 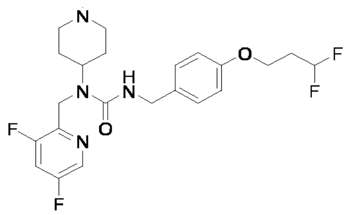 | MS m/z (ESI): 469.2 [M+1]; $^{1}H$ NMR (400 MHz, $CDCl_3$) δ 8.16 (d, 1H), 7.25-7.22 (m, 3H), 7.11-6.98 (m, 3H), 6.23-5.95 (m, 1H), 4.42-4.23 (m, 4H), 4.19-4.10 (m, 3H), 2.92 (d, 2H), 2.38-2.11 (m, 7H), 1.85-1.62(m, 4H). |
| 94 | 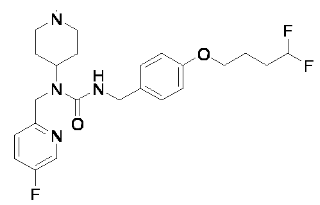 | MS m/z (ESI): 465.2 [M+1]; $^{1}H$ NMR (400 MHz, $CDCl_3$) δ 8.26 (d, 1H), 7.39-7.36 (m, 2H), 7.21-7.08 (m, 3H), 6.84-6.81(m, 2H), 6.07-5.75 (m, 1H), 4.47-4.32 (m, 5H), 4.03 (t, 2H), 3.03 (d, 2H), 2.41-2.15 (m, 5H), 2.11-1.96 (m, 4H), 1.75-1.56(m, 4H). |
| 95 | 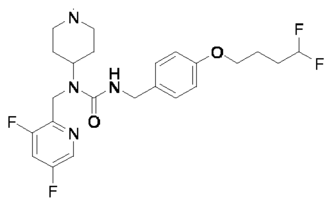 | MS m/z (ESI): 483.2 [M+1]; $^{1}H$ NMR (400 MHz, $CDCl_3$) δ 8.16 (d, 1H), 7.25-7.22 (m, 3H), 7.11-6.98 (m, 3H), 6.23-5.95 (m, 1H), 4.47-4.36 (m, 4H), 4.23-4.03 (m, 3H), 2.93 (d, 2H), 2.31 (s, 3H), 2.11-1.96(m, 6H), 1.85-1.66 (m, 4H). |

(continued)

| Compound number | Structural formula | Characterization data |
|---|---|---|
| 96 | 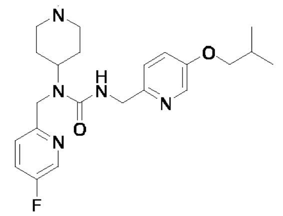 | MS m/z (ESI): 430.2 [M+1]; $^1$H NMR (400 MHz, DMSO-$d_6$) δ 8.48 (d, 1H), 8.16 (d, 1H), 7.69-7.66 (m, 1H), 7.37-7.34 (m, 2H), 7.19-7.15 (m, 2H), 4.48(s, 2H), 4.28 (d, 2H), 3.95-3.79(m, 3H), 2.72 (d, 2H), 2.13 (s, 3H), 2.04-1.87 (m, 3H), 1.61-1.46 (m, 4H), 0.95 (d, 6H). |
| 97 | 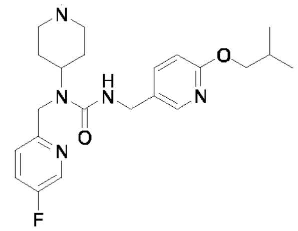 | MS m/z (ESI): 430.2 [M+1]; $^1$H NMR (600 MHz, $CDCl_3$) δ 8.30-8.23 (m, 1H), 8.06 (d, 1H), 7.69 (s, 1H), 7.53 (dd, 1H), 7.11 (d, 2H), 6.69 (d, 1H), 4.41-4.32 (m, 5H), 4.03 (d, 2H), 3.08 (d, 2H), 2.43 (s, 3H), 2.34-2.27 (m, 2H), 2.11-2.02 (m, 3H), 1.69 (d, 2H), 1.01 (d, 6H). |
| 98 | 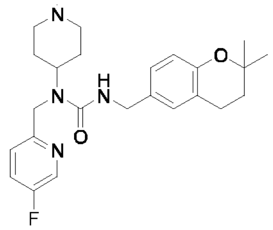 | MS m/z (ESI): 441.3 [M+1]; $^1$H NMR (600 MHz, $CDCl_3$) δ 8.25 (d, 1H), 7.48-7.35 (m, 2H), 7.15-7.08 (m, 1H), 7.02-6.93 (m, 2H), 6.71 (d, 1H), 4.51-4.38 (m, 3H), 4.30 (d, 2H), 3.24-3.14 (m, 2H), 2.73 (t, 2H), 2.51 (s, 5H), 2.29-2.16 (m, 2H), 1.81-1.68 (m, 4H), 1.31 (s, 6H). |
| 99 | 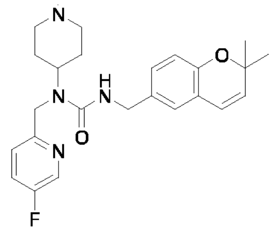 | MS m/z (ESI): 439.3 [M+1]; $^1$H NMR (600 MHz, DMSO-$d_6$) δ 8.47 (d, 1H), 7.75-7.60 (m, 2H), 7.34 (m,1H), 7.04 (d, 1H), 6.95 (m, 1H), 6.91-6.84 (m, 1H), 6.63 (m, 1H), 6.34 (d, 1H), 4.47 (s, 2H), 4.14 (m, 3H), 3.05 (d, 2H), 2.50 (m, 3H), 2.41 (s, 2H), 1.75 (m, 2H), 1.62-1.50 (m, 2H), 1.30 (d, 6H). |
| 100 | 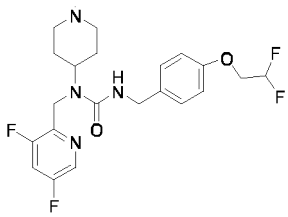 | MS m/z (ESI): 454.3 [M+1]; $^1$H NMR (600 MHz, $CDCl_3$) δ 8.14 (d, 1H), 7.26-7.20 (m, 3H), 7.10-7.04 (m, 1H), 6.90-6.85 (m, 2H), 6.08 (tt, 1H), 4.48 (d, 2H), 4.39-4.30 (m, 3H), 4.17 (td, 2H), 3.12 (d, 2H), 2.46 (s, 3H), 2.36 (s, 2H), 1.74-1.68 (m, 2H). |
| 101 | 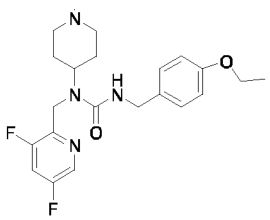 | MS m/z (ESI): 437.2 [M+1]; $^1$H NMR (600 MHz, $CDCl_3$) δ 8.14 (d, 1H), 7.25-7.20 (m, 3H), 7.04 (t, 1H), 6.90-6.86 (m, 2H), 4.81-4.77 (m, 1H), 4.73-4.70 (m, 1H), 4.48 (d, 2H), 4.36 (d, 3H), 4.21 (ddd, 2H), 3.29-3.14 (m, 2H), 2.51 (d, 5H), 2.19 (s, 2H), 1.77-1.71(m, 2H). |

**Example 2:**

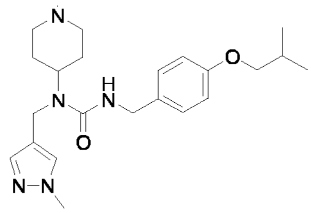

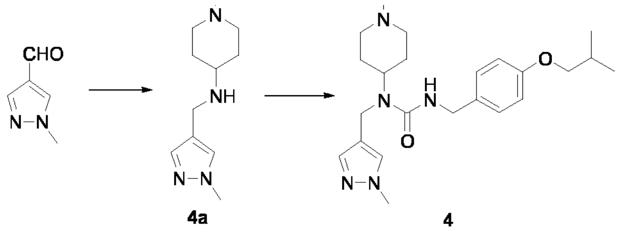

4a 4

Under nitrogen protection, in an ice-water bath, 1-methylpyrazole-4-carbaldehyde (440 mg, 4.0 mmol) was dissolved in 10 ml of methanol. N-methyl-4-piperidone (452 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **4a** (617 mg).

Under nitrogen protection, compound **4a** (208 mg, 1.0 mmol) was dissolved in 5 ml of acetonitrile. N-(4-isobutyloxybenzyl)-1*H*-imidazol-formamide (273 mg, 1.0 mmol) and potassium carbonate (207 mg, 1.5 mmol) were added, and the resulting mixture was heated to 60°C and reacted under stirring for 12 h. The reaction solution was cooled to room temperature and filtered. 10 ml of water was added to the filtrate, and then the resulting solution was extracted with dichloromethane (5 mL * 3). The organic phases were combined, dried over anhydrous sodium sulfate, filtered, concentrated under reduced pressure and separated by column chromatography (dichloromethane:methanol = 10 : 1) to obtain **compound 4** (172 mg, yield: 43%). MS m/z (ESI): 414.3 [M+1]; $^1$H NMR(400 MHz, $CDCl_3$) $\delta$ 7.38(s, 1H), 7.17(s, 1H), 7.12-7.07(m, 2H), 6.82-6.77(m, 2H), 4.69(m, 1H), 4.30-4.25(m, 2H), 4.23-4.20(m, 2H), 3.78(s, 3H), 3.69-3.64(d, 2H), 2.98-2.88(m, 2H), 2.32(s, 3H), 2.18-2.02(m, 3H), 1.80-1.65(m, 4H), 1.04-0.99(d, 6H).

Compounds 5, 8 and 22 were prepared in a similar manner to compound 4.

Table 2: Structures and characterization data of compounds 5, 8 and 22

| Compound number | Structural formula | Characterization data |
|---|---|---|
| 5 | 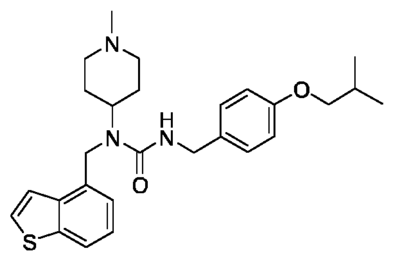 | MS m/z (ESI): 466.2 [M+1]; $^1$H NMR(400 MHz, $CDCl_3$) $\delta$ 7.79(d, 1H), 7.52(d, 1H), 7.40-7.37(m, 1H), 7.30-7.27(m, 1H), 7.25-7.23(m,1H), 7.02-6.98(m, 2H), 6.78-6.75(m, 2H), 4.75(s, 3H), 4.68-4.59(m, 2H), 4.26(d, 2H), 3.70(d, 2H), 3.20-3.05(m, 3H), 2.50-2.34(m, 4H), 2.10-1.98(m, 2H), 1.89-1.82(m, 2H), 1.41-1.35(m, 2H), 1.02-0.98(d, 6H). |
| 8 | 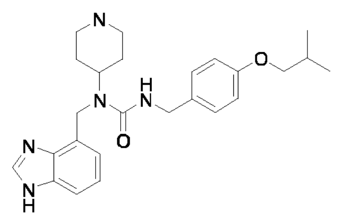 | MS m/z (ESI): 450.3 [M+1]; $^1$H NMR(400 MHz, $CDCl_3$) $\delta$ 7.82(d, 1H), 7.51(d, 1H), 7.20-7.09(m, 5H), 6.78-6.73(m, 2H), 4.75(s, 2H), 4.28(d, 2H), 3.68(d, 2H), 3.20-3.05(m, 2H), 2.50-2.34(m, 5H), 2.15-1.95 (m, 4H), 1.69-1.52(m, 2H), 1.00-0.95(d, 6H). |

(continued)

| Compound number | Structural formula | Characterization data |
|---|---|---|
| 22 | 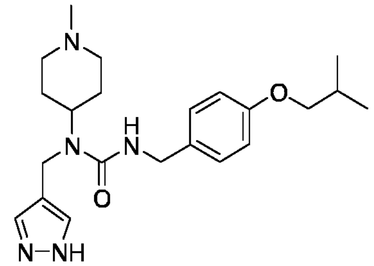 | MS m/z (ESI): 400.3 [M+1]; $^{1}H$ NMR(400 MHz, $CDCl_3$) $\delta$ 7.42(s, 2H), 7.12-7.06(m, 2H), 6.84-6.79(m, 2H), 4.73(m, 1H), 4.41(m, 1H), 4.31-4.25(m, 4H), 3.70-3.66(d, 2H), 3.18-3.10(m, 2H), 2.47(s, 3H), 2.44-2.34(m, 2H), 2.10-1.98 (m, 3H), 1.80-1.72(m, 2H), 1.03-0.98(d, 6H). |

**Example 3:**

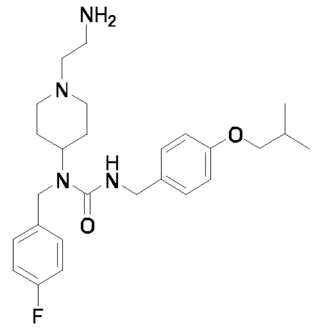

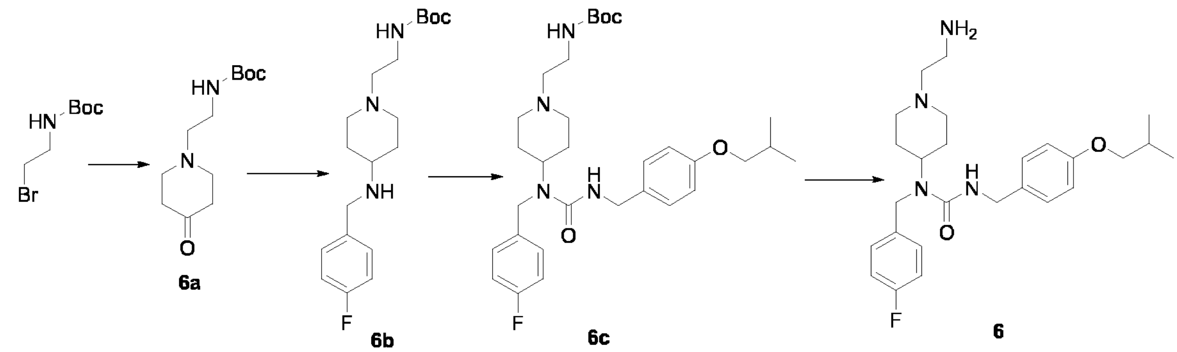

6a 6b 6c 6

N-Boc-bromoethylamine (2.23 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 mL of water was added, and then the resulting solution was extracted with dichloromethane (60 mL * 3). The organic phases were combined, dried over anhydrous sodium sulfate, filtered and concentrated under reduced pressure to obtain compound **6a** (1.97 g).

Under nitrogen protection, in an ice-water bath, 4-fluorobenzylamine (500 mg, 4.0 mmol) was dissolved in 10 ml of methanol. Compound **6a** (968 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **6b** (912 mg).

Under nitrogen protection, compound **6b** (702 mg, 2.0 mmol) was dissolved in 10 ml of acetonitrile. N-(4-isobutyloxybenzyl)-1*H*-imidazol-formamide (546 mg, 2.0 mmol) and potassium carbonate (414 mg, 3.0 mmol) were added, and the resulting mixture was heated to 60°C and reacted under stirring for 12 h. The reaction solution was

cooled to room temperature and filtered. 20 mL of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane:methanol = 10 : 1) to obtain compound **6c** (467 mg).

Under nitrogen protection, compound **6c** (278 mg, 0.5 mmol) was dissolved in 5 mL of dichloromethane. Trifluoroacetic acid (285 mg, 2.5 mmol) was added, and the resulting mixture was reacted at room temperature for 2 h. An aqueous solution of $NaHCO_3$ was added to adjust the pH value to alkaline. The organic phase was concentrated and then extracted with dichloromethane (5 ml * 3). The organic phases were combined, dried over anhydrous sodium sulfate, filtered and concentrated under reduced pressure to obtain **compound 6** (201 mg, yield: 88%). MS m/z (ESI): 457.2 [M+1]; $^1$H NMR(400 MHz, $CDCl_3$) $\delta$ 7.25-7.23(m,2H), 7.02-6.98(m, 4H), 6.78-6.75(m, 2H), 4.48-4.27(m, 5H), 3.62-3.59(m, 2H), 3.51-3.29(m, 2H), 2.94-2.89(m, 2H), 2.84-2.80(m, 2H), 2.47-2.41(m, 2H), 2.18-2.02(m, 3H), 1.75-1.68(m, 4H), 1.02-0.98(d, 6H).

**Example 4:**

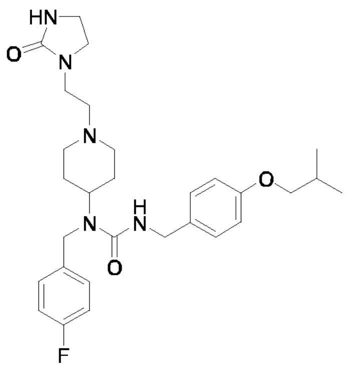

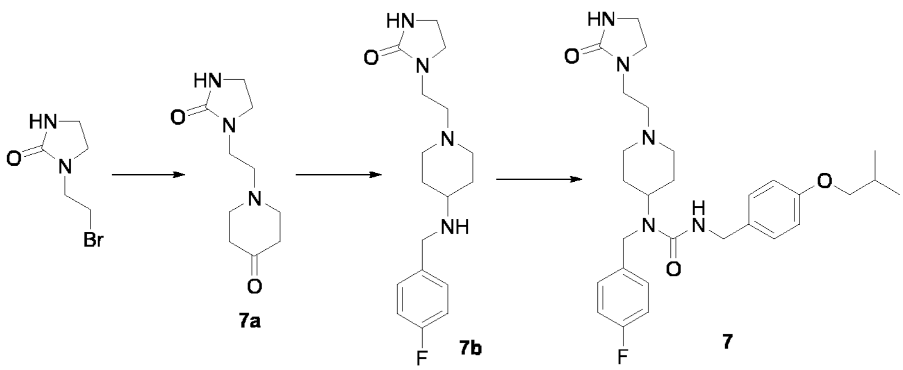

7a 7b 7

1-(2-Bromoethyl)imidazolidine-2-one (1.93 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 ml of water was added, and then the resulting solution was extracted with dichloromethane (60 mL * 3). The organic phases were combined, dried over anhydrous sodium sulfate, filtered and concentrated under reduced pressure to obtain compound **7a** (1.56 g).

Under nitrogen protection, in an ice-water bath, 4-fluorobenzylamine (500 mg, 4.0 mmol) was dissolved in 10 ml of methanol. Compound 7a (844 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **7b** (812 mg).

Under nitrogen protection, compound **7b** (640 mg, 2.0 mmol) was dissolved in 10 ml of acetonitrile. N-(4-isobutyloxybenzyl)-1*H*-imidazol-formamide (546 mg, 2.0 mmol) and potassium carbonate (414 mg, 3.0 mmol) were added, and the resulting mixture was heated to 60°C and reacted under stirring for 12 h. The reaction solution was cooled to room temperature and filtered. 20 mL of water was added to the filtrate, and then the resulting solution was

extracted with dichloromethane (10 ml * 3). The organic phases were combined, dried over anhydrous sodium sulfate, filtered, concentrated under reduced pressure and separated by column chromatography (dichloromethane:methanol = 10 : 1) to obtain **compound 7** (494 mg, yield: 47%). MS m/z (ESI): 526.3 [M+1]; $^1H$ NMR(400 MHz, $CDCl_3$) δ 7.24-7.22(m,2H), 7.02-6.98(m, 4H), 6.78-6.75(m, 2H), 4.60-4.54(m, 2H), 4.32-4.26(m, 4H), 3.72-3.62(m, 4H), 3.51-3.44(m, 2H), 3.41-3.32(m, 2H), 3.30-3.26(m, 2H), 2.98-2.92(m, 2H), 2.47-2.41(m,2H), 2.18-2.02(m, 3H), 1.74-1.65(m, 4H), 1.02-0.99(d, 6H).

**Example 5:**

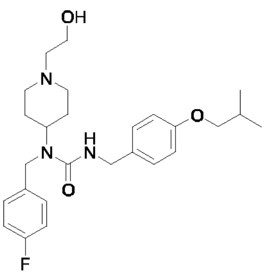

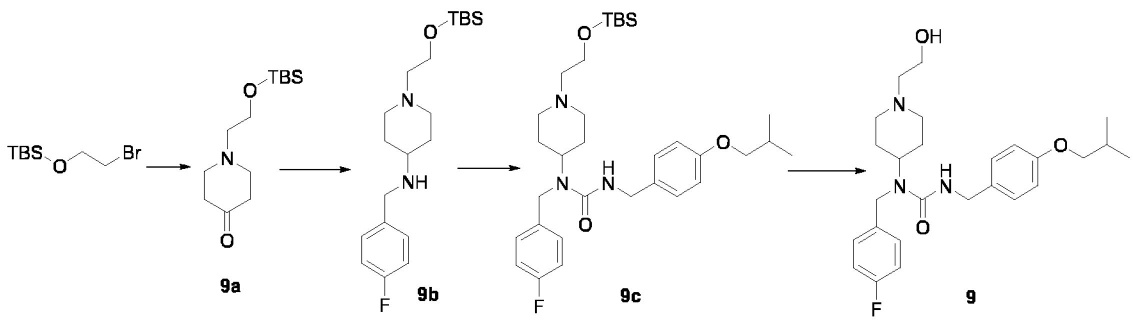

9a 9b 9c 9

Tert-butyl dimethyl bromoethoxysilane (2.38 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 ml of water was added, and then the resulting solution was extracted with dichloromethane (60 mL * 3). The organic phases were combined, dried over anhydrous sodium sulfate, filtered and concentrated under reduced pressure to obtain compound **9a** (1.72 g).

Under nitrogen protection, in an ice-water bath, 4-fluorobenzylamine (500 mg, 4.0 mmol) was dissolved in 10 ml of methanol. Compound **9a** (1028 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **9b** (812 mg).

Under nitrogen protection, compound **9b** (732 mg, 2.0 mmol) was dissolved in 10 ml of acetonitrile. N-(4-isobutyloxybenzyl)-1*H*-imidazol-formamide (546 mg, 2.0 mmol) and potassium carbonate (414 mg, 3.0 mmol) were added, and the resulting mixture was heated to 60°C and reacted under stirring for 12 h. The reaction solution was cooled to room temperature and filtered. 20 mL of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane:methanol = 10 : 1) to obtain **compound 9c** (327 mg).

Under nitrogen protection, compound **9c** (286 mg, 0.5 mmol) was dissolved in 5 ml of THF. A 1 M tetrabutylammonium fluoride tetrahydrofuran solution (1 ml, 1.0 mmol) was added, and the resulting mixture was reacted at room temperature for 2 h. The organic phase was concentrated and then extracted with dichloromethane (5 mL * 3). The organic phases were combined, dried over anhydrous sodium sulfate, filtered and concentrated under reduced pressure to obtain **compound 9** (118 mg, yield: 52%). MS m/z (ESI): 458.3 [M+1]; $^1H$ NMR(400 MHz, $CDCl_3$) δ 7.23-7.20(m,2H), 7.02-6.98(m, 4H), 6.81-6.76(m, 2H), 4.70-4.50(m, 2H), 4.38-4.27(m, 4H), 3.78-3.69(m, 4H), 3.41-3.19(m, 2H), 2.84-2.74(m, 2H), 2.67-2.51(m, 2H), 2.18-2.02(m, 2H), 1.81-1.58(m, 3H), 1.02-0.98(d, 6H).

**Example 6:**

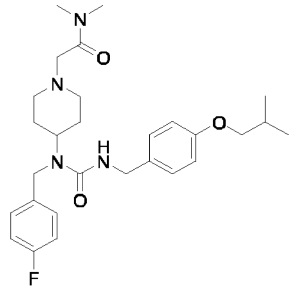

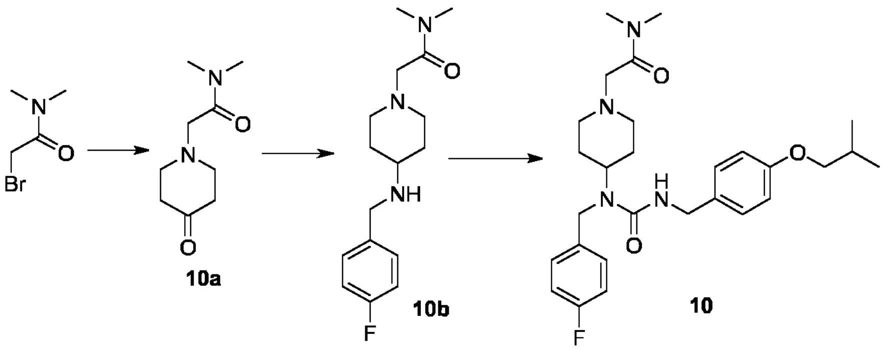

2-Bromo-N,N-dimethylacetamide (1.66 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 ml of water was added, and then the resulting solution was extracted with dichloromethane (60 mL * 3). The organic phases were combined, dried over anhydrous sodium sulfate, filtered and concentrated under reduced pressure to obtain compound **10a** (1.15 g).

Under nitrogen protection, in an ice-water bath, 4-fluorobenzylamine (500 mg, 4.0 mmol) was dissolved in 10 ml of methanol. Compound **10a** (736 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound 10b (832 mg).

Under nitrogen protection, compound **10b** (586 mg, 2.0 mmol) was dissolved in 10 ml of acetonitrile. N-(4-isobutyloxybenzyl)-1*H*-imidazol-formamide (546 mg, 2.0 mmol) and potassium carbonate (414 mg, 3.0 mmol) were added, and the resulting mixture was heated to 60°C and reacted under stirring for 12 h. The reaction solution was cooled to room temperature and filtered. 20 mL of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane:methanol = 10 : 1) to obtain **compound 10** (535 mg, yield: 54%). MS m/z (ESI): 499.3 [M+1]; $^1$H NMR(400 MHz, $CDCl_3$) $\delta$ 7.20-7.13(m, 2H), 7.02-6.95(m, 4H), 6.77-6.73(m, 2H), 4.48-4.43(m, 1H), 4.38-4.27(m, 3H), 4.26-4.23(m, 2H), 3.66(d, 2H), 3.15(d, 2H), 3.02-2.88(m, 8H), 2.30-2.20(m, 2H), 2.10-2.00(m, 1H), 1.80-1.65(m, 4H), 1.01-0.96(d, 6H).

**Example 7:**

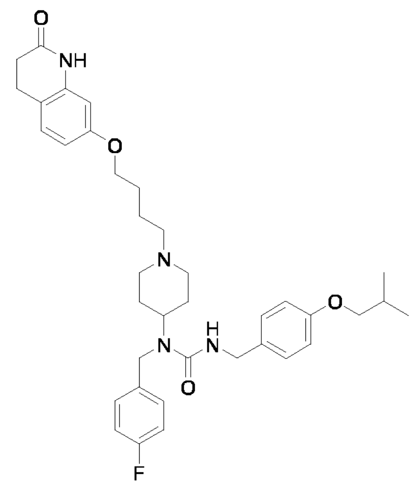

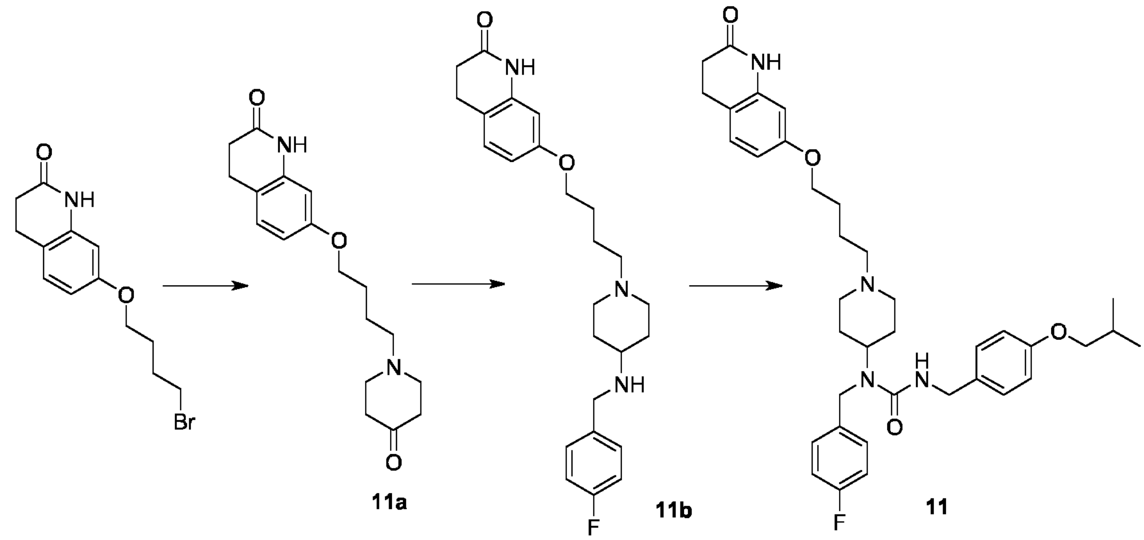

11a 11b 11

7-(4-Bromobutoxy)-3,4-dihydro-2(1H)-quinolinone (2.98 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 ml of water was added, and then the resulting solution was extracted with dichloromethane (60 mL * 3). The organic phases were combined, dried over anhydrous sodium sulfate, filtered and concentrated under reduced pressure to obtain compound **11a** (2.26 g).

Under nitrogen protection, in an ice-water bath, 4-fluorobenzylamine (500 mg, 4.0 mmol) was dissolved in 10 ml of methanol. Compound **11a** (1.26 g, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **11b** (1.03 g).

Under nitrogen protection, compound **11b** (425 mg, 1.0 mmol) was dissolved in 10 ml of acetonitrile. N-(4-isobutyloxybenzyl)-1*H*-imidazol-formamide (273 mg, 1.0 mmol) and potassium carbonate (207 mg, 1.5 mmol) were added, and the resulting mixture was heated to 60°C and reacted under stirring for 12 h. The reaction solution was cooled to room temperature and filtered. 20 mL of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane methanol = 10 : 1) to obtain **compound 11** (311 mg, yield: 49%). MS m/z (ESI): 631.3 [M+1]; $^1$H NMR(400 MHz, $CDCl_3$) δ 7.96(s, 1H), 7.20-7.15(m, 2H), 7.04-6.96(m, 4H), 6.79-6.75(m, 2H), 6.50-6.43(m, 1H), 6.32-6.28(m, 1H), 4.70-4.52(m, 2H), 4.48-4.43(m, 2H), 4.31-4.27(m, 2H), 3.98-3.90(m, 2H), 3.64(d, 2H), 3.50-3.20(d, 2H), 2.82-2.58(m, 4H), 2.56-2.44(m, 4H), 2.10-2.00(m, 2H), 1.90-1.65(m, 7H), 1.02-0.97(d, 6H).

**Example 8:**

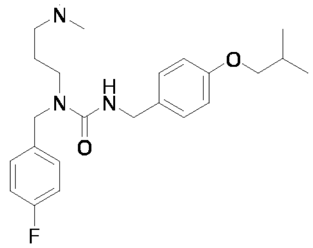

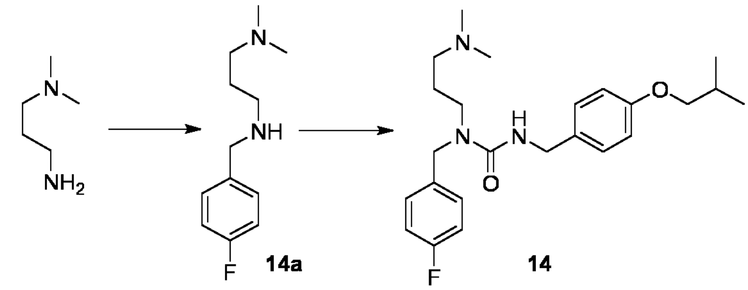

Under nitrogen protection, in an ice-water bath, 4-fluorobenzylamine (500 mg, 4.0 mmol) was dissolved in 10 ml of methanol. N,N-dimethyl-1,3-diaminopropane (408 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **14a** (587 mg).

Under nitrogen protection, compound **14a** (210 mg, 1.0 mmol) was dissolved in 5 ml of acetonitrile. N-(4-isobutyloxybenzyl)-1*H*-imidazol-formamide (273 mg, 1.0 mmol) and potassium carbonate (207 mg, 1.5 mmol) were added, and the resulting mixture was heated to 60°C and reacted under stirring for 12 h. The reaction solution was cooled to room temperature and filtered. 10 ml of water was added to the filtrate, and then the resulting solution was extracted with dichloromethane (5 ml * 3). The organic phases were combined, dried over anhydrous sodium sulfate, filtered, concentrated under reduced pressure and separated by column chromatography (dichloromethane:methanol = 10 : 1) to obtain **compound 13** (202 mg, yield: 48%). MS m/z (ESI): 416.3 [M+1]; $^1$H NMR(400 MHz, $CDCl_3$) δ 7.26-7.20(m, 4H), 7.02-6.97(m, 2H), 6.81-6.76(m, 2H), 4.47(s, 2H), 4.38-4.35(m, 2H), 3.71(s, 2H), 3.30-3.24(m, 2H), 2.35-2.25(m, 2H), 2.15-2.00(m, 7H), 1.65-1.58(m, 2H), 1.02-0.98(d, 6H).

**Example 9:**

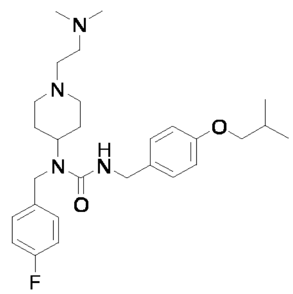

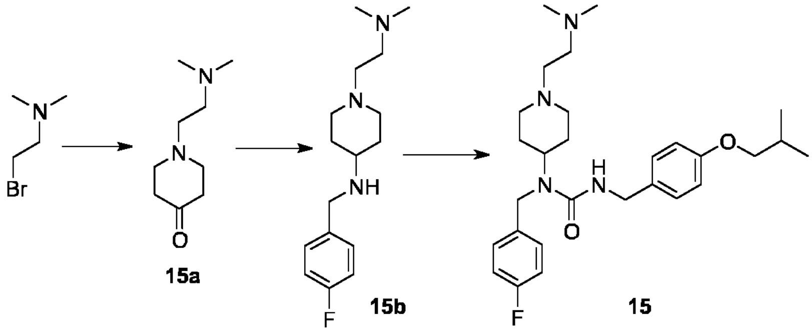

15a 15b 15

(2-Bromomethyl)dimethylamine (1.52 g, 10.0 mmol) was dissolved in 20 ml of DMF. 4-Piperidone (0.99 g, 10.0 mmol) and potassium carbonate (2.07 g, 15.0 mmol) were added, and the resulting mixture was heated to 80°C and reacted under stirring for 8 h. The reaction solution was cooled to room temperature. 60 ml of water was added, and then the resulting solution was extracted with dichloromethane (60 mL * 3). The organic phases were combined, dried over anhydrous sodium sulfate, filtered and concentrated under reduced pressure to obtain compound **15a** (1.17 g).

Under nitrogen protection, in an ice-water bath, 4-fluorobenzylamine (500 mg, 4.0 mmol) was dissolved in 10 ml of methanol. Compound 15a (680 mg, 4.0 mmol) and sodium triacetoxyborohydride (933 mg, 4.4 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (10 ml * 3). The organic phases were combined, dried over anhydrous sodium sulfate, filtered and concentrated under reduced pressure to obtain compound **15b** (611 mg).

Under nitrogen protection, compound **15b** (279 mg, 1.0 mmol) was dissolved in 10 ml of acetonitrile. N-(4-isobutyloxybenzyl)-1*H*-imidazol-formamide (273 mg, 1.0 mmol) and potassium carbonate (207 mg, 1.5 mmol) were added, and the resulting mixture was heated to 60°C and reacted under stirring for 12 h. The reaction solution was cooled to room temperature and filtered. 20 mL of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane:methanol = 10:1) to obtain **compound 15** (287 mg, yield: 59%). MS m/z (ESI): 485.3 [M+1]; $^1H$ NMR(400 MHz, $CDCl_3$) $\delta$ 7.22-7.16(m, 2H), 7.02-6.95(m, 4H), 6.77-6.75(m, 2H), 4.55-4.46(m, 1H), 4.38-4.32(m, 2H), 4.26-4.23(m, 2H), 3.67(d, 2H), 3.19(d, 1H), 2.98-2.68(m, 6H), 2.61(s, 6H), 2.45-2.35(m, 2H), 2.10-2.00(m, 2H), 1.80-1.65(m, 2H), 1.01-0.96(d, 6H).

**Example 10:**

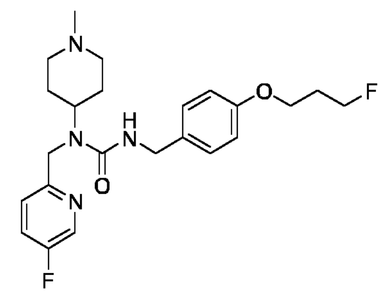

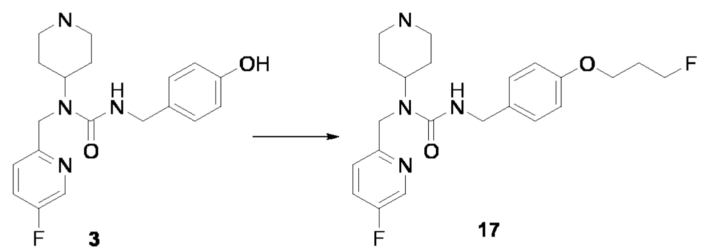

3 17

Under nitrogen protection, **compound 3** (372 mg, 1.0 mmol) was dissolved in 10 ml of acetonitrile. 1-Bromo-3-fluoropropane (211 mg, 1.5 mmol) and cesium carbonate (652 mg, 2.0 mmol) were added, and the resulting mixture was heated to 60°C and reacted under stirring for 5 h. The reaction solution was cooled to room temperature and filtered. 10 ml of water was added to the filtrate, and then the resulting solution was extracted with dichloromethane (10 ml * 3). The organic phases were combined, dried over anhydrous sodium sulfate, filtered, concentrated under reduced pressure and separated by column chromatography (dichloromethane:methanol = 10 : 1) to obtain **compound 17** (238 mg, yield: 54%). MS m/z (ESI): 433.2 [M+1]; $^1$H NMR(400 MHz, $CDCl_3$) δ 8.22(s, 1H), 7.45-7.35(m, 2H), 7.22-7.14(m, 2H), 7.15-7.07(m, 1H), 6.85-6.81(m, 2H), 4.67(t, 1H), 4.60(t, 1H), 4.44-4.34(m, 3H), 4.34(d, 2H), 4.07(t, 2H), 3.12(d, 2H), 2.46(s, 3H), 2.40-2.32(m, 2H), 2.19-2.05(m, 4H), 1.75-1.67(m, 2H).

Compounds 18, 55-57, 59-60, 62, 65, 69, 73-74, 78 and 82 were prepared in a similar manner to compound 17.

Table 3: Structures and characterization data of compounds 18, 55-57, 59-60, 62, 65, 69, 73-74, 78 and 82

| Compound number | Structural formula | Characterization data |
|---|---|---|
| 18 | 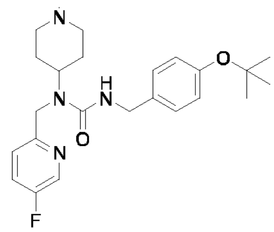 | MS m/z (ESI): 429.3 [M+1]; $^1$H NMR(400 MHz,$CDCl_3$) δ 8.23 (s,1H), 7.39-7.34(m, 2H), 7.17-7.14(m, 2H), 6.98-6.90(m, 3H), 4.37(s, 2H), 4.41(s, 2H), 4.38-4.34(m, 2H), 3.06-3.00(m, 2H), 2.39(s, 3H), 2.30-2.24(m, 2H), 2.02-1.93(m, 2H), 1.73-1.68(m, 2H), 1.32(s, 9H). |
| 55 | 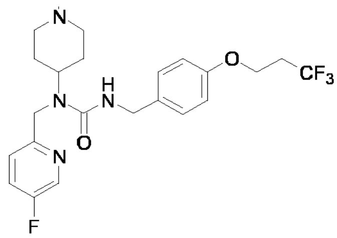 | MS m/z (ESI): 469.2 [M+1]; 1H NMR(400 MHz, $CDCl_3$) δ 8.27 (d, 1H), 7.44-7.35(m, 2H), 7.25-7.19(m, 2H), 7.17-7.08(m, 1H), 6.85-6.80(m, 2H), 4.42-4.34(m, 5H), 4.18(t, 2H), 3.10(d, 2H), 2.65-2.58(m, 2H), 2.45(s, 3H), 2.37-2.30(m, 2H), 2.11-2.02(m, 2H), 1.73-1.67(m, 2H). |
| 56 | 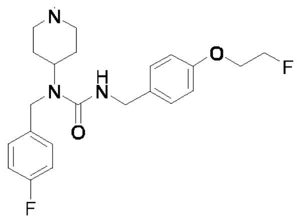 | MS m/z (ESI): 418.2 [M+1]; 1H NMR(400 MHz, $CDCl_3$) δ 7.20-7.15(m, 2H), 7.04-6.94(m, 4H), 6.85-6.77(m, 2H), 4.77(t, 1H), 4.68(t, 1H), 4.54-4.38(m, 2H), 4.34(s, 2H), 4.27(d, 2H), 4.22-4.14(m, 2H), 3.02(d, 2H), 2.36(s, 3H), 2.30-2.18(m, 2H), 1.95-1.73(m, 4H). |
| 57 | 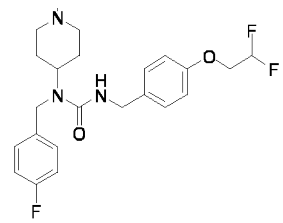 | MS m/z (ESI): 436.2 [M+1]; 1H NMR(400 MHz, $CDCl_3$) δ 7.21-7.15(m, 2H), 7.05-6.96(m, 4H), 6.83-6.77(m, 2H), 6.15-5.97(m, 1H), 4.55(t, 1H), 4.50-4.35(m, 3H), 4.28(d, 2H), 4.15(dt, 2H), 3.03(d, 2H), 2.38(s, 3H), 2.31-2.22(m, 2H), 1.92-1.73(m, 4H). |
| 59 | 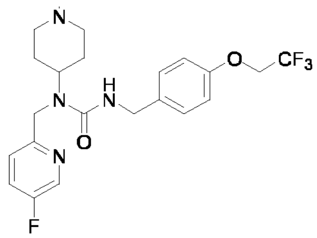 | MS m/z (ESI): 455.5[M+1],477.5[M+23]1H NMR (600 MHz, DMSO-d6) δ 8.49 (d, 1H), 7.74 -7.65 (m, 1H), 7.33 (m, 1H), 7.23 -7.17 (m, 2H), 7.13 (s, 1H), 7.00 -6.95 (m, 2H), 4.72 (q, 2H), 4.47 (s, 2H), 4.21 (d, 2H), 4.16 -4.05 (m, 1H), 3.07 (s, 2H), 2.50 (d, 3H), 2.44 (s, 2H), 1.77 (d, 2H), 1.58 (d, 2H). |

(continued)

| Compound number | Structural formula | Characterization data |
|---|---|---|
| 60 | 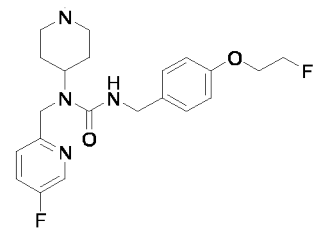 | MS m/z (ESI): 419.48[M+1]1H NMR (600 MHz, $CDCl_3$) δ 8.24 (d, 1H), 7.52 (m, 1H), 7.40 (m, 2H), 7.23 (d, 2H), 6.88 (d, 2H), 4.81 -4.77 (m, 1H), 4.72 -4.69 (m, 1H), 4.52 -4.45 (m, 1H), 4.43 (s, 2H), 4.36 (d, 2H), 4.25 -4.21 (m, 1H), 4.20 -4.16 (m, 1H), 3.29 -3.23 (m, 2H), 2.58 (s, 3H), 2.56 (s, 2H), 2.33 (d, 2H), 1.74 (m, 2H). |
| 62 | 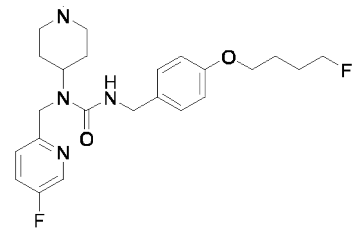 | MS m/z (ESI): 447.26[M+1]1H NMR (600 MHz, DMSO-d6) δ 8.48 (d, 1H), 7.68 (d, 1H), 7.33 (m, 1H), 7.16 -7.07 (m, 3H), 6.85 (d, 2H), 4.54 (t, 1H), 4.46 (q, 3H), 4.18 (d, 2H), 3.97 (t, 1H), 3.09 (s, 2H), 2.50 (m, 3H), 2.47 -2.41 (m, 2H), 1.86 -1.73 (m, 6H), 1.62 -1.54 (m, 2H), 1.29 -1.19 (m, 2H). |
| 65 | 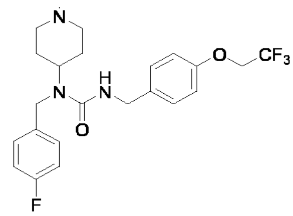 | MS m/z (ESI): 454.2[M+1],476.3[M+23]$^1$H NMR (600 MHz, DMSO-$d_6$) $\delta$ 7.24 (m, 2H), 7.19 -7.15 (m, 2H), 7.14 -7.10 (m, 2H), 6.98 (m, 3H), 4.72 (q, 2H), 4.41 (s, 2H), 4.20 (d, 2H), 4.04 (s, 1H), 2.93 (s, 2H), 2.50 (t, 3H), 2.30 (s, 2H), 1.82 -1.65 (m, 2H), 1.50 (d, 2H). |
| 69 | 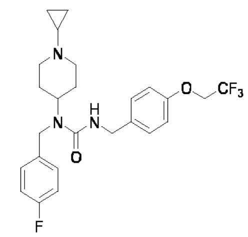 | MS m/z (ESI): 480.4[M+1]$^1$H NMR (600 MHz, DMSO-$d_6$) $\delta$7.25 -7.20 (m, 2H), 7.19 -7.15 (m, 2H), 7.14 -7.07 (m, 2H), 7.00 -6.96 (m, 2H), 6.93 (t, 1H), 4.72 (q, 2H), 4.39 (s, 2H), 4.20 (d, 2H), 3.96 (s, 1H), 2.88 (d, 2H), 2.17 (d, 2H), 1.43 (d, 4H), 1.24 (d, 1H), 0.36 (d, 2H), 0.21 (s, 2H). |
| 73 | 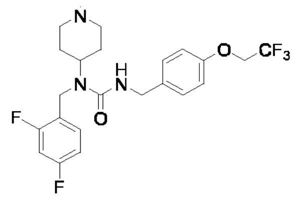 | MS m/z (ESI): 472.2[M+1]$^1$H NMR (600 MHz, DMSO-$d_6$) $\delta$7.24 -7.15 (m, 4H), 7.15 -7.08 (m, 1H), 7.03 (m, 1H), 6.97 (d, 2H), 4.72 (d, 2H), 4.40 (s, 2H), 4.19 (d, 2H), 4.14 -4.04 (m, 1H), 2.97 (s, 2H), 2.50 (m, 3H), 2.34 (s, 2H), 1.71 (s, 2H), 1.56 (d,2H). |
| 74 | 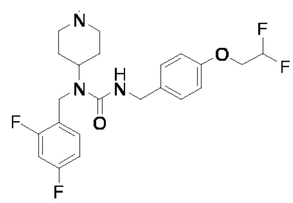 | MS m/z (ESI): 454.1[M+1]$^1$H NMR (600 MHz, DMSO-$d_6$) $\delta$7.24 -7.18 (m, 2H), 7.16 (d, 2H), 7.13 (s, 1H), 7.04 (td, 1H), 6.93 (d, 2H), 6.38 (m, 1H), 4.41 (s, 2H), 4.28 (d, 2H), 4.19 (d, 3H), 3.21 -3.08 (m, 2H), 2.64 (d,2H),2.51 (p, 3H), 1.94 -1.80 (m, 2H), 1.62 (d, 2H). |
| 78 | 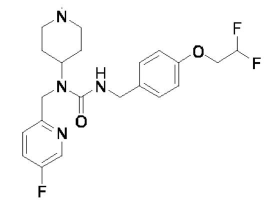 | MS m/z (ESI): 437.2[M+1]$^1$H NMR (600 MHz, $CDCl_3$) $\delta$ 8.23 (d, 1H), 7.56 (m, 2H), 7.40 (m, 1H), 7.26 -7.22 (m, 2H), 6.88 -6.82 (m, 2H), 6.07 (s, 1H), 4.50 (t, 1H), 4.43 (s, 2H), 4.36 (d, 2H), 4.16 (m, 2H), 3.37 -3.29 (m, 2H), 2.67(s,2H), 2.64 (s, 2H), 2.43 (m, 3H), 1.78 -1.66 (m, 2H). |

(continued)

| Compound number | Structural formula | Characterization data |
|---|---|---|
| 82 | 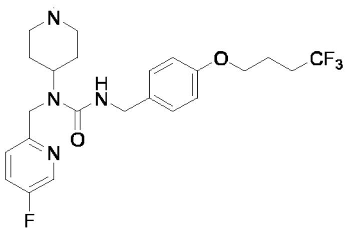 | MS m/z (ESI): 483.2[M+1] $^1$H NMR (600 MHz, DMSO-$d_6$) $\delta$ 8.48 (d, 1H), 7.77 -7.57 (m, 1H), 7.33 (m, 1H), 7.21 -7.08 (m, 3H), 6.96- 6.82 (m, 2H), 4.46 (s, 2H), 4.18 (d, 2H), 4.16 -4.12 (m, 1H), 4.00 (t, 2H), 3.07 (s, 2H), 2.50 (m, 3H), 2.48 -2.36 (m, 2H), 1.96 -1.88 (m, 2H), 1.87 -1.75 (m, 2H), 1.58 (d, 2H), 1.42 -1.21 (m, 2H). |

**Example 11:**

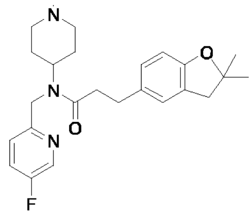

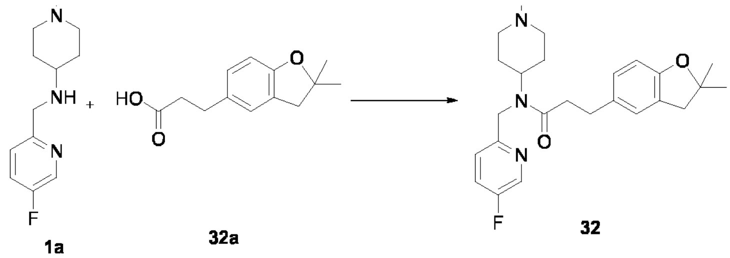

1a

32a

32

Under nitrogen protection, compound **1a** (446 mg, 2.0 mmol) was dissolved in 10 ml of acetonitrile. Compound **32a** (440 mg, 2.0 mmol), HATU (760 mg, 2.0 mmol) and diisopropylethylamine (387 mg, 3.0 mmol) were added, and the resulting mixture was heated to 60°C and reacted under stirring for 12 h. The reaction solution was cooled to room temperature and filtered. 20 ml of water was added to the filtrate, and then the resulting solution was extracted with dichloromethane (10 mL * 3). The organic phases were combined, dried over anhydrous sodium sulfate, filtered, concentrated under reduced pressure and separated by column chromatography (dichloromethane:methanol = 10 : 1) to obtain **compound 32** (561 mg, yield: 66%). MS m/z (ESI): 426.2 [M+1]; $^1$H NMR(400 MHz, DMSO-$d_6$) $\delta$ 8.50(s, 1H), 7.72-7.64(m, 1H), 7.26-7.20(m, 1H), 6.99-6.87(m, 2H),6.62-6.50(m, 1H), 4.53(s, 2H), 4.38-4.24(m, 1H), 2.97-2.92 (m, 2H), 2.80-2.61(m, 5H), 2.55-2.52(m, 1H), 2.12(s, 3H), 1.98-1.88(m, 2H), 1.65-1.30(m, 10H).

Compounds 53 and 54 were prepared in a similar manner to compound 32.

Table 4: Structures and characterization data of compounds 53 and 54

| Compound number | Structural formula | Characterization data |
|---|---|---|
| 53 | 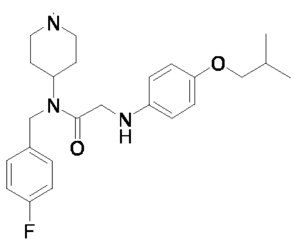 | MS m/z (ESI): 428.3 [M+1]; $^1$H NMR(400 MHz, DMSO-$d_6$) $\delta$ 7.34-7.27(m, 2H), 7.18-7.09(m, 2H), 6.75-6.68(m, 2H), 6.62-6.50 (m, 2H), 4.99(s, 1H), 4.55(s, 2H), 3.96-3.76(m, 3H), 3.63(m, 2H), 2.72(d, 2H), 2.18(s, 3H), 2.03-1.85(m, 3H), 1.75-1.53(m, 4H), 0.92 (d, 6H). |

(continued)

| Compound number | Structural formula | Characterization data |
|---|---|---|
| 54 | 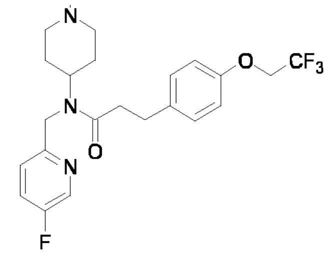 | MS m/z (ESI): 454.2 [M+1]; $^1$H NMR(400 MHz, DMSO-$d_6$) δ 8.5 (d, 1H), 7.74-7.67(m, 1H), 7.38-7.19(m, 2H), 7.12-7.07(m, 1H), 6.99-6.88(m, 2H), 4.75-4.65(m, 2H), 4.60-4.50(m, 2H), 4.40-4.31 (m, 1H), 3.25-3.15(m, 2H), 2.90-2.75(m, 5H), 2.65-2.55(m, 4H), 1.85-1.75(m, 2H), 1.70-1.58(m, 2H). |

**Example 12:**

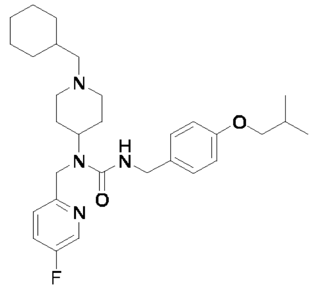

Synthesis route:

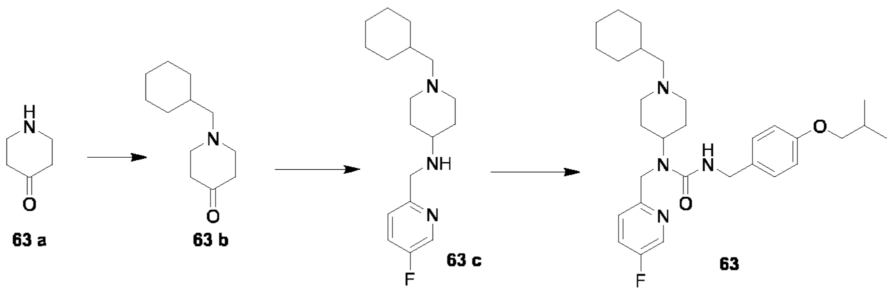

63 a 63 b 63 c 63

Under nitrogen protection, compound **63a** (1.212 g, 11.3 mmol) was dissolved in 10 ml of acetonitrile. Bromomethyl cyclohexane (2 g, 11.3 mmol) and potassium carbonate (4.68 g, 33.9 mmol) were added, and the resulting mixture was heated to 60°C and reacted under stirring for 12 h. The reaction solution was cooled to room temperature and filtered, and then the filtrate was extracted with dichloromethane (50 mL * 3). The organic phases were combined, dried over anhydrous sodium sulfate, filtered, concentrated under reduced pressure and separated by column chromatography (dichloromethane:methanol = 10 : 1) to obtain compound **63b** (1.49 g, a light yellow oily liquid).

Under nitrogen protection, compound **63b** (1.49 g, 7.63 mmol) was dissolved in 20 ml of methanol. (5-Fluoropyridin-2-methyl)amine (962.4 mg, 7.63 mmol) and sodium triacetoxyborohydride (1.972 g, 9.31 mmol) were added, and the resulting mixture was heated to room temperature and reacted for 15 h. An aqueous solution of $NaHCO_3$ was added to adjust the pH value to alkaline. The organic phase was concentrated and then extracted with dichloromethane (50 mL * 3). The organic phases were combined, dried over anhydrous sodium sulfate, filtered, concentrated under reduced pressure and subjected to column chromatography to obtain compound **63c** (217 mg).

Under nitrogen protection, compound **63c** (217 mg, 0.71 mmol) was dissolved in 10 ml of acetonitrile. N-(4-isobutyloxybenzyl)-1H-imidazol-1-formamide (194 mg, 0.710 mmol) and potassium carbonate (107.9 mg, 0.781 mmol)

were added, and the resulting mixture was heated to 60°C and reacted under stirring for 12 h. The reaction solution was cooled to room temperature and filtered, and then the filtrate was extracted with dichloromethane (30 mL * 3). The organic phases were combined, dried over anhydrous sodium sulfate, filtered, concentrated under reduced pressure and separated by column chromatography (dichloromethane : methanol = 10 : 1) to obtain **compound 63** (50.5 mg, a light yellow oily liquid, yield: 14%). MS m/z (ESI): 511.69[M+1]$^1$H NMR (600 MHz, $CDCl_3$) $\delta$ 8.24 (d ,1H), 7.37 (d, 2H), 7.17 (d, 2H), 6.90 -6.75 (m, 2H), 4.42 (s, 2H), 4.33 (d, 2H), 3.70 (d, 2H), 3.11 (s, 2H), 2.41 -2.14 (m, 1H), 2.11 -2.01 (m, 2H), 1.85 -1.63 (m, 10H), 1.46 -1.36 (m, 2H), 1.31 (d, 2H), 1.24 (d, 2H), 1.16 (s, 2H), 1.02 (s, 3H), 1.01 (s, 3H).

**Example 13:**

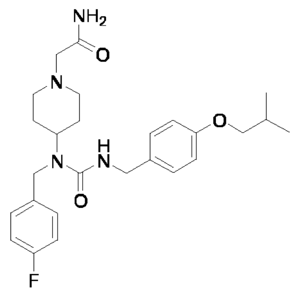

Synthesis route:

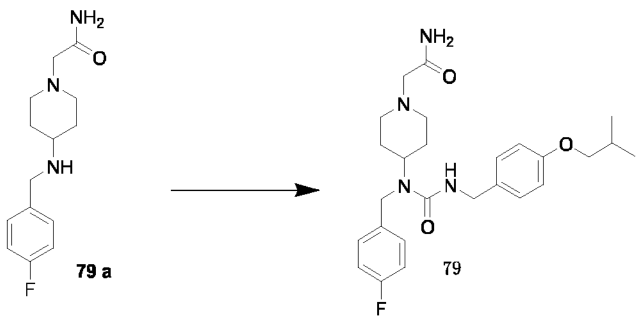

Under nitrogen protection, compound **79a** (200 mg, 0.75 mmol) was dissolved in 10 ml of acetonitrile. N-(4-isobutyloxybenzyl)-1H-imidazol-1-formamide (207 mg, 0.75 mmol) and potassium carbonate (105 mg, 0.75 mmol) were added, and the resulting mixture was heated to 60°C and reacted under stirring for 5 h. The reaction solution was cooled to room temperature and filtered, and then the filtrate was extracted with dichloromethane (20 mL * 3). The organic phases were combined, dried over anhydrous sodium sulfate, filtered, concentrated under reduced pressure and separated by column chromatography (dichloromethane : methanol = 10 : 1) to obtain **compound 79** (166 mg, a light yellow oily liquid, yield: 47%). MS m/z (ESI): 471.2[M+1]$^1$H NMR (600 MHz, $CDCl_3$) $\delta$ 7.20 (m, 2H), 7.01 (m, 4H), 6.92 (s, 1H), 6.81 -6.74 (m, 2H), 5.40 (s, 1H), 4.44 (t, 1H), 4.34 (s, 3H), 4.27 (d, 2H), 3.68 (d, 2H), 2.98 (s, 2H), 2.94 -2.87 (m, 2H), 2.31 (m, 2H), 2.06 (m, 1H), 1.80 - 1.74 (m, 2H), 1.66 (m, 2H), 1.01 (d, J = 6.7 Hz, 6H).

**Test example 1. In-vitro activity test of 5-$HT_{2A}$ receptor**

1. Screening for 5-$HT_{2A}$ inverse agonistic activity

1.1 Test materials:

Cell lines: Adherent cells NIH3T3-5-$HT_{2A}$ R

Cell culture medium: DMEM + 10% FBS (purchased from GBICO)
Cell culture plate: White-wall clear-bottom 96-well plate (purchased from Perkin Elmer)
Detection kit: Bright-Glo™ Luciferase (purchased from Promega)
Detection instrument: BioTek multifunctional microplate reader

1.2 Test drugs

Pimavanserin: Purchased from MCE Corporation
Other compounds: Prepared according to the preceding examples

1.3 Test method:

$NIH_3T3$-$5HT_{2A}R$ cells in the logarithmic growth phase were seeded into a white-wall clear-bottom 96-well plate at a density of 1000 cells per well, and cultured overnight in a 37°C, 5% $CO_2$ incubator. The next day, a compound to be tested was added to the cells, wherein the compound to be tested was subjected to 3.16-fold gradient dilution with PBS to obtain 9 concentrations, with the highest concentration of 10 uM, and double replicate wells were set up for each concentration; and PBS was used as a negative control group, and pimavanserin with the same concentration was used as a positive control group. After the addition, the cells were cultured in a 37°C, 5% $CO_2$ incubator for another 120 h. On the sixth day, a Bright-Glo™ Luciferase reagent with a volume equal to that of cell sap was added to the cells, and the cells were incubated at room temperature in the dark for 20 min. The plate was shaken every 5 min with a plate shaker, the luminescent intensity was detected by a microplate reader, and the cell inhibition rate was calculated. The data was processed with GraphPad Prism 7.0 to obtain a cell inhibition rate curve, and the $IC_{50}$ was calculated. The test results were as shown in Table 5.

$$\text{Cell inhibition rate (\%)} = [100 - (\text{Lum test drug} - \text{Lum culture solution}) / (\text{Lum cell control} - \text{Lum culture solution}) \times 100]\%$$

2. Test for 5-HT2A antagonistic activity (i.e., calcium ion antagonism)

2.1 Test materials:

Cell lines: CHO-K1/5-$HT_{2A}$ (Chempartner)
Cell culture medium: DMEM/F12 + 10% FBS (purchased from GBICO)
Cell culture plate: 384-well Assay Plate (purchased from Corning)
Detection kit: FLIPR® Calcium 4 Assay Kit (purchased from Molecular Devices)

2.2 Test drugs

Pimavanserin: Purchased from MCE Corporation
Other compounds: Prepared according to the preceding examples

2.3 Test method:

CHO-K1/5-$HT_{2A}$ cells in the logarithmic growth phase were seeded into a 384-well plate at a density of 10000 cells per well, and cultured in a 37°C, 5% $CO_2$ incubator for 16-24 h. After that, centrifugation was performed to remove the culture medium in the cell culture plate. Dye was added, and the cells were incubated in a 37°C, 5% $CO_2$ incubator for another 1 h. The cell culture plate was placed on the FLIPR. A compound to be tested was added, and the $Ca^{2+}$ signal was detected, wherein the compound to be tested was subjected to 3-fold gradient dilution with PBS to obtain 10 concentrations, with the highest concentration of 10 uM, and double replicate wells were set up. 100 nM a-methyl-5-HT was added 15 min later, and the $Ca^{2+}$ signal was detected again, wherein the value obtained by only adding 100 nM a-methyl-5-HT was taken as the maximum signal. In order to determine the test stability, risperidone was used as a positive control. The data were processed with GraphPad Prism 7.0 to obtain a cell inhibition rate curve, and the $IC_{50}$ was calculated. The test results were as shown in Table 5.

$$\text{Inhibition rate (\%)} = 100\% - [(\text{signal value of the compound to be tested} - \text{signal value of the test solution}) / (\text{the maximum signal value} - \text{the signal value of the test solution})] \times 100\%$$

**Test example 2. hERG inhibitory activity**

1. Test materials and instruments

1.1 Positive control compound

Name: Cisapride

1.2 Solvent

Name: Dimethyl sulfoxide (DMSO)

1.3 Cells

Species & strains: CHO-hERG cell lines (Chinese hamster ovary cells stably expressing hERG channels)
Culture medium: 90% F12, 10% fetal bovine serum, 100 μg/mL G418 and 100 μg/mL hygromycin B
Culture conditions: 5% CO2, 37°C incubator
Freezing conditions: Liquid nitrogen

1.4 Test instruments

Patch clamp amplifier (Axoclamp 200B, Multiclamp 700B, Axon, US)
Digital analog converter (DigiData 1440A, DigiData 1550B, Axon, US)
Inverted microscope (IX51, IX71, Olympus, Japan)
Rapid drug delivery system (RSC-200, Bio-Logic, France)
Micromanipulator (MX7600R, Syskiyou, US)
Electrode puller (P-97, Sutter, US)
Glass electrode (BF150-86-10, Sutter, US)
Vibration isolation table and shielding mesh (63-534, TMC, US)
Data acquisition and analysis software (pClamp 10, Axon, US)
Carbon dioxide incubator (HERacell 150i, Thermo, US)
Biological safety cabinet (MODEL 1384, Thermo, US)
Water purifier (Milli Q, Millipore, US)

2. Test method

2.1 Cell culture and treatment

CHO cells stably expressing hERG were cultured in a cell culture dish with the diameter of 35 mm in a 37°C, 5% CO2 incubator, and passaged at a ratio of 1 : 5 every 48 h. On the day of the test, the cell culture solution was pipetted, the cells were rinsed once with extracellular fluid, and then a 0.25% Trypsin-EDTA (Invitrogen) solution was added. Digestion was performed at room temperature for 3-5 min. The digestion solution was pipetted, and the cells were resuspended in extracellular fluid and transferred to an experimental dish for electrophysiological recording for later use.

2.2 Compound preparation

On the day of the test, the compound was diluted with DMSO to an intermediate concentration. 10 μL of compound with the intermediate concentration was taken, transferred to 4990 μL of extracellular fluid, and subjected to 500-fold dilution to obtain a final concentration to be tested.

Preparation of positive control compound Cisapride: 10 μL of 150 μM Cisapride DMSO mother liquor was taken, transferred to 4990 μL of extracellular fluid, and subjected to 500-fold dilution to obtain a final concentration 300 nM to be tested.

2.3 Electrophysiological recording process

CHO (Chinese Hamster Ovary) cells stably expressing hERG potassium channels were taken, and hERG potassium channel currents were recorded at room temperature by means of a whole-cell patch-clamp technique. A glass microelectrode was formed by pulling a glass electrode blank (BF150-86-10, Sutter) with a glass microelectrode puller. The tip resistance was about 2-5 MΩ after perfusion of a pippette solution. The glass microelectrode could be connected to a patch clamp amplifier by inserting the glass microelectrode into an amplifier probe. Clamping voltages and data recording were controlled and recorded by a computer with pClamp 10 software, with a sampling frequency of 10 kHz and a filtering frequency of 2 kHz. After the whole-cell recording was obtained, the cells were clamped at -80 mV, and the step voltage evoking hERG potassium current ($I_{hERG}$) was changed from -80 mV to +20 mV by giving a 2 s depolarization voltage and then repolarized to -50 mV for 1 s, and returned to -80 mV. Such voltage stimulation was given every 10 s, and the administration process was started after it was determined that the hERG potassium currents were stable (1 min). The compound at each test concentration was given at least 1 min, and at least 2 cells ($n \geq 2$) were tested for each concentration.

2.4 Data processing and analysis

Data analysis was performed by means of pClamp 10 and GraphPad Prism 5.0 software.

The formula for calculating the degree of inhibition of different compound concentrations on hERG potassium currents (the hERG tail current peak value evoked at - 50 mV) was:

$$\text{Inhibition \%} = [1 - (I/Io)] \times 100\%$$

wherein Inhibition % represents the percentage of inhibition of hERG potassium currents by the compound, and I and Io represent the amplitudes of hERG potassium current after administration and before administration, respectively.

Compound $IC_{50}$ was calculated via the following equation fitting by means of GraphPad Prism 5 software, and the test results were as shown in Table 5:

$$Y = \text{Bottom} + (\text{Top} - \text{Bottom}) / (1+10\text{^}((\text{LogIC}_{50} - X) * \text{HillSlope}))$$

wherein X is a Log value of a test concentration of a test sample, Y is the inhibition percentage at a corresponding concentration, and Bottom and Top are the minimum inhibition percentage and the maximum inhibition percentage, respectively.

**Test results**

Table 5 In-vitro test results of compounds of the present invention

| **Compound number** | **5-$HT_{2A}$ antagonistic activity $IC_{50}$ (nM)** | **5-$HT_{2A}$ inverse agonistic activity $IC_{50}$ (nM)** | **hERG inhibitory activity $IC_{50}$ (μM)** |
|---|---|---|---|
| Pimavanserin | 27.3 | 50 | 0.41 |
| 1 | 8.64 | 22.80 | 0.41 |
| 3 | 11.6 | -- | > 10 |

(continued)

| Compound number | 5-$HT_{2A}$ antagonistic activity $IC_{50}$ (nM) | 5-$HT_{2A}$ inverse agonistic activity $IC_{50}$ (nM) | hERG inhibitory activity $IC_{50}$ (μM) |
|---|---|---|---|
| 13 | 1.92 | -- | 1.3 |
| 14 | 2.17 | -- | -- |
| 17 | 1.93 | 3.05 | 1.55 |
| 18 | 5.75 | 1.12 | 0.85 |
| 19 | 6.94 | 49.85 | 0.48 |
| 20 | 9.14 | -- | 0.68 |
| 21 | 2.1 | 34.15 | 0.91 |
| 23 | 6.02 | 5.64 | > 10 |
| 24 | 3.49 | 2.54 | 3.61 |
| 25 | 6.73 | 16.87 | 1.03 |
| 26 | 6.86 | 9.79 | 0.53 |
| 30 | 9.92 | 10.66 | -- |
| 31 | 17.4 | 30.04 | 1.85 |
| 37 | 2.05 | 42.78 | 3.99 |
| 38 | -- | 10.04 | -- |
| 41 | 9.98 | 4.29 | 0.79 |
| 45 | 2.42 | 7.04 | 5.5 |
| 46 | 4.15 | 11.06 | 10.4 |
| 47 | 3.13 | 9.22 | 1.39 |
| 48 | 3.14 | 22.5 | 2.4 |
| 49 | 4.1 | 7.3 | 2.43 |
| 50 | 20.9 | -- | 0.55 |
| 52 | -- | 42.74 | -- |
| 55 | 4.7 | 2.23 | 0.69 |
| 56 | 5.77 | 9.90 | -- |
| 57 | 6.41 | 6.83 | -- |
| 59 | 0.54 | 2.04 | 0.81 |
| 60 | 2.11 | 16.4 | > 10 |
| 61 | 3.32 | 31.21 | 2.55 |
| 62 | 2.35 | 32.8 | 1.43 |
| 63 | -- | 20.84 | -- |
| 65 | 7.98 | 18.4 | 0.67 |
| 66 | 12 | 8.85 | -- |
| 70 | 8.09 | 12.33 | 0.5 |
| 71 | 9.52 | -- | 3.32 |
| 74 | 8.95 | -- | -- |
| 75 | 8.35 | 14.37 | -- |

(continued)

| Compound number | 5-$HT_{2A}$ antagonistic activity $IC_{50}$ (nM) | 5-$HT_{2A}$ inverse agonistic activity $IC_{50}$ (nM) | hERG inhibitory activity $IC_{50}$ ($\mu$M) |
|---|---|---|---|
| 76 | 20.68 | -- | -- |
| 77 | 6.59 | 6.44 | -- |
| 78 | 2.17 | 6.01 | 1.74 |
| 81 | 2.78 | 30.06 | 0.68 |
| 82 | 3.78 | 1.95 | 0.5 |
| 89 | 7.72 | -- | 2.05 |
| 91 | 14.7 | -- | 0.58 |
| 92 | 4.64 | 6.54 | -- |
| 93 | 4.26 | 10.58 | -- |
| 94 | 4.29 | 7.59 | -- |
| 95 | 5.13 | 27.92 | -- |
| 96 | 11.39 | 26.38 | -- |
| 98 | 21.25 | -- | 1.46 |
| 99 | 17.27 | 33.34 | 0.65 |

The results show that:
the compounds of the present invention have superior 5-$HT_{2A}$ antagonistic activity and/or 5-$HT_{2A}$ inverse agonistic activity to those of pimavanserin, and have lower cardiotoxicity.

**Test example 3. In-vitro stability evaluation of pimavanserin and compound 59 in liver microsomes**

1 Solution formulation

1) formulation of test sample working solution: the test sample was diluted to 100 $\mu$M with methanol;
2) formulation of liver microsome working solution: liver microsomes were diluted to 0.56 mg/ml with a 100 mM phosphate buffer;
3) formulation of reduced nicotinamide adenine dinucleotide phosphate (NADPH) working solution: an appropriate amount of NADPH was weighted and diluted to 20 mM with a phosphate buffer, and then a 60 mM $MgCl_2$ solution with the equal volume was added;
4) formulation of stop solution: tolbutamide was diluted to 20 ng/mL with acetonitrile to serve as a stop solution containing an internal standard.

2 Incubation process

1) anti-adsorption EP tubes for incubation were prepared, and species, test samples, reference substances (testosterone and dextromethorphan), time points (0 min, 5 min, 10 min, 20 min, 30 min, 60 min, Blank60 and NCF60), etc. were labeled;
2) 2 $\mu$L of test sample or reference substance working solution and 178 $\mu$L of liver microsome working solution were added to each tube, 2 $\mu$L of acetonitrile in place of the test samples was added to the Blank tube, and the mixture was placed in a 37°C water bath kettle and pre-incubated for about 10 min, wherein each sample was divided in triplicate aliquots;
3) after the pre-incubation was completed, 20 $\mu$L of NADPH working solution was added to each tube except for 0 min and NCF60 to initiate the reaction, and 20 $\mu$L of phosphate buffer (containing 30 mM $MgCl_2$) was added to the NCF60 tube, wherein in the incubation system, the test sample or the reference substance had a final concentration of 1 $\mu$M, the liver microsomes had a final concentration of 0.5 mg/mL, the NADPH had a final concentration of 1 mM, and the

$MgCl_2$ had a final concentration of 3 mM;
4) 600 μL of stop solution was added to the 0-min sample followed by an NADPH working solution, and 600 μL of stop solution was added to stop the reaction after each sample was incubated for a corresponding period of time;
5) each sample was vortexed for 30 s after the reaction was stopped and then centrifuged at 13500 rpm for 10 min; 100 μL of supernatant was added to an EP tube, 100 μL of Milli-Q water was added, and the resulting mixture was vortexed and mixed uniformly; and LC-MS/MS analysis was performed by using the method in Table 6; and
6) testosterone and dextromethorphan were used as positive controls under the same conditions to test the stability and reliability of the system.

Table 6 LC-MS-MS analysis methods

<table>
<tr><td>Analyte</td><td colspan="6">Pimavanserin, compound 59, testosterone and dextromethorphan</td></tr>
<tr><td colspan="7">Liquid-phase method</td></tr>
<tr><td>Mobile phase A</td><td colspan="6">0.1% formic acid in water</td></tr>
<tr><td>Mobile phase B</td><td colspan="6">0.1% formic acid in methanol</td></tr>
<tr><td>Chromatographic column</td><td colspan="6">ACQUITY UPLC BEH C18 2.1 × 50 mm, particle size 1.7 uM Waters</td></tr>
<tr><td>Internal standard</td><td colspan="6">Tolbutamide</td></tr>
<tr><td>Injection volume</td><td colspan="6">5 μL</td></tr>
<tr><td rowspan="7">Gradient</td><td>Time (min)</td><td colspan="2">Flow rate (μL/min)</td><td colspan="2">A (%)</td><td>B (%)</td></tr>
<tr><td>0.00</td><td colspan="2">0.5</td><td colspan="2">80</td><td>20</td></tr>
<tr><td>1.00</td><td colspan="2">0.5</td><td colspan="2">80</td><td>20</td></tr>
<tr><td>1.10</td><td colspan="2">0.5</td><td colspan="2">10</td><td>90</td></tr>
<tr><td>2.00</td><td colspan="2">0.5</td><td colspan="2">10</td><td>90</td></tr>
<tr><td>2.30</td><td colspan="2">0.5</td><td colspan="2">80</td><td>20</td></tr>
<tr><td>3.00</td><td colspan="2">0.5</td><td colspan="2">80</td><td>20</td></tr>
<tr><td colspan="7">Mass spectrometry method</td></tr>
<tr><td>Scanning mode</td><td colspan="6">MRM</td></tr>
<tr><td>Compound name</td><td>Ion pair</td><td>DP (eV)</td><td colspan="2">EP (eV)</td><td>CE (eV)</td><td>CXP (eV)</td></tr>
<tr><td>Pimavanserin</td><td>428.2/223.2</td><td>84</td><td colspan="2">11</td><td>24</td><td>18</td></tr>
<tr><td>Compound 59</td><td>429.2/224.2</td><td>50</td><td colspan="2">6</td><td>23</td><td>8</td></tr>
<tr><td>Testosterone</td><td>289.1/97.0</td><td>99</td><td colspan="2">14</td><td>29</td><td>8</td></tr>
<tr><td>Dextromethorphan</td><td>272.2/171.1</td><td>130</td><td colspan="2">10</td><td>46</td><td>7</td></tr>
<tr><td>Tolbutamide</td><td>271.1/155.3</td><td>30</td><td>8</td><td>21</td><td colspan="2">7</td></tr>
</table>

3 Data analysis

The remaining percentage of the test sample was tested 60 min later. The test results were as shown in Table 7.

Table 7 Test data of compounds of the present invention in human and rat liver microsomes

| Compounds | Species | Remaining amount 60 min later % |
|---|---|---|
| Pimavanserin | Dog | 29.72 |
| | Human | 77.44 |
| Compound 59 | Dog | 76.37 |
| | Human | 89.19 |

The results show that: compound 59 has obviously superior stability to that of pimavanserin in dog and human liver microsomes in vitro, and has better druggability.

**Test example 4. In-vivo pharmacokinetic test of pimavanserin and compound 59 in rats**

1 Test drugs

Pimavanserin: Purchased from MCE Corporation.
Compound 59: Prepared according to the previous examples

2 Test method

Eight SD rats weighting about 220 g were randomly divided into 2 groups, 4 rats per group, fasted for 12 h before administration, and intragastrically given pimavanserin and compound 59 at a dose of 46.7 μmol/kg, respectively, with vehicles both being 20% Solutol. Blood was collected before administration and 0.25 h, 0.5 h, 1 h, 2 h, 3 h, 4 h, 6 h, 8 h, 12 h and 24 h after administration, respectively and placed in a heparinized EP tube, and the plasma was centrifugally separated. After the plasma was pre-treated, the concentrations of compounds in the plasma were determined by LC-MS/MS, and the pharmacokinetic parameters were calculated. The test results were as shown in Figure 1 and Table 8.

Table 8 Comparison of pharmacokinetic parameters of pimavanserin and compound 59 after intragastric administration

| Parameter | Unit | Pimavanserin | Compound 59 |
|---|---|---|---|
| $T_{max}$ | h | 2.75 ± 1.26 | 5.88 ± 5.14 |
| $C_{max}$ | nmol/L | 536 ± 260 | 1135 ± 365 |
| $AUC_{last}$ | nmol/L * h | 4286 ± 1664 | 16987 ± 2969 |
| $T_{1/2}$ | h | 2.38 ± 0.8 | 5.51 ± 1.33 |
| $MRT_{last}$ | h | 5.61 ± 0.34 | 8.66 ± 0.89 |

The results show that: the $T_{1/2}$ of compound 59 is higher than that of pimavanserin; the $C_{max}$ of compound 59 is 2 times that of pimavanserin; the $AUC_{last}$ of compound 59 is 4 times that of pimavanserin; and the in-vivo pharmacokinetic properties of compound 59 are obviously better than those of pimavanserin.

## Claims

**1.** A compound of formula (I) or a pharmaceutically acceptable salt thereof,

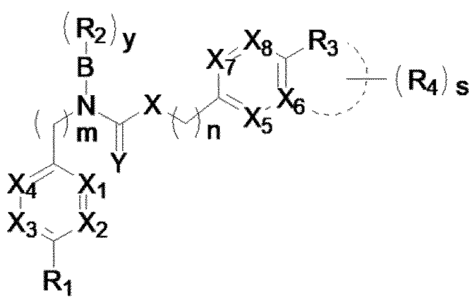

(I)

wherein

at least one of $X_1$ and $X_4$ is N, and the other one is optionally $CR_1$ or N;
$X_2$ and $X_3$ are each independently selected from $CR_1$ and N;

$X_5$ is independently selected from $CR_{3a}$ or N;
$X_6$ is independently selected from $CR_{3b}$ or N;
$X_7$ is independently selected from $CR_{3c}$ or N;
$X_8$ is independently selected from $CR_{3d}$ or N;
group B is linear or branched $C_{1-6}$ alkyl or a 5-6-membered nitrogen heterocyclic group, and the linear or branched $C_{1-6}$ alkyl or the 5-6-membered nitrogen heterocyclic group is optionally substituted with one or more deuterium atoms;
each $R_1$ is the same or different and is independently selected from a hydrogen atom, linear or branched $C_{1-10}$ alkyl or halogen;
each $R_2$ is the same or different and is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;
$R_3$, $R_{3a}$, $R_{3b}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;
or when $X_6$ is $CR_{3b}$, $X_6$ and $R_3$ together with the atoms to which they are attached form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy;
X is selected from -NH- or $-(CH_2)_{1-4}NH-$;
Y is selected from O or S;
m and n are independently selected from 0, 1, 2 and 3;
s is independently selected from 1, 2, 3, 4, 5 and 6; and
y is independently selected from 0, 1, 2, 3, 4 and 5.

**2.** A compound of formula (II) or a pharmaceutically acceptable salt thereof,

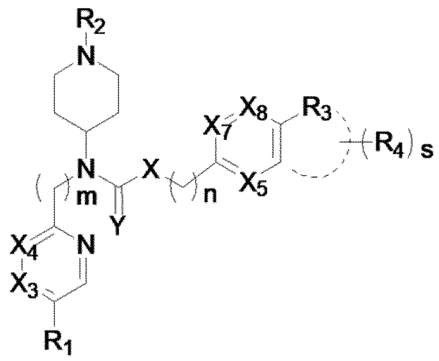

(II)

wherein

$X_3$ and $X_4$ are each independently selected from $CR_1$ and N;
$X_5$ is independently selected from $CR_{3a}$ or N;
$X_7$ is independently selected from $CR_{3d}$ or N;
$X_8$ is independently selected from $CR_{3d}$ or N;
each $R_1$ is the same or different and is independently selected from a hydrogen atom, linear or branched $C_{1-10}$ alkyl or halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;

$R_3$, $R_{3a}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;

or $R_3$ and the carbon atom to which it is attached together with adjacent carbon atoms form a ring system which is selected from dihydrofuran, pyrroline or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy;

X is selected from -NH- or $-(CH_2)_{1-4}NH-$;

Y is selected from O or S;

m and n are independently selected from 0, 1, 2 and 3; and

s is independently selected from 1, 2, 3, 4, 5 and 6.

**3.** A compound of formula (III) or a pharmaceutically acceptable salt thereof,

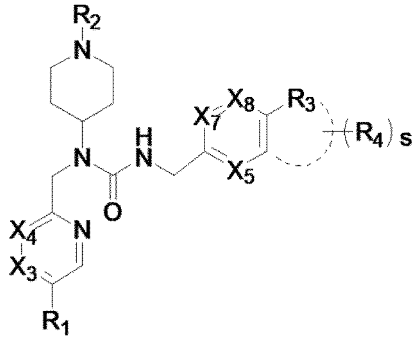

(III)

wherein

$X_3$ and $X_4$ are each independently selected from $CR_1$ and N;

$X_5$ is independently selected from $CR_{3a}$ or N;

$X_7$ is independently selected from $CR_{3d}$ or N;

$X_8$ is independently selected from $CR_{3d}$ or N;

each $R_1$ is the same or different and is independently selected from a hydrogen atom, linear or branched $C_{1-10}$ alkyl or halogen;

$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;

$R_3$, $R_{3a}$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;

or $R_3$ and the carbon atom to which it is attached together with adjacent carbon atoms form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy; and

s is independently selected from 1, 2, 3, 4, 5 and 6.

**4.** A compound of formula (IV) or a pharmaceutically acceptable salt thereof,

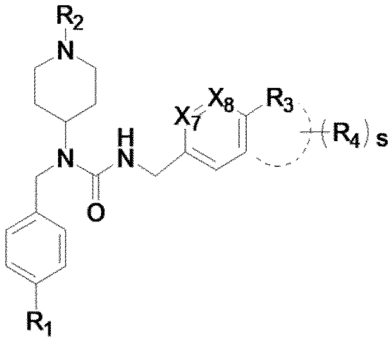

(IV)

wherein

$X_7$ is independently selected from $CR_{3d}$ or N;
$X_8$ is independently selected from $CR_{3d}$ or N;
$R_1$ is independently selected from a hydrogen atom, linear or branched $C_{1-10}$ alkyl or halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms;
$R_3$, $R_{3c}$ and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy;
or $R_3$ and the carbon atom to which it is attached together with adjacent carbon atoms form a ring system which is selected from dihydrofuran, dihydropyrrole or dihydrothiophene and are substituted with one or more of the same or different $R_4$, and each $R_4$ is independently selected from a hydrogen atom, halogen, linear or branched $C_{1-10}$ alkyl, linear or branched $C_{1-10}$ alkoxy or linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, hydroxyl and linear or branched $C_{1-10}$ alkoxy; and
s is independently selected from 1, 2, 3, 4, 5 and 6.

**5.** A compound of formula (V) or a pharmaceutically acceptable salt thereof,

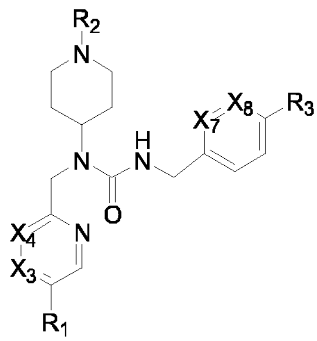

(V)

wherein

$X_3$ is independently selected from $CR_5$ or N;
$X_4$ is independently selected from $CR_6$ or N;
$X_7$ is independently selected from $CR_{3c}$ or N;
$X_8$ is independently selected from $CR_{3d}$ or N;
$R_1$, $R_5$ and $R_6$ are the same or different and are each independently selected from a hydrogen atom, linear or branched $C_{1-10}$ alkyl and halogen;

$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms; and

$R_3$, $R_3$, and $R_{3d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy.

**6.** A compound of formula (VI) or a pharmaceutically acceptable salt thereof,

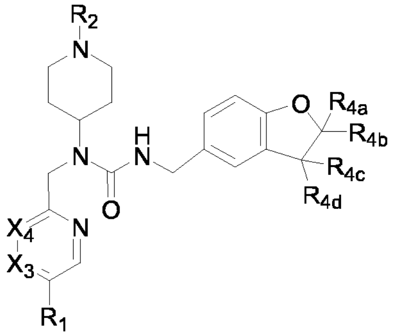

(VI)

wherein

$X_3$ is independently selected from $CR_5$ or N;
$X_4$ is independently selected from $CR_6$ or N;
$R_1$, $R_5$ and $R_6$ are the same or different and are each independently selected from a hydrogen atom, linear or branched $C_{1-10}$ alkyl and halogen;
$R_2$ is independently selected from a hydrogen atom, a deuterium atom, linear or branched $C_{1-10}$ alkyl, (linear or branched $C_{1-6}$ alkyl)$_2$ amine or 3-8-membered cycloalkyl, and the linear or branched $C_{1-10}$ alkyl, the (linear or branched $C_{1-6}$ alkyl)$_2$ amine or the 3-8-membered cycloalkyl is optionally substituted with one or more deuterium atoms; and
$R_{4a}$, $R_{4b}$ and $R_4$, and $R_{4d}$ are the same or different and are each independently selected from a hydrogen atom, halogen, hydroxyl, linear or branched $C_{1-10}$ alkyl, linear or branched $C_{1-10}$ alkoxy and linear or branched $C_{1-10}$ haloalkoxy, wherein the linear or branched $C_{1-10}$ alkyl and the linear or branched $C_{1-10}$ alkoxy are substituted with one or more substituents selected from a hydrogen atom, halogen, hydroxyl and linear or branched $C_{1-10}$ alkoxy.

**7.** The compound or the pharmaceutically acceptable salt thereof according to any one of claims 1 to 6, wherein

$R_1$, $R_5$ and $R_6$ are the same or different and are each independently selected from a hydrogen atom, F, Cl, Br, I, methyl, ethyl, propyl, isopropyl, butyl, isobutyl, sec-butyl and tert-butyl;
group B is $-CH_2-$, $-(CH_2)_2-$, $-(CH_2)_3-$, $-(CH_2)_4-$, $-CD_2-$, $-(CD_2)_2-$, $-(CD_2)_3-$ or $-(CD_2)_4-$, and $R_2$ is independently selected from dimethylamine or diethylamine, wherein the dimethylamine or the diethylamine is optionally substituted with one or more deuterium atoms;
or group B is selected from piperidinyl, wherein the piperidinyl is optionally substituted with one or more deuterium atoms, and $R_2$ is independently selected from a hydrogen atom, a deuterium atom, methyl, ethyl, propyl, isopropyl, butyl, isobutyl, sec-butyl, tert-butyl, cyclopropyl, cyclobutyl, cyclopentyl or cyclohexyl, wherein the methyl, the ethyl, the propyl, the isopropyl, the butyl, the isobutyl, the sec-butyl, the tert-butyl, the cyclopropyl, the cyclobutyl, the cyclopentyl or the cyclohexyl is optionally substituted with one or more deuterium atoms;
$R_3$, $R_{3a}$, $R_{3b}$, $R_{3c}$ and $R_{3d}$ are the same or different and are each independently selected from a hydrogen atom, F, Cl, Br, I, hydroxyl, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, oxy, ethoxy, propoxy, isopropoxy, butoxy, isobutoxy, sec-butoxy, tert-butoxy, pentyloxy, hexyloxy, fluoromethoxy, difluoromethoxy, trichloromethoxy, trifluoromethoxy, 2-fluoroethoxy, 2,2-difluoroethoxy, 2,2,2-trifluoroethoxy, tetrafluoroethoxy, pentafluoroethoxy, 3-fluoropropoxy, 3,3-difluoropropoxy, 2,2'-difluoroisopropoxy,

3,3,3-trifluoropropoxy, 4-fluorobutoxy, 4,4-difluorobutoxy, 4,4,4-trifluorobutoxy, 2-fluoro-2-methylpropyl, 5,5,5-trifluoropentyloxy, 6,6,6-trifluorohexyloxy, 2-methyl-3-hydroxy-butyl and i-Pr-O-$CH_2$-; and $R_4$ and/or $R_{4a}$, $R_{4b}$, $R_{4c}$ and $R_{4d}$ are the same or different and are each independently selected from a hydrogen atom, methyl, ethyl, propyl, isopropyl, butyl, isobutyl, sec-butyl and tert-butyl.

**8.** A compound or a pharmaceutically acceptable salt thereof or a deuterated analog thereof, which is selected from the following:

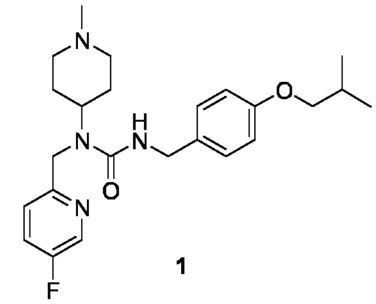
1

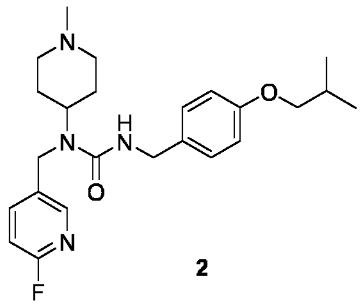
2

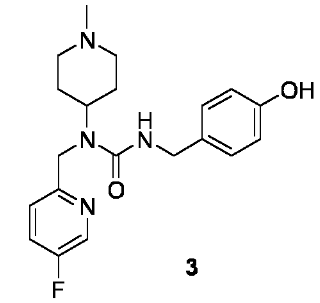
3

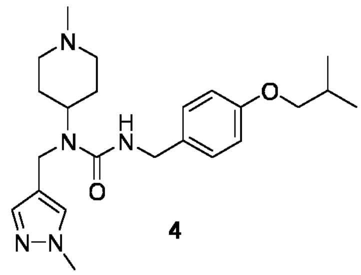
4

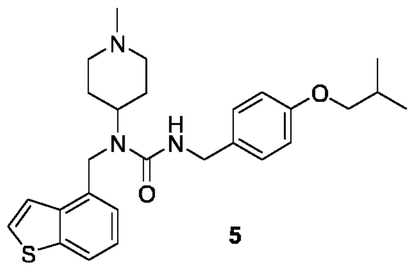
5

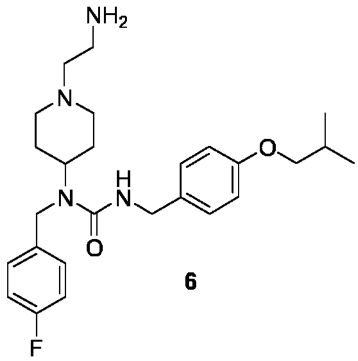
6

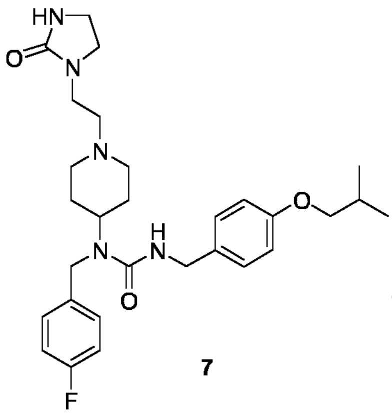
7

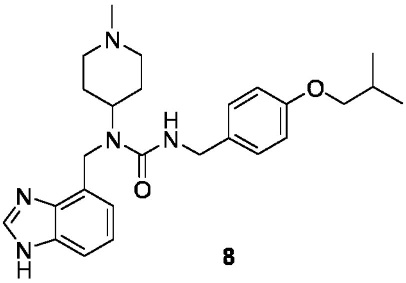
8

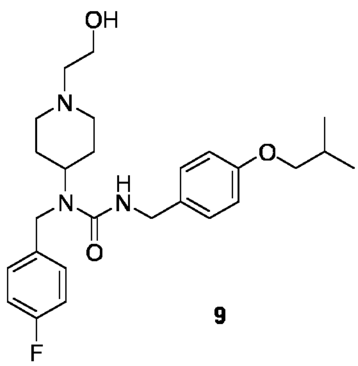
9

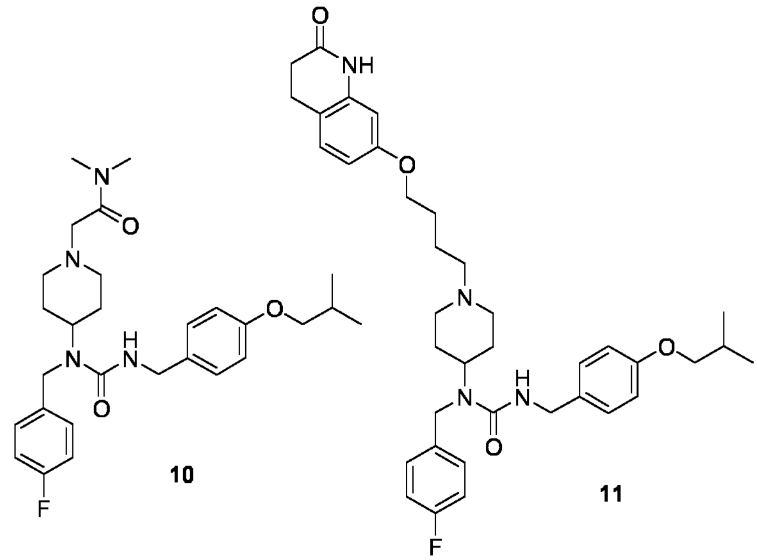

10

11

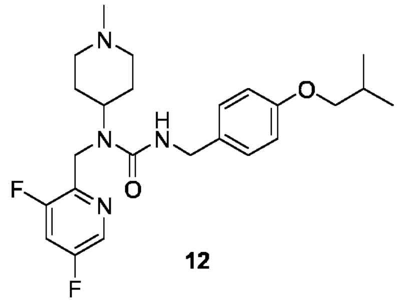

12

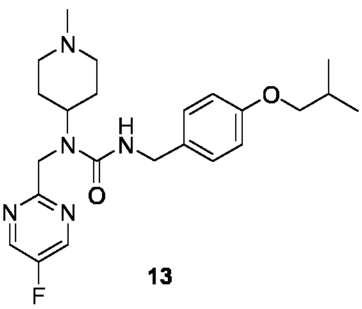

13

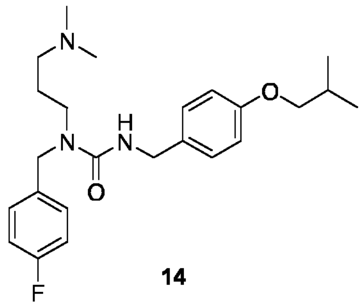

14

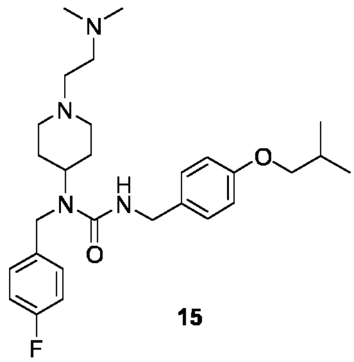

15

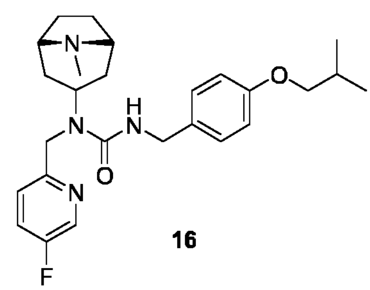

16

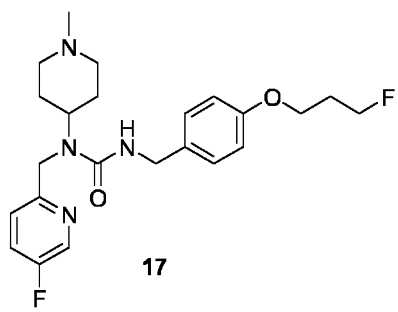

17

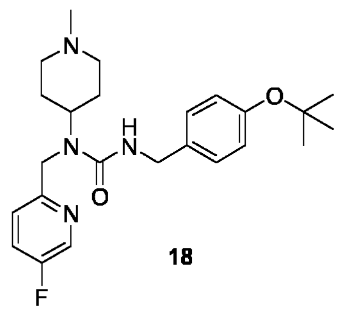

18

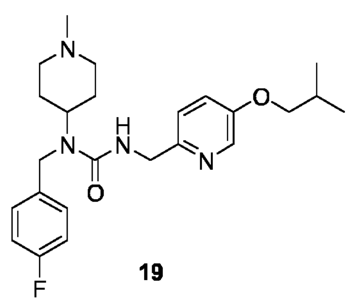

19

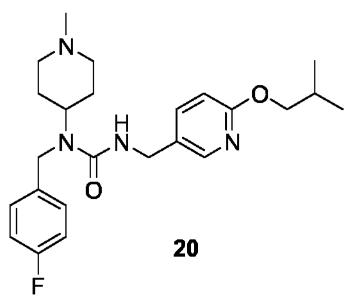

20

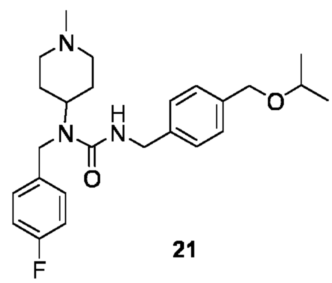

21

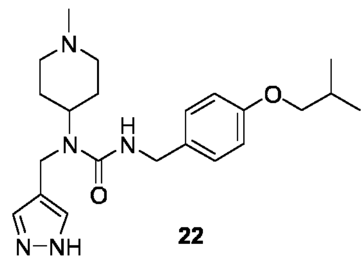

**22**

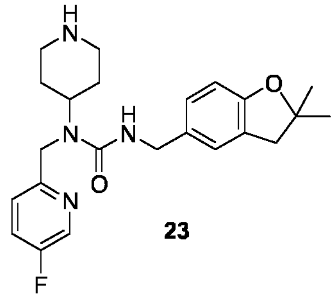

**23**

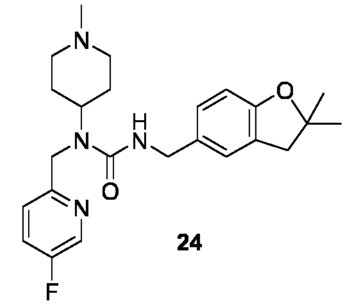

**24**

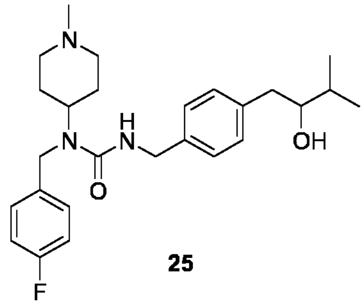

**25**

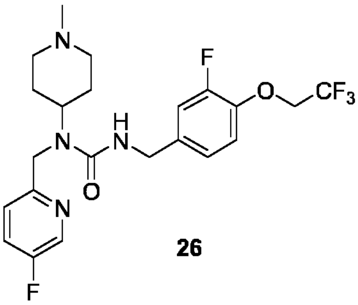

**26**

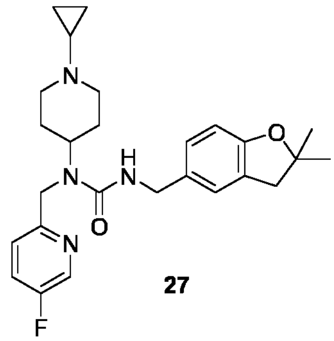

**27**

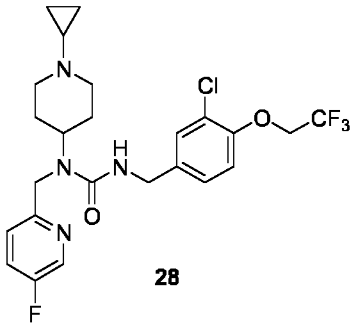

**28**

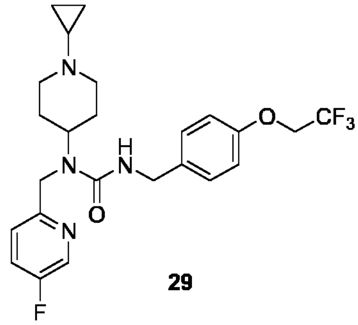

**29**

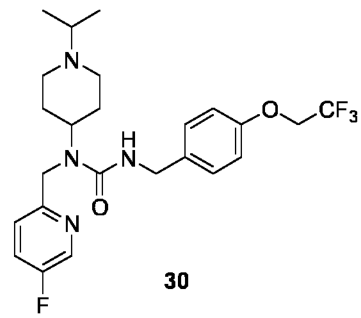

**30**

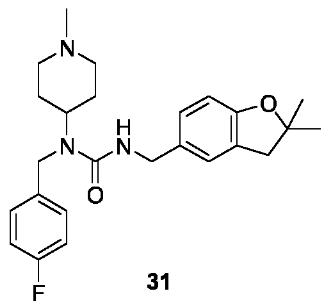

**31**

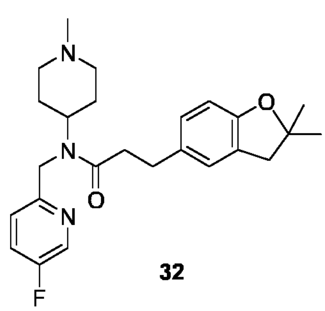

**32**

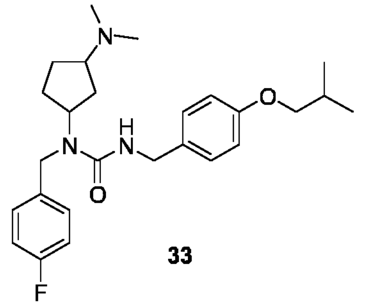

**33**

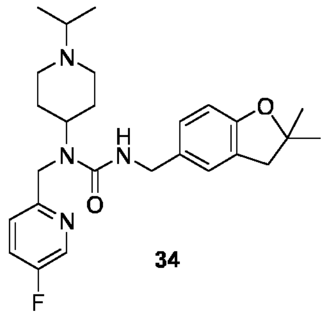

**34**

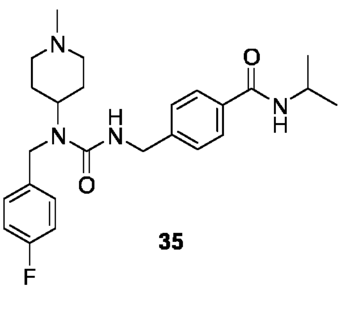

**35**

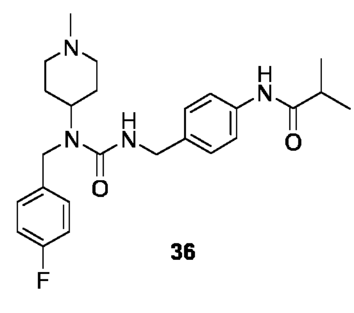

**36**

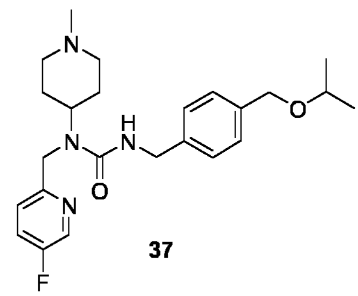
37

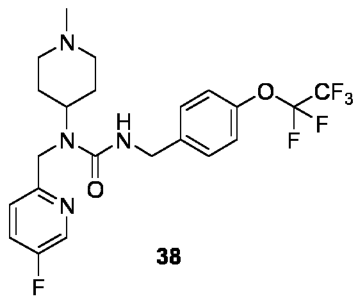
38

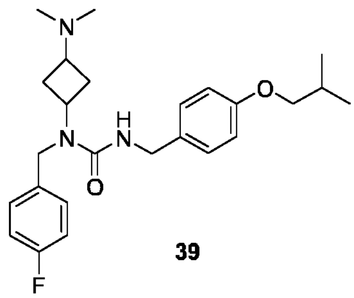
39

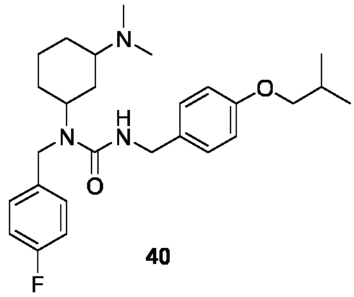
40

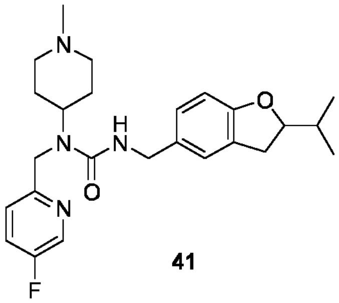
41

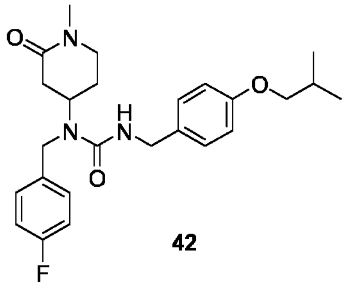
42

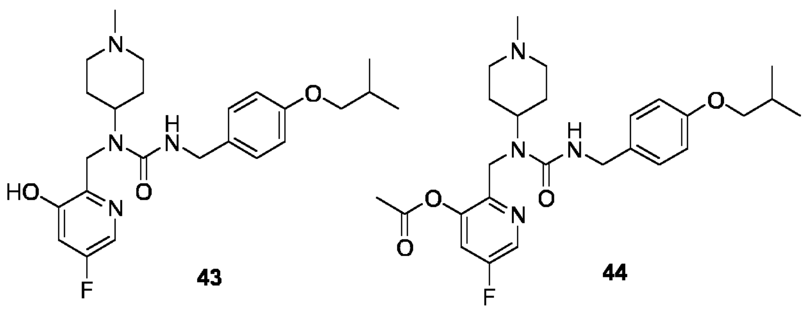
43

44

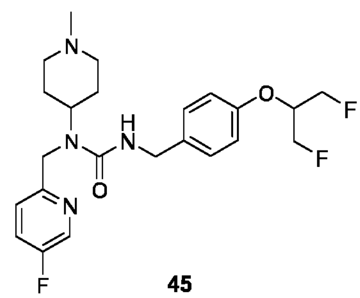
45

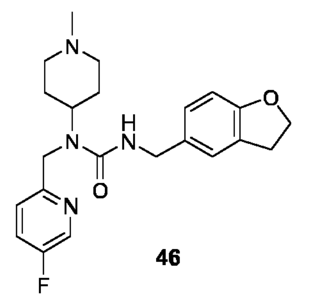
46

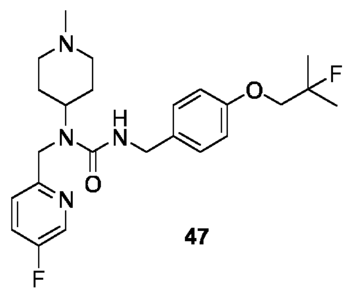
47

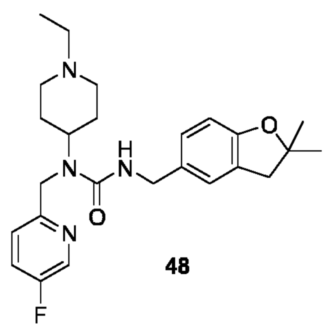
48

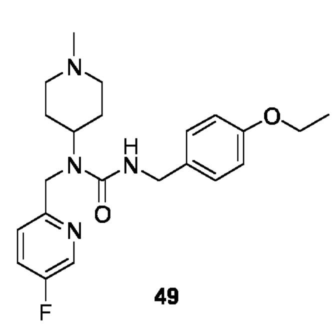
49

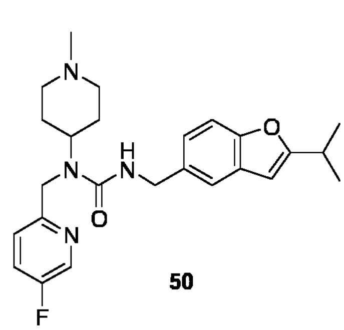
50

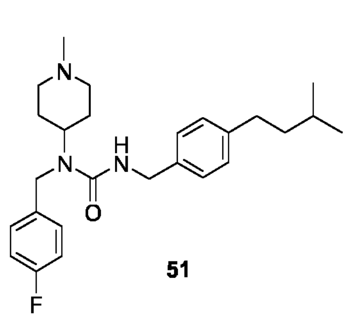
51

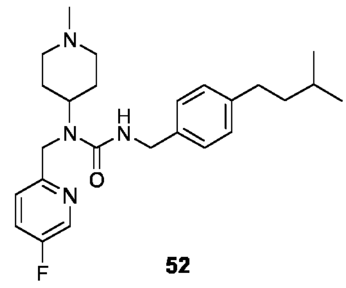
52

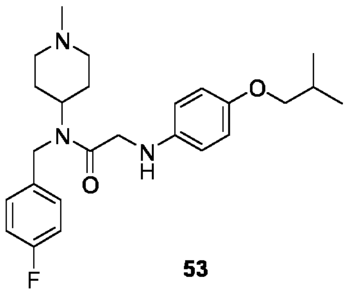
53

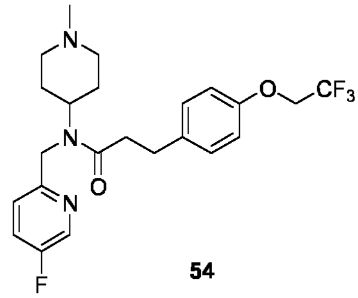
54

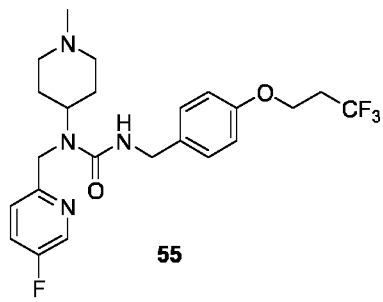
55

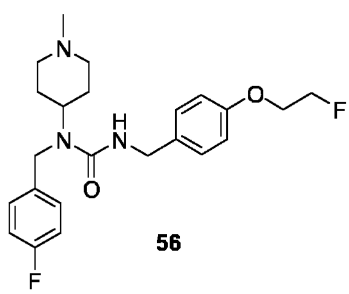
56

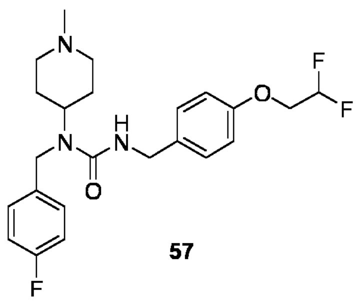
57

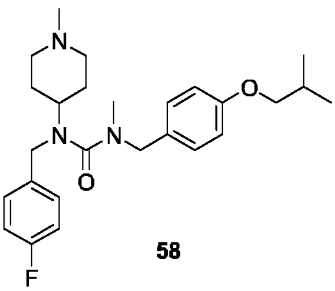
58

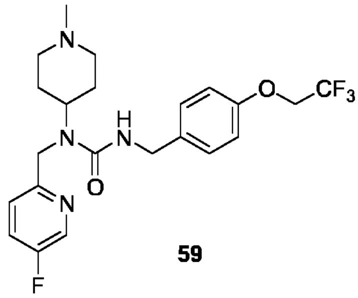
59

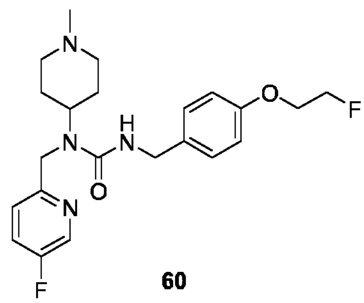
60

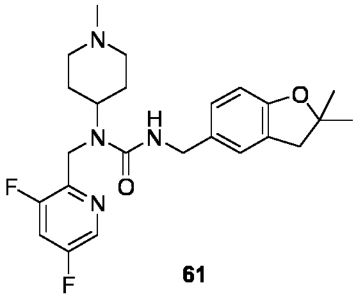
61

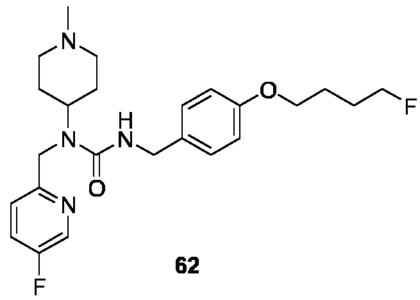
62

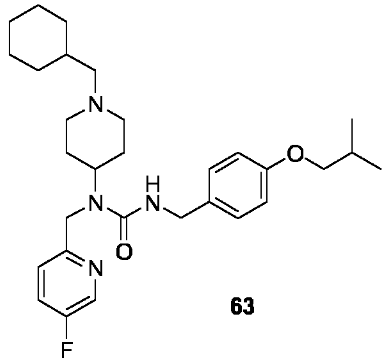
63

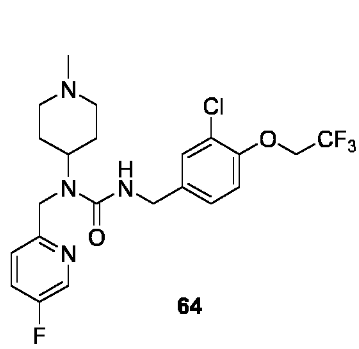
64

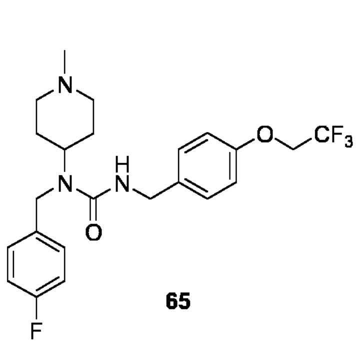
65

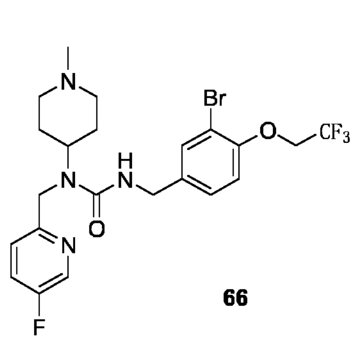
66

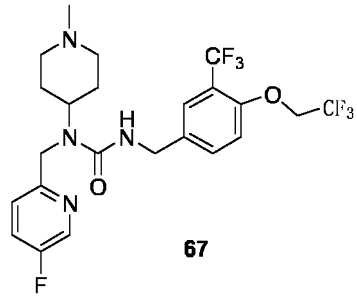
67

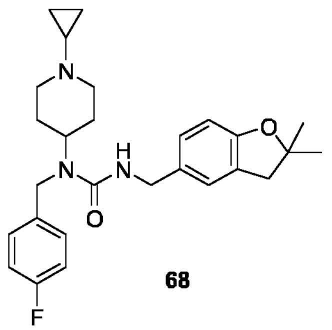
68

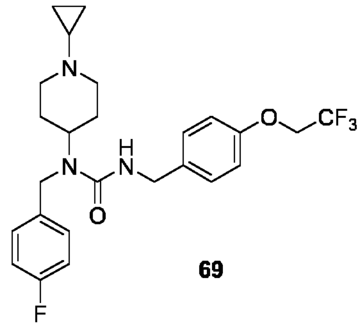
69

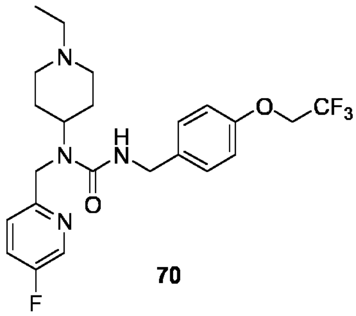
70

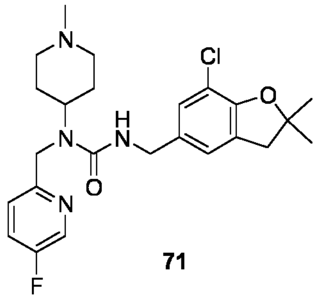
71

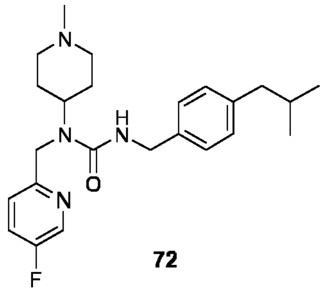
72

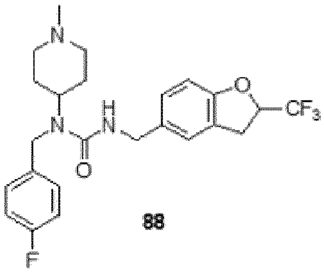
88

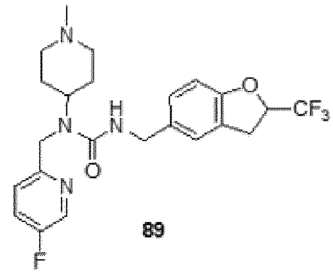
89

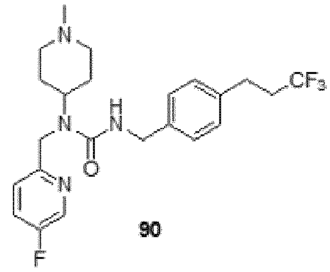
90

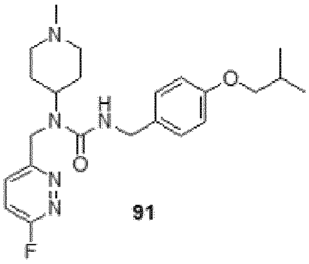
91

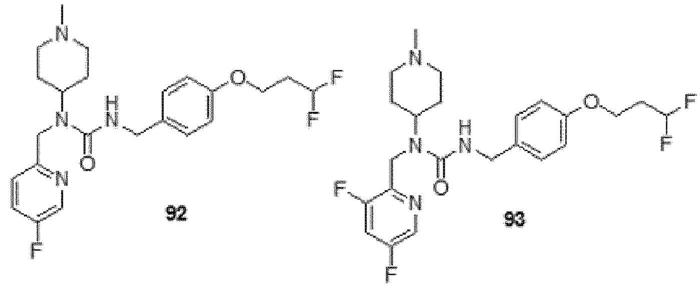
92

93

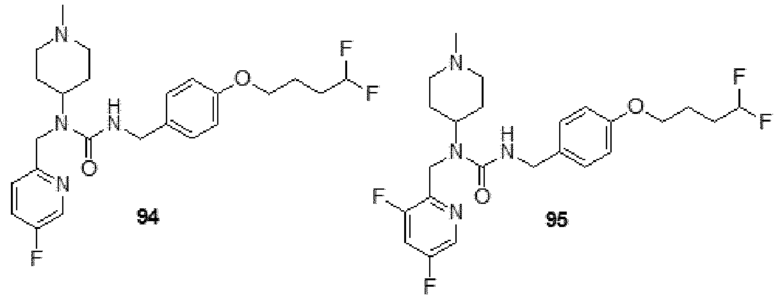
94

95

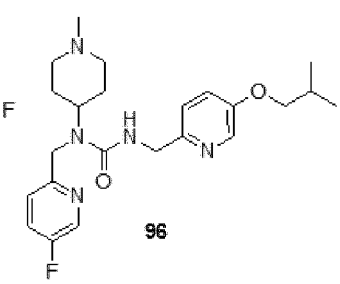
96

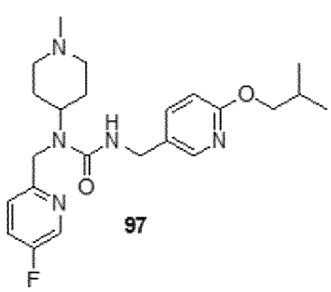 97 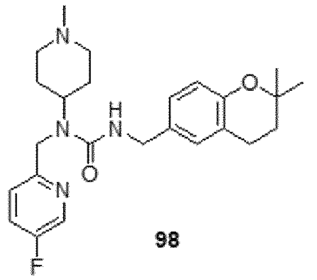 98 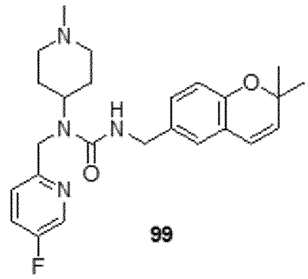 99

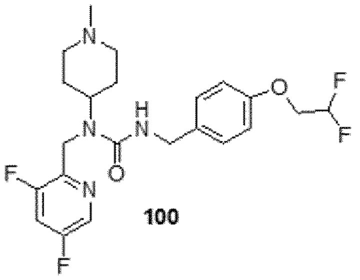 100 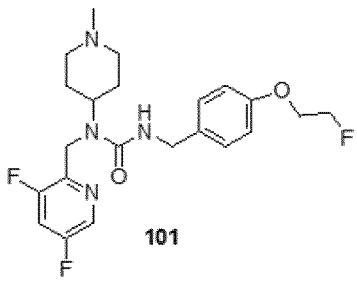 101 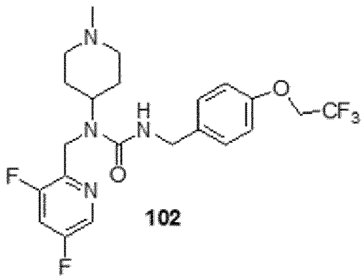 102 .

**9.** A pharmaceutical composition, comprising the compound or the pharmaceutically acceptable salt thereof according to any one of claims 1 to 8 and a pharmaceutically acceptable carrier.

**10.** Use of the compound or the pharmaceutically acceptable salt thereof according to any one of claims 1 to 8 or the pharmaceutical composition according to claim 9 in the preparation of a drug for treating 5-HT receptor-related diseases, wherein preferably the 5-HT receptor-related diseases comprise: schizophrenia, psychosis, schizoaffective disorder, mania, psychotic depression, affective disorder, dementia, anxiety disorder, sleep disorder, dysorexia, bipolar disorder, psychosis secondary to hypertension, migraine, hypertension, thrombosis, vasospasm, ischemia, motor tics, depression, major depressive disorder, anxiety, sleep disturbance, eating disorder, non-motor symptoms caused by Parkinson's disease, delusion, illusion, cognitive disorder, dementia-related mental diseases, negative symptoms of schizophrenia, Parkinson's disease, Huntington's disease, Alzheimer's disease, spinocerebellar ataxia, Tourette's syndrome, Friedreich's ataxia, Machado-Joseph disease, Lewy body dementia, dyskinesia, dysmyotonia, myoclonus, tremor or progressive supranuclear paralysis and frontotemporal dementia.

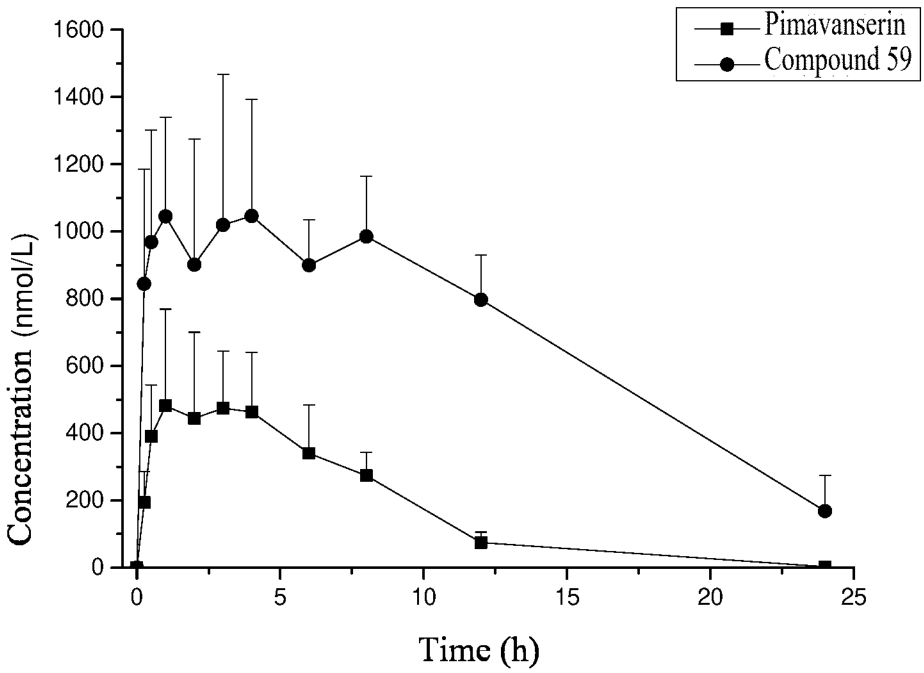
Pimavanserin
Compound 59
Concentration (nmol/L)
1600
1400
1200
1000
800
600
400
200
0
0
5
10
15
20
25
Time (h)

*Fig. 1*

## INTERNATIONAL SEARCH REPORT

International application No.
**PCT/CN2021/107774**

**A. CLASSIFICATION OF SUBJECT MATTER**

C07D 401/12(2006.01)i； A61K 31/4545(2006.01)i； A61P 25/16(2006.01)i； A61P 25/00(2006.01)i； A61P 25/22(2006.01)i； A61P 25/28(2006.01)i； A61P 25/20(2006.01)i； A61P 25/18(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS; DWPI; SIPOABS; CNTXT; WOTXT; EPTXT; USTXT; CNKI; STN-REGISTRY; STN-CAPLUS; 万方; 读秀; ISI-web of science: 山东绿叶制药有限公司, 张睿, 叶亮, 马明旭, 王文艳, 代玉森, 田京伟, HT2A受体抑制剂, 5-羟色胺2A, 帕金森症, PD, 匹莫范色林, Pimavanserin, 5-HT, 5-hydroxytryptamin, 5-HT2A, 脲, 哌啶, +urea, +piperidin+

**C. DOCUMENTS CONSIDERED TO BE RELEVANT**

| Category* | Citation of document, with indication, where appropriate, of the relevant passages | Relevant to claim No. |
|---|---|---|
| X | WO 2019040107 A1 (ACADIA PHARMACEUTICALS, INC.) 28 February 2019 (2019-02-28)<br>description paragraphs [0120]-[0188], [0345], [0354], [0356], [0393], [0581] and description embodiments 6, 7, 14, 100 | 4, 7, 9, 10 |
| X | CN 109111385 A (SHANGHAI HANSOH BIOMEDICAL CO., LTD.) 01 January 2019 (2019-01-01)<br>claims 1-10, 12-14 | 4, 7-10 |
| X | CN 101780080 A (ACADIA PHARMACEUTICALS, INC.) 21 July 2010 (2010-07-21)<br>claim 1 | 4, 7-10 |
| X | WO 2009039461 A2 (ACADIA PHARMACEUTICALS, INC.) 26 March 2009 (2009-03-26)<br>description paragraphs [0012], [0013], [0051] | 4, 7 |
| X | WO 2010111353 A1 (ACADIA PHARM INC et al.) 30 September 2010 (2010-09-30)<br>description, paragraph [0021] | 4, 7, 9, 10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 September 2021** | **14 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/107774**

**C. DOCUMENTS CONSIDERED TO BE RELEVANT**

| Category* | Citation of document, with indication, where appropriate, of the relevant passages | Relevant to claim No. |
|---|---|---|
| X | MICHAEL Berlin et al. "Reduction of hERG Inhibitory Activity in the 4-Piperidinyl Urea Series of H3 Antagonists" *Bioorganic & Medicinal Chemistry Letters,* Vol. 20, No. 7, 28 January 2010 (2010-01-28), ISSN: 0960-894X, page 2360, table 1 | 1, 2, 7 |
| X | WO 2009048752 A2 (DOW AGROSCIENCES LLC) 16 April 2009 (2009-04-16) description, embodiments 124-126 | 1, 7 |
| X | CN 1409703 A (SOCIETE DE CONSEILS DE RECHERCHES ET D'APPLICATIONS SCIENTIFIQUES (S.C.R.A.S)) 09 April 2003 (2003-04-09) description, pages 82-144, tables | 1, 2, 7 |
| A | LUCERO Gonzalez-Sebastian et al. "Selective N-Methylation of Aliphatic Amines with CO2 and Hydrosilanes Using Nickel-Phosphine Catalysts" *Organometallics,* Vol. 34, No. 4, 30 January 2015 (2015-01-30), ISSN: 0276-7333, page 767, table 4 | 4, 7 |
| A | WO 2019040106 A2 (ACADIA PHARMACEUTICALS, INC.) 28 February 2019 (2019-02-28) description paragraphs [00642], [00644], [00646], [00648] | 1-10 |

Form PCT/ISA/210 (second sheet) (January 2015)

**INTERNATIONAL SEARCH REPORT**
**Information on patent family members**

International application No.
**PCT/CN2021/107774**

| Patent document cited in search report | | | Publication date (day/month/year) | Patent family member(s) | | | Publication date (day/month/year) |
|---|---|---|---|---|---|---|---|
| WO | 2019040107 | A1 | 28 February 2019 | SG | 11202001062 | A1 | 30 March 2020 |
| | | | | BR | 112020003477 | A2 | 25 August 2020 |
| | | | | AU | 2018321546 | A1 | 27 February 2020 |
| | | | | IN | 202017011083 | A | 21 August 2020 |
| | | | | CA | 3071644 | A1 | 28 February 2019 |
| | | | | CN | 111132976 | | 08 May 2020 |
| | | | | KR | 20200043409 | A | 27 April 2020 |
| | | | | EP | 3672954 | A1 | 01 July 2020 |
| | | | | US | 2020270239 | A1 | 27 August 2020 |
| | | | | ZA | 202001717 | A | 28 April 2021 |
| | | | | JP | 2020531505 | A | 05 November 2020 |
| CN | 109111385 | A | 01 January 2019 | | None | | |
| CN | 101780080 | A | 21 July 2010 | CN | 102028949 | A | 27 April 2011 |
| | | | | US | 2011257229 | A1 | 20 October 2011 |
| | | | | US | 2012277221 | A1 | 01 November 2012 |
| | | | | US | 2013137727 | A1 | 30 May 2013 |
| | | | | US | 2015202194 | A1 | 23 July 2015 |
| | | | | ES | 2367135 | T3 | 28 October 2011 |
| | | | | CA | 2512639 | C | 30 October 2012 |
| | | | | WO | 2004064738 | A3 | 25 November 2004 |
| | | | | HK | 1126776 | A1 | 16 September 2011 |
| | | | | US | 7994193 | B2 | 09 August 2011 |
| | | | | RU | 2332401 | C2 | 27 August 2008 |
| | | | | US | 2006264465 | A1 | 23 November 2006 |
| | | | | JP | 2006516284 | A | 29 June 2006 |
| | | | | BR | 200406591 | A | 20 December 2005 |
| | | | | US | 2014140982 | A1 | 22 May 2014 |
| | | | | US | 8008323 | B2 | 22 May 2014 |
| | | | | US | 9211289 | B2 | 15 December 2015 |
| | | | | JP | 5184777 | B2 | 17 April 2013 |
| | | | | SG | 170617 | A1 | 30 May 2011 |
| | | | | US | 7659285 | B2 | 09 February 2010 |
| | | | | IN | 235410 | B | 03 July 2009 |
| | | | | NZ | 541146 | A | 30 April 2009 |
| | | | | MX | 255398 | B | 18 March 2008 |
| | | | | EP | 1587789 | A2 | 26 October 2005 |
| | | | | US | 2006199842 | A1 | 07 September 2006 |
| | | | | KR | 20050094857 | A | 28 September 2005 |
| | | | | MX | 2005007568 | A1 | 01 October 2005 |
| | | | | US | 2019030015 | A1 | 31 January 2019 |
| | | | | US | 8377959 | B2 | 19 February 2013 |
| | | | | US | 7601740 | B2 | 13 October 2009 |
| | | | | RU | 2465267 | C2 | 27 October 2012 |
| | | | | CN | 1816524 | A | 09 August 2006 |
| | | | | US | 2006264466 | A1 | 23 November 2006 |
| | | | | IN | 200501635 | P2 | 21 July 2006 |
| | | | | AU | 2004206886 | A1 | 05 August 2004 |
| | | | | WO | 2004064738 | A2 | 05 August 2004 |
| | | | | US | 10525046 | B2 | 07 January 2020 |
| | | | | US | 9566271 | B2 | 17 February 2017 |

Form PCT/ISA/210 (patent family annex) (January 2015)

**INTERNATIONAL SEARCH REPORT**
**Information on patent family members**

International application No.
**PCT/CN2021/107774**

| Patent document cited in search report | Publication date (day/month/year) | Patent family member(s) | Publication date (day/month/year) |
|---|---|---|---|
| | | SG 113945 B | 30 April 2008 |
| | | SG 170617 B | 15 February 2012 |
| | | US 8618130 B2 | 31 December 2013 |
| | | US 2017209424 A1 | 27 July 2017 |
| | | US 2016250200 A1 | 01 September 2016 |
| | | MX 281080 B | 17 November 2010 |
| | | US 2010227886 A1 | 09 September 2010 |
| | | JP 2010174026 A | 12 August 2010 |
| | | ZA 200505680 A | 26 April 2006 |
| | | US 2004213816 A1 | 28 October 2004 |
| | | US 10028944 B2 | 24 July 2018 |
| | | US 8921393 B2 | 30 December 2014 |
| | | KR 101095939 B1 | 19 December 2011 |
| | | US 2010227885 A1 | 09 September 2010 |
| | | US 7732462 B2 | 08 June 2010 |
| | | EP 2009000 A1 | 31 December 2008 |
| | | EP 1587789 B1 | 03 September 2008 |
| | | IN 200703282 P2 | 04 January 2008 |
| | | BR 200406591 B1 | 18 December 2018 |
| | | CN 101780080 B | 04 June 2014 |
| | | ES 2314362 T3 | 16 March 2009 |
| | | US 7713995 B2 | 11 May 2010 |
| | | AU 2004206886 B2 | 02 September 2010 |
| | | EP 2009000 B1 | 08 June 2011 |
| | | CN 1816524 B | 27 June 2012 |
| | | US 8227487 B2 | 24 July 2012 |
| | | US 2020323836 A1 | 15 October 2020 |
| | | DE 602004016288 E | 16 October 2008 |
| WO 2009039461 A2 | 26 March 2009 | CA 2700332 A1 | 26 March 2009 |
| | | US 2009082342 A1 | 26 March 2009 |
| | | WO 2009039461 A3 | 29 October 2009 |
| WO 2010111353 A1 | 30 September 2010 | None | |
| WO 2009048752 A2 | 16 April 2009 | EP 2195302 A2 | 16 June 2010 |
| | | JP 5826327 B2 | 02 December 2015 |
| | | US 2009093486 A1 | 09 April 2009 |
| | | ES 2532119 T3 | 24 March 2015 |
| | | JP 2011500574 A | 06 January 2011 |
| | | JP 2014208648 A | 06 November 2014 |
| | | EP 2195302 B1 | 18 February 2015 |
| | | JP 2014205678 A | 30 October 2014 |
| | | JP 5813825 B2 | 17 November 2015 |
| | | US 8013154 B2 | 06 September 2011 |
| | | WO 2009048752 A3 | 28 May 2009 |
| | | JP 5567483 B2 | 06 August 2014 |
| CN 1409703 A | 09 April 2003 | FR 2802206 A1 | 15 June 2001 |
| | | US 8110574 B2 | 07 February 2012 |
| | | KR 20070014235 A | 31 January 2007 |
| | | JP 4838474 B2 | 14 December 2011 |
| | | US 7858789 B2 | 28 December 2010 |
| | | US 2009149652 A1 | 11 June 2009 |

Form PCT/ISA/210 (patent family annex) (January 2015)

**INTERNATIONAL SEARCH REPORT**
**Information on patent family members**

International application No.
**PCT/CN2021/107774**

| Patent document cited in search report | | | Publication date (day/month/year) | Patent family member(s) | | | Publication date (day/month/year) |
|---|---|---|---|---|---|---|---|
| | | | | AU | 2856001 | A | 25 June 2001 |
| | | | | DE | 60039539 | D1 | 28 August 2008 |
| | | | | EP | 1286966 | A1 | 05 March 2003 |
| | | | | CN | 1207283 | C | 22 June 2005 |
| | | | | US | 2004006089 | A1 | 08 January 2004 |
| | | | | US | 7393861 | B2 | 01 July 2008 |
| | | | | CZ | 305360 | B6 | 19 August 2015 |
| | | | | RU | 2266282 | C2 | 20 December 2005 |
| | | | | WO | 0144191 | A1 | 21 June 2001 |
| | | | | US | 7115634 | B2 | 03 October 2006 |
| | | | | HU | 227838 | B1 | 02 May 2012 |
| | | | | AU | 779341 | B2 | 20 January 2005 |
| | | | | KR | 100725190 | B1 | 07 June 2007 |
| | | | | JP | 2003516965 | A | 20 May 2003 |
| | | | | ES | 2310529 | T3 | 16 January 2009 |
| | | | | CA | 2394086 | C | 03 July 2012 |
| | | | | US | 2011059971 | A1 | 10 March 2011 |
| | | | | KR | 20020062331 | A | 25 July 2002 |
| | | | | US | 2005239796 | A1 | 27 October 2005 |
| | | | | JP | 2011121982 | A | 23 June 2011 |
| | | | | EP | 1286966 | B1 | 16 July 2008 |
| | | | | NZ | 520071 | A | 30 June 2003 |
| | | | | CZ | 200202060 | A3 | 11 December 2002 |
| | | | | HU | 0204515 | A2 | 28 April 2003 |
| WO | 2019040106 | A2 | 28 February 2019 | WO | 2019040106 | A3 | 18 April 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DEGIRMENCI, YILDIZ.** *Cumhuriyet Medical Journal,* 2017, vol. 39 (3), 509-517
- **PRICE, D. L. et al.** *Behavioral Pharmacology,* 2012, vol. 23 (4), 426-433
- **BERGE et al.** Pharmaceutical Salts. *Journal of Pharmaceutical Science,* 1977, vol. 66, 1-19